# EUROPEAN PATENT APPLICATION

(11) **EP 3 828 546 A1**
(43) Date of publication of application: **02.06.2021**
(21) Application number: 19807359.5
(22) Date of filing: 20.05.2019
(51) Int. Cl.: G01N 33/68, G01N 33/574, C12Q 1/6886

(54) **COMPOSITION FOR DIAGNOSING DISEASES**

(30) Priority: 23.05.2018 KR 20180058419
(71) Applicant: Huvet Bio, Inc., Seoul 05836 (KR)
(72) Inventor: LEE, Hyung Keun, Seoul 06279 (KR); LEE, Dong Ki, Seongnam-si Gyeonggi-do 13556 (KR); HAAM, Seung Joo, Seoul 04427 (KR); YOOK, Jong In, Seoul 06004 (KR); JANG, Sung Ill, Seoul 06217 (KR); KIM, So Young, Suwon-si Gyeonggi-do 16357 (KR); YEO, A Reum, Seoul 06372 (KR)
(74) Representative: Mewburn Ellis LLP
(86) International application number: PCT/KR2019/005982
(87) International publication number: WO 2019/225923

(57) **Abstract**

The present invention relates to a composition for diagnosing pancreatic disease, and more particularly to a composition capable of diagnosing pancreatic cancer, a diagnostic kit comprising the same, and a method of providing information for diagnosis using the composition. The present invention makes it possible to accurately predict or identify the likelihood of onset of pancreatic cancer, and furthermore, to diagnose pancreatic cancer by effectively distinguishing from other types of cancer. In addition, according to the present invention, it is possible to simply and rapidly diagnose pancreatic cancer by a non-invasive method using mononuclear cells isolated from blood, serum or plasma obtained from a subject.

## Description

### Technical Field

The present invention relates to a composition capable of diagnosing pancreatic disease, and more particularly to a composition capable of diagnosing pancreatic cancer or the like, a diagnostic kit comprising the same, and a method of providing information for diagnosis using the composition.

### Background Art

Studies on methods for the treatment and diagnosis of cancer among the major diseases of modern people have been actively conducted with a focus on lung cancer, liver cancer and stomach cancer, which have high incidence. However, studies on esophageal cancer, colorectal cancer, pancreatic cancer and the like, which have lower incidence, are relatively insufficient. In particular, pancreatic cancer exhibits almost no symptoms in an early stage, but generally exhibits symptoms such as pain and weight loss after systemic metastasis. Therefore, pancreatic cancer has a lower cure rate, so regular testing therefor is very important. Most clinical symptoms develop slowly, and appetite decline, weakness and weight loss are the most common symptoms. Pancreatic cancer is a fatal cancer with a 5-year survival rate of 1 to 4% and a median survival rate of 5 months and shows the worst prognosis among human cancers. In addition, 80% to 90% of pancreatic cancer patients are diagnosed in a condition in which curative resection enabling complete cure is impossible. For this reason, the prognosis of pancreatic cancer patients is poor, and treatment thereof depends mainly on chemotherapy. Thus, there is an urgent need for the development of methods for early diagnosis of pancreatic cancer more than any other kind of human cancer.

The therapeutic effects of several anticancer drugs, including 5-fluorouracil, gemcitabine and Tarceva, which are currently known to be effective against pancreatic cancer, are extremely poor, and the rate of response to chemotherapy is only about 15%. This suggests that more effective early diagnosis methods and treatment methods are urgently needed in order to improve the prognosis of pancreatic cancer patients.

Diagnosis of pancreatic cancer has been performed by x-ray radiography of the stomach and duodenum, cholangiography via the skin and liver, endoscopic retrograde cholangiography or the like, but in recent years, ultrasonography and computed tomography have been most commonly used. When a lesion of a disease is detected using any of these methods, more precise biopsy can be performed to obtain more accurate examination results. However, these diagnostic methods have low accuracy or cause discomfort and pain to patients, and thus patients are reluctant to participate in such diagnostic methods. Therefore, there has been a need for the development of examination methods capable of simply and rapidly diagnosing pancreatic cancer.

In this regard, Korean Patent No. 10-0819122 discloses a technique that uses matrilin, transthyretin and stratifin as pancreatic cancer markers, and Korean Patent Application Publication No. 2012-0082372 discloses a technique that uses various pancreatic cancer markers. In addition, Korean Patent Application Publication No. 2009-0003308 discloses a method of diagnosing pancreatic cancer by detecting the expression level of REG4 protein in a blood sample from a subject, and Korean Patent Application Publication No. 2012-0009781 discloses an assay method comprising measuring the expression level of XIST RNA in cancer tissue isolated from a subject in order to provide information required for the diagnosis of pancreatic cancer. Korean Patent Application Publication No. 2007-0119250 discloses a novel gene LBFL313 family differentially expressed in human pancreatic cancer tissue compared to pancreatic tissue of normal humans, and U.S. Patent Application Publication No. 2011/0294136 discloses a method of diagnosing pancreatic cancer using biomarkers such as keratin 8 protein. However, the identification of markers with better effects is needed, since there is a great difference in diagnostic efficiency and accuracy between the markers.

Accordingly, the present inventors have made extensive efforts to identify useful markers for early diagnosis of pancreatic cancer among pancreatic diseases, and as a result, have found that the expression of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) or interferon lambda receptor 1 isoform 1 (IFNLR1 isoform 1) or mRNA thereof in liquid biopsys isolated from pancreatic cancer patients significantly increases, thereby completing the present invention.

### Summary of the Invention

An object of the present invention is to provide a composition capable of simply and accurately diagnosing a disease such as pancreatic disease, autoimmune disease, allergy, Grave's disease, Hashimoto's thyroiditis, autoimmune lymphoproliferative syndrome (ALPS), myasthenia gravis, Kawasaki disease or psoriasis, preferably pancreatic cancer, by measuring the expression level of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) or interferon lambda receptor 1 isoform 1 (IFNLR1 isoform 1) protein, or the mRNA expression level of a gene encoding the protein.

Another object of the present invention is to provide a kit capable of simply and accurately diagnosing the above-described disease by measuring the expression level of interleukin 10 receptor, interleukin 22 receptor, interleukin 22, interleukin 28A, interleukin 28B isoform 1, interleukin 28B isoform 2, interleukin 29 or interferon lambda receptor 1 isoform 1, or the mRNA expression level of a gene encoding the protein.

Still another object of the present invention is to provide a method of providing information for diagnosis of the above-described disease by measuring the expression level of interleukin 10 receptor, interleukin 22 receptor, interleukin 22, interleukin 28A, interleukin 28B isoform 1, interleukin 28B isoform 2, interleukin 29 or interferon lambda receptor 1 isoform 1, or the mRNA expression level of a gene encoding the protein.

Yet another object of the present invention is to provide a method of providing information for diagnosis of recurrence of the above-described disease by measuring the expression level of interleukin 10 receptor, interleukin 22 receptor, interleukin 22, interleukin 28A, interleukin 28B isoform 1, interleukin 28B isoform 2, interleukin 29 or interferon lambda receptor 1 isoform 1, or the mRNA expression level of a gene encoding the protein.

Still yet another object of the present invention is to provide a method of screening a drug for treatment of the above-described disease by measuring the expression level of interleukin 10 receptor, interleukin 22 receptor, interleukin 22, interleukin 28A, interleukin 28B isoform 1, interleukin 28B isoform 2, interleukin 29 or interferon lambda receptor 1 isoform 1, or the mRNA expression level of a gene encoding the protein.

To achieve the above objects, the present invention provides a composition for diagnosing pancreatic disease comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein.

The present invention also provides a kit for diagnosing pancreatic disease comprising the composition.

The present invention also provides a method for providing information for diagnosis of pancreatic disease comprising a step of: measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, in a biopsy isolated from a subject of interest.

The present invention also provides a method for predicting the likelihood of recurrence of disease comprising a step of: measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, in a biopsy isolated from a pancreatic disease patient who has received treatment.

The present invention also provides a method for screening a drug for disease treatment comprising steps of:
(a) measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, in a biopsy isolated from a pancreatic disease patient;
(b) administering a candidate drug to the patient; and
(c) measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of the gene encoding the protein, in a biopsy isolated from the patient after administration of the candidate drug.

The present invention also provides a composition for use in diagnosis of pancreatic disease comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein.

The present invention also provides a method for diagnosing pancreatic disease comprising a step of: measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, in a biopsy isolated from a subject of interest.

The present invention also provides the use of a composition comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, for diagnosis of pancreatic disease.

The present invention also provides the use of a composition comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, in the manufacture of a medicament for diagnosis of pancreatic disease.

### Brief Description of Drawings

FIG. 1 shows the proportion (%) of the number of mononuclear cells expressing IL-22 relative to the total number of mononuclear cells in blood collected from a normal control group, pancreatic cancer patients, pancreatitis patients, lung cancer patients and colorectal cancer patients, measured using FACS analysis in Example 1.
FIG. 2 shows the proportion (%) of the number of mononuclear cells expressing IL-22R relative to the total number of mononuclear cells in blood collected from a normal control group, pancreatic cancer patients, pancreatitis patients, lung cancer patients and colorectal cancer patients, measured using FACS analysis in Example 1.
FIG. 3 shows the proportion (%) of the number of mononuclear cells expressing IL-10R relative to the total number of mononuclear cells in blood collected from a normal control group, pancreatic cancer patients, pancreatitis patients, lung cancer patients and colorectal cancer patients, measured using FACS analysis in Example 1.
FIG. 4 is a graph comparing the mRNA expression levels of IL-10RB in mononuclear cells in blood collected from a normal control group, pancreatic cancer patients, cholangiocarcinoma patients and gallbladder cancer patients, measured in Example 2.
FIG. 5 is a graph comparing the mRNA expression levels of IL-22RA in mononuclear cells in blood collected from a normal control group, pancreatic cancer patients, cholangiocarcinoma patients and gallbladder cancer patients, measured in Example 2.
FIG. 6 is a graph comparing the mRNA expression levels of IL-22 in mononuclear cells in blood collected from a normal control group, pancreatic cancer patients, cholangiocarcinoma patients and gallbladder cancer patients, measured in Example 3.
FIG. 7 is a graph comparing the mRNA expression levels of IL-29 in mononuclear cells in blood collected from a normal control group, pancreatic cancer patients, cholangiocarcinoma patients and gallbladder cancer patients, measured in Example 3.
FIG. 8 is a graph comparing the mRNA expression levels of IFNLR1 isoform 1 in mononuclear cells in blood collected from a normal control group, pancreatic cancer patients, cholangiocarcinoma patients and gallbladder cancer patients, measured in Example 4.
FIG. 9 is a graph comparing the mRNA expression levels of IFNLR1 isoform 3 in mononuclear cells in blood collected from a normal control group, pancreatic cancer patients, cholangiocarcinoma patients and gallbladder cancer patients, measured in Example 4.
FIG. 10 shows the proportion (%) of the number of mononuclear cells expressing IL-22 relative to the total number of mononuclear cells in blood collected from pancreatic cancer patients before and after surgery, measured in Example 5.
FIG. 11 shows the proportion (%) of the number of mononuclear cells expressing IL-22R relative to the total number of mononuclear cells in blood collected from pancreatic cancer patients before and after surgery, measured in Example 5.
FIG. 12 shows the proportion (%) of the number of mononuclear cells expressing IL-10R relative to the total number of mononuclear cells in blood collected from pancreatic cancer patients before and after surgery, measured in Example 5.
FIG. 13 is a graph comparing the results of analysis performed by paired T-test after measuring the proportion of the number of mononuclear cells expressing IL-10R relative to the total number of mononuclear cells in blood collected from pancreatic cancer patients before surgery and 3 months after surgery in Example 6.
FIG. 14 is a graph comparing the mRNA expression levels of IL-10RB in exosomes in blood collected from a normal control group, pancreatic cancer patients, cholangiocarcinoma patients and gallbladder cancer patients, measured in Example 7.
FIG. 15 is a graph comparing the mRNA expression levels of IL-22RA in exosomes in blood collected from a normal control group, pancreatic cancer patients, cholangiocarcinoma patients and gallbladder cancer patients, measured in Example 7.
FIG. 16 is a graph comparing the mRNA expression levels of IFNLR1 isoform 1 in exosomes in blood collected from a normal control group, pancreatic cancer patients, cholangiocarcinoma patients and gallbladder cancer patients, measured in Example 7.
FIG. 17 is a graph comparing the mRNA expression levels of IL-10RB (BR), IL-22RA (AR), IFNLR1 isoform 1 (LR) and GPC-1 (M) in exosomes in blood collected from a normal control group, pancreatic cancer patients, cholangiocarcinoma patients and gallbladder cancer patients, measured in Example 8.
FIG. 18 shows the results of score analysis for combinations of IL-10RB (BR) and IFNLR1 isoform 1 (LR) biomarkers for prediction of pancreatic cancer patients, performed in Example 8.
FIG. 19 is a graph comparing the mRNA expression levels of IL-10RB (BR), IL-22RA (AR) and IL-22(A) in blood collected from a normal control group, pancreatic cancer patients, cholangiocarcinoma patients and gallbladder cancer patients, measured in Example 9.
FIG. 20 shows the results of score analysis for various combinations of IL-10RB (BR), IL-22RA (AR) and IL-22 (A) biomarkers for prediction of pancreatic cancer patients, performed in Example 9.
FIG. 21 shows the results of score analysis for various combinations of IL-10RB (BR), IL-22RA (AR) and IL-22 (A) biomarkers for prediction of pancreatic cancer patients, performed in Example 9.
FIG. 22 is a graph comparing the mRNA expression levels of IFNLR1 isoform 1 (LR) and IL-29 (L) in mononuclear cells (PBMC) in blood collected from a normal control group, pancreatic cancer patients, cholangiocarcinoma patients and gallbladder cancer patients, measured in Example 10.
FIG. 23 shows the results of analyzing the proportion of the number of cells expressing IFNLR1 isoform 1 relative to the total number of mononuclear cells in blood from pancreatic cancer patients by FACS analysis in Example 11.
FIG. 24 shows the results of analyzing the proportion of the number of cells expressing IFNLR1 isoform 1 relative to the total number of mononuclear cells in blood from chronic pancreatitis patients by FACS analysis in Example 11.
FIG. 25 shows the proportion (%) of the number of mononuclear cells expressing IL-10RB (BR), IL-22RA (AR) or IL-22 (A) relative to the total number of mononuclear cells in blood collected from a normal control group, pancreatic cancer patients, acute pancreatitis patients and chronic pancreatitis patients, measured in Example 12.
FIG. 26 shows the results of performing score analysis of IL-10RB (BR) and IL-22 (A) biomarkers for prediction of pancreatic cancer patients by FACS analysis in Example 12.
FIG. 27 graphically shows the results of measuring the mRNA expression level of each of IL-10RB, IL-22RA, IFNLR1 isoform 1, IL-22 and GPC-1 in mononuclear cells isolated from blood collected before and after surgery of pancreatic cancer patients who underwent surgical resection, in Example 13.
FIG. 28 shows the results of measuring the mRNA expression level of each of IL-10RB (BR), IL-22RA (AR), IL-22 (A), CEA and CA 19-9 in mononuclear cells isolated from blood collected before and after surgery of pancreatic cancer patients who underwent surgical resection, in Example 14, and graphically shows the proportion of the number of cells expressing IL-10RB (BR), IL-22RA (AR), IL-22 (A) and CA19-9 relative to the total number of mononuclear cells before and after surgery.
FIG. 29 shows the results of measuring the mRNA expression level of each of IL-10RB (BR), IL-22RA (AR), IL-22 (A), CEA and CA 19-9 in mononuclear cells isolated from blood collected before and after surgery of pancreatic cancer patients who have undergone surgical resection, in Example 14, and graphically shows the proportion of the number of cells expressing IL-10RB (BR), IL-22RA (AR), IL-22 (A) and CA19-9 relative to the total number of mononuclear cells before and after surgery.
FIG. 30 shows the results of measuring the mRNA expression level of each of IL-10RB (BR), IL-22RA (AR), IL-22 (A), CEA and CA 19-9 in mononuclear cells isolated from blood collected before and after surgery of pancreatic cancer patients who have undergone surgical resection, in Example 14, and graphically shows the proportion of the number of cells expressing IL-10RB (BR), IL-22RA (AR), IL-22 (A) and CA19-9 relative to the total number of mononuclear cells before and after surgery.
FIG. 31 shows the results of measuring the mRNA expression level of each of IL-10RB (BR), IL-22RA (AR), IL-22 (A), CEA and CA 19-9 in mononuclear cells isolated from blood collected before and after surgery of pancreatic cancer patients who have undergone surgical resection, in Example 14.
FIG. 32 shows the results of measuring the mRNA expression level of each of IL-10RB (BR), IL-22RA (AR), IL-22 (A), CEA and CA 19-9 in mononuclear cells isolated from blood collected before and after surgery of pancreatic cancer patients who have undergone surgical resection, in Example 14.
FIG. 33 shows the results of measuring the mRNA expression level of each of IL-10RB, IL-22RA, IFNLR1 isoform 1, IL-22, IL-29 and GPC-1 in mononuclear cells isolated from blood collected before and after treatment of pancreatic cancer patients who received anticancer chemotherapy, in Example 15.
FIG. 34 shows the results of analyzing the proportion of the number of cells expressing IL-10RB (BR) and IL-22RA (AR) relative to the total number of mononuclear cells isolated from blood collected before and after treatment of pancreatic cancer patients who received anticancer chemotherapy, using FACS analysis in Example 16.
FIG. 35 shows the results of performing FACS analysis and Giemsa staining using antibodies specific to IL-10RB (BR) and IL-22RA (AR) for mononuclear cells isolated from blood collected from pancreatic cancer patients, in Example 17.
FIG. 36 shows the results of performing FACS analysis and Giemsa staining using antibodies specific to IL-10RB (BR) and IL-22RA (AR) for mononuclear cells isolated from blood collected from acute pancreatitis patients, in Example 17.
FIG. 37 shows the results of performing FACS analysis and H&E staining using antibodies specific to IL-10RB (BR) and IL-22RA (AR) for whole-blood cells isolated from blood collected from pancreatic cancer patients, in Example 18.
FIG. 38 shows the results of isolating cells expressing IL-10RB (BR), IL-22RA (AR) and IFNLR1 isoform 1 (LR) through transcriptome analysis for whole-blood cells isolated from blood collected from pancreatic cancer patients, in Example 19.
FIG. 39 shows the experimental design of Example 20.
FIG. 40 shows images of the pancreas and spleen, obtained after injecting Panc-1 cells and Apsc cells into mice in Example 20.
FIG. 41 shows H&E staining images of pancreatic tissue, obtained after injecting Panc-1 cells and Apsc cells into mice in Example 20.
FIG. 42 shows the results of performing FACS analysis using antibodies specific to IL-10RB (BR) and IL-22RA (AR) for immune cells (B cells) infiltrating the pancreases after injecting Panc-1 cells and Apsc cells into mice, in Example 20.
FIG. 43 shows the results of performing FACS analysis using antibodies specific to IL-10RB (BR) and IL-22RA (AR) for immune cells (dendritic cells and macrophages) infiltrating the pancreases after injecting Panc-1 cells and Apsc cells into mice, in Example 20.
FIG. 44 shows the results of performing FACS analysis using antibodies specific to IL-10RB (BR) and IL-22RA (AR) for immune cells (neutrophil cells) infiltrating the pancreases after injecting Panc-1 cells and Apsc cells into mice, in Example 20.
FIG. 45 graphically shows the results of measuring the mRNA expression level of each of IL-28a, IL-28b isoform 1 and IL-28b isoform 2 in mononuclear cells isolated from blood collected from a normal control group and pancreatic cancer patients, in Example 21.

### Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

Pancreatic cancer exhibits almost no symptoms in an early stage, but generally exhibits symptoms such as pain and weight loss after systemic metastasis thereof. Therefore, pancreatic cancer has a lower cure rate, so regular diagnosis therefor is very important. Most clinical symptoms develop slowly, and appetite decline, weakness and weight loss are the most common symptoms. Pancreatic cancer is a fatal cancer with a 5-year survival rate of 1 to 4% and a median survival rate of 5 months and shows the worst prognosis among human cancers. In addition, 80% to 90% of pancreatic cancer patients are diagnosed in a condition in which curative resection enabling complete cure is impossible. For this reason, the prognosis of pancreatic cancer patients is poor, and treatment thereof depends mainly on chemotherapy. Thus, there is an urgent need for the development of methods for early diagnosis of pancreatic cancer more than any other kind of human cancer.

Inventors of the present invention have made extensive efforts to identify useful markers for early diagnosis of pancreatic cancer among pancreatic diseases, and as a result, have found that the expression of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) or interferon lambda receptor 1 isoform 1 (IFNLR1 isoform 1) or mRNA thereof in liquid biopsies isolated from pancreatic cancer patients significantly increases, thereby completing the present invention.

Therefore, in one aspect, the present invention is directed to a composition for diagnosing pancreatic disease comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein.

Examples of the disease whose onset or likelihood of onset may be predicted using the diagnostic composition of the present invention comprise, but are not limited to, pancreatic disease, autoimmune disease, allergy, Grave's disease, Hashimoto's thyroiditis, autoimmune lymphoproliferative syndrome (ALPS), myasthenia gravis, Kawasaki disease, and psoriasis.

In particular, in the present invention, the diagnostic composition may be used to diagnose a pancreatic disease such as pancreatic cancer or pancreatitis, preferably pancreatic cancer. Specifically, the diagnostic composition of the present invention may detect or diagnose pancreatic cancer by distinguishing between a normal group and a pancreatic cancer patient group. In addition, the diagnostic composition may selectively detect or diagnose pancreatic cancer by distinguishing pancreatic cancer from other types of cancer such as lung cancer or colorectal cancer.

As used herein, the term "pancreatic cancer" refers to cancer originating from pancreatic cells. There are various types of pancreatic cancer. Pancreatic ductal adenocarcinoma arising from pancreatic duct cells accounts for about 90% of pancreatic cancer cases, and thus pancreatic cancer generally refers to pancreatic ductal adenocarcinoma. In addition, there are cystadenocarcinoma, endocrine tumor and the like. About 5% to 10% of pancreatic cancer patients have a genetic predisposition thereto. The proportion of pancreatic cancer patients that have family history of pancreatic cancer is about 7.8%, which is higher than 0.6%, the proportion of pancreatic cancer onset in normal people. Pancreatic cancer has a very poor prognosis, with a 5-year survival rate of less than 5%. The reason for this is that most cases are detected after the cancer has progressed, so surgical resection is possible in less than 20% of cases at the time of detection, and even if the cancer is determined to have been completely resected by visual examination, the increase in survival rate is low due to micrometastasis, and responsiveness to chemotherapy and radiotherapy is low. Therefore, the most important method for improving the survival rate is early detection and operation when symptoms are absent or nonspecific.

As used herein, the term "pancreatitis" refers to a disease caused by inflammation of the pancreas, and comprises acute pancreatitis and chronic pancreatitis. The pancreatic juice contains digestive enzymes such as amylase (acting on carbohydrate hydrolysis), trypsin (acting on protein hydrolysis), and lipase (acting on lipid hydrolysis). Pancreatitis results from various causes such as metabolic disturbances, drugs, and abdominal injury, as well as self-degradation of the pancreas induced by the enzymes due to defective flow of pancreatic juice attributable to alcohol abuse, gallstones and the like. Pancreatitis is an inflammatory disease of the pancreas that induces damage to pancreatic acinar cells, extensive interstitial edema, and migration of neutrophil granulocytes to hemorrhage and injury sites. Pancreatitis can be broadly divided into two types: a mild type of pancreatitis wherein interstitial edema and peripancreatic fat necrosis are detected, and a severe type of pancreatitis which is accompanied by extensive peripancreatic and intrapancreatic fat necrosis, pancreatic parenchymal necrosis, and hemorrhaging (Bank PA., Am. J. gastroenterol., 89, pp151-152, 1994.; Bradley EL., Arch. Surg., 128, pp586-590, 1993).

As used herein, the term "autoimmune disease" refers to a non-malignant disease or disorder that is generated and specified for subject's own tissues. One of the most important properties in all normal subjects is that they do not respond negatively to self-antigenic substances, but they can recognize and respond to non-self-antigens and remove the same. The unresponsiveness of a living body to a self-antigen is referred to as "immunologic unresponsiveness" or "tolerance". However, when abnormalities occur in induction or maintenance of self-tolerance, an immune response to the self-antigen occurs, and as a result, a phenomenon of attacking self-tissue occurs. Diseases caused by the aforementioned process are called "autoimmune diseases". Autoimmune disease is an inflammatory disease in which an antibody is produced against a patient's own organ tissue or ingredient, and may generally refer to diseases that cause chronic systemic inflammation in many tissues and organs.

Specifically, examples of the autoimmune disease in the present invention comprise, but are not limited to, rheumatoid arthritis (SLE), systemic lupus erythematosus, Crohn's disease, ulcerative colitis, multiple sclerosis, uveitis, autoimmune meningitis, Sjogren's syndrome, scleroderma, Wegener's granulomatosis, sarcoidosis, septic shock, dacryoadenitis, stroke, arteriosclerosis, vascular restenosis, type I diabetes, type II diabetes, urticaria, conjunctivitis, psoriasis, systemic inflammatory syndrome, multiple myositis, dermatomyositis, nodular polyarticular arthritis, mixed connective tissue disease, gout, Parkinson's disease, amyotrophic lateral sclerosis, diabetic retinopathy, chronic thyroiditis, celiac disease, myasthenia gravis, vesical pemphigus, viral diseases, bacterial diseases, arteriosclerosis, angioma, angiofibroma, reperfusion injury, and cardiac hypertrophy.

As used herein, the term "allergy" may be used interchangeably with "atopic disorder" and may refer to a disease caused by a biochemical phenomenon that shows a specific and modified response to foreign substance, that is, an allergen.

In the present invention, the allergy may be atopic dermatitis, contact dermatitis, eczema, asthma, hypersensitivity, allergic rhinitis, allergic conjunctivitis, allergic dermatitis, urticaria, insect allergy, food allergy or drug allergy, but is not limited thereto.

In the present invention, the term "diagnosis" comprises determining the susceptibility of a subject to a particular disease or disorder, determining whether the subject has a particular disease or disorder at present, determining the prognosis of the subject having a particular disease or disorder (e.g., identifying a pre-metastatic or metastatic cancerous condition, determining the stage of the cancer, or determining the response of the cancer to treatment), or therametrics (e.g., monitoring the status of the subject in order to provide information for therapeutic effects). For the purpose of the present invention, diagnosis means determining the onset or likelihood (risk) of onset of the disease.

The diagnostic composition of the present invention preferably comprises an agent for measuring the expression level of interleukin 10 receptor or the mRNA expression level of a gene encoding the interleukin 10 receptor. In addition, the diagnostic composition of the present invention may further comprise an agent for measuring the expression level of at least one protein selected from the group consisting of interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, in order to increase accuracy in diagnosing the disease.

The diagnostic composition of the present invention preferably comprises an agent for measuring the expression of each of interleukin 10 receptor protein and interferon lambda receptor 1 isoform 1 protein.

The diagnostic composition of the present invention preferably comprises an agent for measuring the mRNA expression level of a gene encoding each of the interleukin 10 receptor protein and the interferon lambda receptor 1 isoform 1 protein.

The diagnostic composition of the present invention preferably comprises an agent for measuring the expression level of each of the interleukin 10 receptor protein and the interleukin 22 receptor protein.

The diagnostic composition of the present invention preferably comprises an agent for measuring the mRNA expression level of a gene encoding each of the interleukin 10 receptor protein and the interleukin 22 receptor protein.

The diagnostic composition of the present invention preferably comprises an agent for measuring the expression level of each of the interleukin 10 receptor protein and the interleukin 22 protein.

The diagnostic composition of the present invention preferably comprises an agent for measuring the mRNA expression level of a gene encoding each of the interleukin 10 receptor protein and the interleukin 22 protein.

The diagnostic composition of the present invention preferably comprises an agent for measuring the expression level of each of the interleukin 10 receptor protein, the interleukin 22 receptor protein and the interleukin 22 protein.

The diagnostic composition of the present invention preferably comprises an agent for measuring the mRNA expression level of a gene encoding each of the interleukin 10 receptor protein, the interleukin 22 receptor protein and the interleukin 22 protein.

The diagnostic composition of the present invention preferably comprises an agent for measuring the expression level of interleukin 28A protein, interleukin 28B isoform 1 protein or interleukin 28B isoform 2 protein.

The diagnostic composition of the present invention preferably comprises an agent for measuring the mRNA expression level of a gene encoding interleukin 28A, interleukin 28B isoform 1 or interleukin 28B isoform 2.

As used herein, the term "interleukin 10 receptor (IL-10R)" refers to a type II cytokine receptor which corresponds to a tetramer consisting of two α-subunits and two β-subunits. The α-subunit is expressed in hematopoietic cells such as T cells, B cells, NK cells, mast cells and dendritic cells, and the β-subunit is expressed in various ways. In the present invention, the interleukin 10 receptor is preferably the interleukin 10 receptor β-subunit (IL-10RB) represented by SEQ ID NO: 1.

As used herein, the term "interleukin 22 receptor (IL-22R)" refers to a type II cytokine receptor which corresponds to a heterodimer consisting of an α1 subunit and a β2 subunit and binds to interleukin 22. In the present invention, the interleukin 22 receptor is preferably the interleukin 22 receptor α1subunit (IL-22RA1) represented by SEQ ID NO: 2.

As used herein, the term "interleukin' 22 (IL-22)" refers to cytokine belonging to a cytokine group called "IL-10 family" or "IL-10 superfamily" (IL-19, IL-20, IL-24 and IL-26), which mediates a cellular immune response. The interleukin 22 binds to heterodimeric cell surface receptors consisting of IL-10R2 and IL-22R1 subunits. In the present invention, the interleukin 22 may be represented by the amino acid sequence of SEQ ID NO: 3.

As used herein, the "interleukin 28A (IL-28A)" is also called "interferon-lambda 2" and corresponds to a type III interferon that is upregulated by viral infection. The interleukin 28A may interact with receptor complexes comprising IL-10R beta and IL-28R alpha/IFN-lambda R1 in epithelial cells. In the present invention, the interleukin 28A may be represented by the amino acid sequence of SEQ ID NO: 4.

In the present invention, the "interleukin 28B (IL-28B)" is also called "interferon lambda 3 (IFNL3)", and corresponds to a cytokine associated with type I interferon and IL-10 family. The interleukin 28B, interleukin 28A and interleukin 29 genes form a cytokine gene cluster on a chromosomal region mapped to 19q13. The expression of cytokines encoded by these three genes is induced by viral infection. These three cytokines interact with a class II cytokine receptor, a heterodimer consisting of interleukin 10 receptor beta (IL10RB) and interleukin 28 receptor alpha (IL28RA). In the present invention, the interleukin 28B isoform 1 may be represented by the amino acid sequence of SEQ ID NO: 5, and the interleukin 28B isoform 2 may be represented by the amino acid sequence of SEQ ID NO: 6.

As used herein, the term "interleukin 29 (IL-29)", which is also called "interferon lambda-1", refers to a protein encoded by interleukin 29 gene located on chromosome 19. Interleukin 29 belongs to the helical cytokine family, and corresponds to type III interferon. Interleukin 29 plays a key role in host defenses against microorganisms, and the expression level thereof significantly increases in virus-infected cells. In the present invention, the interleukin 29 may be represented by the amino acid sequence of SEQ ID NO: 7.

As used herein, the term "interferon lambda receptor 1 (IFNLR1)" refers to a protein encoded by a gene belonging to the type II cytokine receptor family. The interferon lambda receptor 1 binds to an interleukin 10 receptor beta subunit to form a receptor complex. This receptor complex may interact with interleukin 28A, interleukin 28B and interleukin 29. In the present invention, the interferon lambda receptor 1 may be interferon lambda receptor 1 isoform 1 (IFNLR1 isoform 1) represented by the amino acid sequence of SEQ ID NO: 8.

In the present invention, the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the expression level of a gene encoding the protein, may be measured from a biopsy, preferably liquid biopsy, isolated from a subject of interest, for example, may be measured from cells or exosomes isolated from blood, serum or plasma, more preferably mononuclear cells, granulocytes or exosomes isolated from the liquid biopsy.

In conventional methods for measuring the expression levels of biomarkers for diagnosis of various diseases, particularly pancreatic diseases, cells are isolated mainly from pancreatic tissue and then the expression levels of biomarkers in the cells are measured. These methods have the inconvenience of isolating cells from pancreatic tissue for diagnosis of pancreatic disease, preferably pancreatic cancer, and have a disadvantage that it is almost impossible to diagnose pancreatic cancer in the early stages because there are no subjective symptoms in the early stages of pancreatic cancer.

However, according to the present invention, it is possible to rapidly, simply and very accurately diagnose the onset of various diseases comprising pancreatic cancer and the likelihood of onset thereof by measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29, and interferon lambda receptor 1 isoform 1, or the mRNA of a gene encoding the protein, in a liquid biopsy, which is blood, serum or plasma isolated from a subject of interest, or mononuclear cells, granulocytes, or exosomes isolated therefrom.

Specifically, when the expression level of the marker according to the present invention is measured in a biopsy or a liquid biopsy isolated from a subject of interest, or mononuclear cells, granulocytes or exosomes isolated from the liquid biopsy, as described above, the onset of disease or the likelihood of the onset thereof may be rapidly and simply diagnosed, since there is no need for invasive procedures, for example, comprising performing laparotomy on a patient and isolating tissue cells from tissue (for example, pancreatic tissue), and it takes about 5 minutes or less to obtain a biopsy containing mononuclear cells, granulocytes or exosomes and about 2 hours or less to measure the expression levels of various disease biomarkers according to the present invention from the mononuclear cells, granulocytes or exosomes.

In the present invention, the agent for measuring the expression level of interleukin 10 receptor, interleukin 22 receptor, interleukin 22, interleukin 28A, interleukin 28B isoform 1, interleukin 28B isoform 2, interleukin 29 or interferon lambda receptor 1 isoform 1 is not particularly limited, but may preferably comprise at least one selected from the group consisting of antibodies, oligopeptides, ligands, PNAs (peptide nucleic acids) and aptamers, which bind specifically to each of the interleukin 10 receptor, interleukin 22 receptor, interleukin 22, interleukin 28A, interleukin 28B isoform 1, interleukin 28B isoform 2, interleukin 29, and interferon lambda receptor 1 isoform 1 proteins.

As used herein, the expression "measuring the expression level of proteins" refers to a process of detecting the presence or expression level of Interleukin 10 receptor, interleukin 22 receptor, interleukin 22, interleukin 28A, interleukin 28B isoform 1, interleukin 28B isoform 2, interleukin 29 or interferon lambda receptor 1 isoform 1 protein, which is a marker for diagnosis of pancreatic disease, in a biological sample in order to diagnose diseases comprising pancreatic cancer according to the present invention. Methods for measuring or comparatively analyzing the expression level of the receptor comprise, but are not limited to, protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, and enzyme-linked immunosorbent assay (ELISA).

As used herein, the term "antibody" refers to a substance that specifically binds to an antigen to induce an antigen-antibody reaction. For the purpose of the present invention, the antibody refers to an antibody that binds specifically to interleukin 10 receptor, interleukin 22 receptor, interleukin 22, interleukin 28A, interleukin 28B isoform 1, interleukin 28B isoform 2, interleukin 29 or interferon lambda receptor 1 isoform 1. Examples of the antibody of the present invention comprise all of polyclonal antibodies, monoclonal antibodies and recombinant antibodies. The antibody may be easily produced using techniques that are well-known in the art. For example, a polyclonal antibody may be produced by a method well-known in the art, which comprises injecting the interleukin 10 receptor, interleukin 22 receptor, interleukin 22, interleukin 28A, interleukin 28B isoform 1, interleukin 28B isoform 2, interleukin 29 or interferon lambda receptor 1 isoform 1 protein into an animal, and collecting blood from the animal to obtain a serum containing an antibody. This polyclonal antibody may be produced from any animals such as goats, rabbits, sheep, monkeys, horses, pigs, cows and dogs. In addition, the monoclonal antibody may be produced using a hybridoma method well-known in the art (see Kohler and Milstein (1976) European Journal of Immunology 6:511-519) or a phage antibody library technique (see Clackson et al, Nature, 352: 624-628, 1991; Marks et al., J. Mol. Biol., 222: 58, 1-597, 1991). The antibody produced by the above-described method may be isolated and purified by a method such as gel electrophoresis, dialysis, salt precipitation, ion exchange chromatography, or affinity chromatography. In addition, the antibody of the present invention comprises not only a complete form having two full-length light chains and two full-length heavy chains, but also a functional fragment of the antibody molecule. The functional fragment of the antibody molecule refers to a fragment having at least an antigen-binding function, and examples thereof comprise Fab, F(ab'), F(ab')2, and Fv.

As used herein, the term "PNA (peptide nucleic acid)" refers to an artificially synthesized DNA- or RNA-like polymer, which was first introduced by professors Nielsen, Egholm, Berg and Buchardt at the University of Copenhagen, Denmark in 1991. DNA has a phosphate-ribose sugar backbone, but PNA has repeated N-(2-aminoethyl)-glycine backbones linked via peptide bonds, and thus has a significantly increased binding affinity for DNA or RNA and increased stability. Thus, PNA is used for molecular biology, diagnostic assays and antisense therapies. The PNA is disclosed in detail in the literature [Nielsen PE, Egholm M, Berg RH, Buchardt O (December 1991). "Sequence-selective recognition of DNA by strand displacement with a thymine-substituted polyamide". Science 254 (5037): 1497-1500].

As used herein, the term "aptamer" refers to an oligonucleotide or a peptide molecule, and the general contents of the aptamer are disclosed in detail in the literature [Bock LC et al., Nature 355(6360):5646(1992); Hoppe-Seyler F, Butz K "Peptide aptamers: powerful new tools for molecular medicine". J Mol Med. 78(8) : 42630 (2000); Cohen BA, Colas P, Brent R. "An artificial cell-cycle inhibitor isolated from a combinatorial library". Proc Natl Acad Sci USA. 95(24): 142727 (1998)].

In the diagnostic composition according to the present invention, the agent for measuring the mRNA expression level of the gene encoding interleukin 10 receptor, interleukin 22 receptor, interleukin 22, interleukin 28A, interleukin 28B isoform 1, interleukin 28B isoform 2, interleukin 29 or interferon lambda receptor 1 isoform 1 may comprise at least one selected from the group consisting of primers, probes and antisense nucleotides, which bind specifically to the mRNA of the gene encoding each of the interleukin 10 receptor, interleukin 22 receptor, interleukin 22, interleukin 28A, interleukin 28B isoform 1, interleukin 28B isoform 2, interleukin 29 and interferon lambda receptor 1 isoform 1. Since information on the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 according to the present invention is known, any person skilled in the art can easily design primers, probes, or antisense nucleotides, which specifically bind to the mRNA of the gene encoding the protein, based on the information.

As used herein, the expression "measuring the mRNA expression level" refers to measuring the amount of mRNA by a process of determining the presence or expression level of the mRNA of the gene, which encodes interleukin 10 receptor, interleukin 22 receptor, interleukin 22, interleukin 28A, interleukin 28B isoform 1, interleukin 28B isoform 2, interleukin 29 or interferon lambda receptor 1 isoform 1, in a biological sample in order to diagnose diseases comprising pancreatic cancer according to the present invention. Analysis methods for measurement of the mRNA expression level comprise, but are not limited to, reverse transcription polymerase chain reaction (RT-PCR), competitive reverse transcription polymerase chain reaction (competitive RT-PCR), real-time RT-PCR, RNase protection assay (RPA), Northern blotting, DNA chip assay, and the like.

As used herein, the term "primer" refers to a fragment that recognizes a target gene sequence, and comprises a pair of forward and reverse primers, but is preferably a pair of primers providing analysis results with specificity and sensitivity. When the nucleic acid sequence of the primer is a sequence inconsistent with the non-target sequence present in the sample, and thus is a primer that amplifies only the target gene sequence containing the complementary primer binding site without inducing non-specific amplification, high specificity can be imparted.

As used herein, the term "probe" refers to a substance capable of binding specifically to a target substance to be detected in the sample, and refers to a substance capable of specifically detecting the presence of the target substance in the sample through the binding. The type of probe is not particularly limited so long as it is commonly used in the art. The probe is preferably PNA (peptide nucleic acid), LNA (locked nucleic acid), a peptide, a polypeptide, a protein, an RNA or a DNA, most preferably PNA. More specifically, the probe is a biomolecule derived from an organism or an analogue thereof, or is produced in vitro. For example, the probe comprises an enzyme, a protein, an antibody, a microorganism, an animal and/or plant cell and organ, DNA and RNA. The DNA may comprise cDNA, genomic DNA, or an oligonucleotide, the RNA may comprise genomic RNA, mRNA or an oligonucleotide, and examples of the protein comprise antibodies, antigens, enzymes, peptides, and the like.

As used herein, the term "LNA (locked nucleic acid)" means a nucleic acid analogue containing a 2'-0 or 4'-C methylene bridge [J Weiler, J Hunziker and J Hall Gene Therapy (2006) 13, 496.502]. LNA nucleosides comprise the common bases of DNA and RNA, and can form base pairs according to the Watson-Crick base-pair rule. However, LNA fails to form an ideal shape in the Watson-Crick bond due to "locking" of the molecule attributable to the methylene bridge. When LNA is incorporated in a DNA or RNA oligonucleotide, it can more rapidly pair with a complementary nucleotide chain, thus increasing the stability of the double strand.

As used herein, the term "antisense" means an oligomer that has a nucleotide sequence and a backbone between subunits, wherein an antisense oligomer is hybridized with the target sequence in the RNA by Watson-Crick base pairing to allow the formation of the mRNA and RNA:oligomer heterodimers in the target sequence. The oligomer may have an accurate or approximate sequence complementarity to the target sequence.

In another aspect, the present invention is directed to a kit for diagnosing disease comprising the diagnostic composition according to the present invention.

Examples of the disease whose onset or likelihood of onset may be predicted using the diagnostic kit of the present invention comprise, but are not limited to, pancreatic disease, autoimmune disease, allergy, Grave's disease, Hashimoto's thyroiditis, autoimmune lymphoproliferative syndrome, myasthenia gravis, Kawasaki disease, and psoriasis.

In particular, in the present invention, the diagnostic kit may be used to diagnose a pancreatic disease such as pancreatic cancer or pancreatitis, more preferably pancreatic cancer. Specifically, when the kit of the present invention is used, it may detect or diagnose pancreatic cancer by distinguishing between a normal group and a pancreatic cancer patient group. In addition, the kit may selectively detect or diagnose pancreatic cancer by distinguishing from other types of cancer such as lung cancer or colorectal cancer.

In the present invention, the kit may be an RT-PCR kit, a DNA chip kit, an ELISA kit, a protein chip kit, a rapid kit, or a multiple-reaction monitoring (MRM) kit, but is not limited thereto.

The kit for diagnosing disease according to the present invention may further comprise one or more other component compositions, solutions or devices suitable for the analysis method.

For example, the diagnostic kit may further comprise essential elements required for performing reverse transcription polymerase chain reaction. The RT-PCR kit comprises a primer pair specific for the gene encoding the marker protein. The primer is an oligonucleotide having a sequence specific for the nucleic acid sequence of the gene, and may have a length of about 7 bp to 50 bp, more preferably about 10 bp to 30 bp. The kit may also comprise a primer specific for the nucleic acid sequence of the control gene. In addition, the RT-PCR kit may comprise test tubes or other appropriate containers, reaction buffers (at various pHs and magnesium concentrations), deoxynucleotides (dNTPs), enzymes such as Taq-polymerase and reverse transcriptase, DNase and/or RNase inhibitors, DEPC water, sterile water, and the like.

In addition, the diagnostic kit of the present invention may comprise essential elements required for performing DNA chip assay. The DNA chip kit may comprise a substrate to which a cDNA or oligonucleotide corresponding to a gene or a fragment thereof is attached, and a reagent, an agent, an enzyme, and the like for producing a fluorescent-labeled probe. The substrate may also comprise a cDNA or oligonucleotide corresponding to a control gene or a fragment thereof.

In addition, the diagnostic kit of the present invention can comprise essential elements required for performing ELISA. The ELISA kit comprises an antibody specific for the protein. The antibody has high specificity and affinity for the marker protein and little cross-reactivity with other proteins, and is a monoclonal antibody, a polyclonal antibody or a recombinant antibody. The ELISA kit may also comprise antibodies specific for the control protein. In addition, the ELISA kit may comprise reagents capable of detecting the bound antibody, such as a labeled secondary antibody, chromophores, an enzyme (e.g., conjugated to an antibody) and substrates thereof, or other substances that can bind to the antibody.

In still another aspect, the present invention is directed to a method for providing information for diagnosis of disease, the method comprising a step of measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, in a biopsy isolated from a subject of interest.

Examples of the disease whose onset or likelihood of onset may be predicted in the method for providing information according to the present invention comprise, but are not limited to, pancreatic disease, autoimmune disease, allergy, Grave's disease, Hashimoto's thyroiditis, autoimmune lymphoproliferative syndrome, myasthenia gravis, Kawasaki disease, and psoriasis.

According to the present invention, it is possible to relatively accurately diagnose and predict the onset or likelihood of onset of the aforementioned diseases, particularly pancreatic diseases such as pancreatic cancer or pancreatitis, more preferably pancreatic cancer.

According to the method for providing information according to the present invention, it is possible to detect or diagnose pancreatic cancer by distinguishing between a normal group and a pancreatic cancer patient group. In addition, it is possible to selectively detect or diagnose pancreatic cancer by distinguishing pancreatic cancer from other types of cancer such as lung cancer or colorectal cancer.

In the present invention, the specific types of the autoimmune disease and allergy overlap with those described in the diagnostic composition of the present invention, and thus the description thereof will be omitted.

As used herein, the term "subject of interest" refers to a subject whose onset of the above-listed diseases is uncertain and who has a high likelihood of onset.

As used herein, the term "biopsy" isolated from the subject refers to tissue for histopathological examination, which is collected by inserting a hollow needle or the like into an *in vivo* organ without cutting the skin of a patient having a high likelihood of onset. Examples of the biopsy comprise a patient's tissue, cells, blood, serum, plasma, saliva or sputum. The biopsy is preferably a patient's blood, serum or plasma, more preferably mononuclear cells, granulocytes or exosomes isolated from the liquid biopsy.

In conventional methods for measuring the expression level of a biomarker for diagnosis of pancreatic disease, cells are isolated from the tissue (for example, pancreatic tissue) in which it is predicted that a disease will develop, and the expression level of the biomarker in the cells is measured. However, according to the present invention, it is possible to rapidly, simply and very accurately diagnose the onset of various diseases such as pancreatic cancer and the likelihood of onset thereof by measuring the expression level of the disease biomarker according to the present invention in the mononuclear cells, granulocytes or exosomes contained in the blood, serum or plasma isolated from the subject of interest.

Specifically, when the expression level of the marker according to the present invention in mononuclear cells, granulocytes or exosomes is measured as described above, the onset of disease or the likelihood of the onset thereof may be rapidly and simply determined, since there is no need for invasive procedures, for example, comprising performing laparotomy on a patient and isolating tissue cells from tissue (for example, pancreatic tissue), and about 5 minutes or less is required to obtain a biopsy containing mononuclear cells, granulocytes or exosomes and about 2 hours or less to measure the expression levels of various disease biomarkers according to the present invention from the mononuclear cells, granulocytes or exosomes.

The method for providing information according to the present invention may preferably comprise a step of measuring the expression level of interleukin 10 receptor as a biomarker or the mRNA expression level of a gene encoding the interleukin 10 receptor, in the biopsy isolated as described above. In addition, the method for providing information according to the present invention may further comprise a step of measuring the expression level of at least one protein selected from the group consisting of interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, from the biopsy, in order to increase accuracy in diagnosing the disease.

The method for providing information according to the present invention may preferably comprise a step of measuring the expression level of each of the interleukin 10 receptor and interferon lambda receptor 1 isoform 1 proteins from the biopsy.

The method for providing information according to the present invention may preferably comprise a step of measuring the mRNA expression level of a gene encoding each of the interleukin 10 receptor and interferon lambda receptor 1 isoform 1 proteins from the biopsy.

The method for providing information according to the present invention may preferably comprise a step of measuring the expression level of each of the interleukin 10 receptor and interleukin 22 receptor proteins from the biopsy.

The method for providing information according to the present invention may preferably comprise a step of measuring the mRTN expression level of a gene encoding each of the interleukin 10 receptor and interleukin 22 receptor proteins from the biopsy.

The method for providing information according to the present invention may preferably comprise a step of measuring the expression level of each of the interleukin 10 receptor and interleukin 22 proteins from the biopsy.

The method for providing information according to the present invention may preferably comprise a step of measuring the mRNA expression level of a gene encoding each of the interleukin 10 receptor and interleukin 22 proteins from the biopsy.

The method for providing information according to the present invention may preferably comprise a step of measuring the expression level of each of the interleukin 10 receptor, interleukin 22 receptor and interleukin 22 proteins from the biopsy.

The method for providing information according to the present invention preferably comprises a step of measuring the mRNA expression level of a gene encoding each of the interleukin 10 receptor, interleukin 22 receptor and interleukin 22 proteins from the biopsy.

The method for providing information according to the present invention preferably comprises a step of measuring the expression level of the interleukin 28A, interleukin 28B isoform 1 or interleukin 28B isoform 2 protein from the biopsy.

The method for providing information according to the present invention preferably comprises a step of measuring the mRNA expression level of a gene encoding the interleukin 28A, interleukin 28B isoform 1 or interleukin 28B isoform 2 protein from the biopsy.

In the present invention, the agent for measuring the expression level of Interleukin 10 receptor, interleukin 22 receptor, interleukin 22, interleukin 28A, interleukin 28B isoform 1, interleukin 28B isoform 2, interleukin 29 or interferon lambda receptor 1 isoform 1 is not particularly limited, but preferably comprises at least one selected from the group consisting of antibodies, oligopeptides, ligands, PNAs (peptide nucleic acids) and aptamers, which bind specifically to each of the interleukin 10 receptor, interleukin 22 receptor, interleukin 22, interleukin 28A, interleukin 28B isoform 1, interleukin 28B isoform 2, interleukin 29, and interferon lambda receptor 1 isoform 1.

In the present invention, preferably, the expression level of the interleukin 10 receptor, interleukin 22 receptor, interleukin 22, interleukin 28A, interleukin 28B isoform 1, interleukin 28B isoform 2, interleukin 29 or interferon lambda receptor 1 isoform 1 may be measured and compared using an antibody that binds specifically to the interleukin 10 receptor, interleukin 22 receptor, interleukin 22, interleukin 28A, interleukin 28B isoform 1, interleukin 28B isoform 2, interleukin 29 or interferon lambda receptor 1 isoform 1. This may be carried out using a method of allowing the antibody and the corresponding protein in the biological sample to form an antigen-antibody complex and detecting the antigen-antibody complex.

Meanwhile, methods for measuring or comparatively analyzing the expression level of the protein comprise, but are not limited to, protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, and enzyme-linked immunosorbent assay (ELISA).

In addition, in the present invention, the agent for measuring the mRNA expression level of the gene encoding interleukin 10 receptor, interleukin 22 receptor, interleukin 22, interleukin 28A, interleukin 28B isoform 1, interleukin 28B isoform 2, interleukin 29 or interferon lambda receptor 1 isoform 1 may comprise at least one selected from the group consisting of primers, probes and antisense nucleotides, which bind specifically to the mRNA of the gene encoding each of the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 proteins. Since information on the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 according to the present invention is known, any person skilled in the art can easily design primers, probes, or antisense nucleotides, which specifically bind to the mRNA of the gene encoding the protein, based on the information.

In the present invention, measurement or comparison of the mRNA expression level of the gene encoding the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 may be performed using, but not limited to, reverse transcription polymerase chain reaction (RT-PCR), competitive reverse transcription polymerase chain reaction (competitive RT-PCR), real-time RT-PCR, RNase protection assay (RPA), Northern blotting, or DNA chip assay. Through these measurement methods, the mRNA expression level in the normal control group and the mRNA expression level in the subject in need of diagnosis as to the onset of disease may be determined, and the likelihood of the onset of a disease such as pancreatic cancer may be diagnosed or predicted through comparison between these expression levels.

When the expression level of interleukin 10 receptor or the mRNA expression level of the gene encoding interleukin 10 receptor, measured from the biopsy isolated from the subject of interest, is higher than the expression level in the normal control group, the likelihood of the onset of a disease, particularly a pancreatic disease, may be determined to be high. More preferably, when the expression level measured from the biopsy is at least 2 times, at least 3 times, at least 4 times, at least 5 times, at least 6 times, at least 7 times, at least 8 times or at least 9 times higher than the expression level in the normal control group, the likelihood of the onset of a pancreatic disease may be determined to be high.

In the present invention, when the expression level of interleukin 10 receptor measured from the biopsy isolated from the subject of interest, for example, the number of cells expressing interleukin 10 receptor, is 3 to 20 times, 4 to 18 times, 5 to 13 times, 7 to 11 times, or 8 to 10 times larger than that in the normal control group, the likelihood of the onset of a pancreatic disease, particularly pancreatic cancer, may be determined to be high. Preferably, when the expression level of interleukin 10 receptor measured from the biopsy isolated from the subject of interest is 8 to 10 times higher than the expression level in the normal control group, the likelihood of the onset of a pancreatic disease, particularly pancreatic cancer, may be determined to be high.

In addition, in the present invention, when the mRNA expression level of the gene encoding interleukin 10 receptor, measured from the biopsy isolated from the subject of interest, is 2 to 20 times, 2 to 15 times, 2.5 to 15 times, 2 to 10 times or 2.5 to 10 times higher than the expression level in the normal control group, the likelihood of onset of a pancreatic disease, particularly pancreatic cancer, may be determined to be high. Preferably, when the mRNA expression level of the gene encoding interleukin 10 receptor, measured from the biopsy isolated from the subject of interest, is 2.5 to 10 times higher than the expression level in the normal control group, the likelihood of the onset of a pancreatic disease, particularly pancreatic cancer, may be determined to be high.

In addition, in the present invention, when the expression level of interleukin 22 receptor or the mRNA expression level of the gene encoding interleukin 22 receptor, measured from the biopsy isolated from the subject of interest, is higher than the expression level of the normal control group, the likelihood of the onset of a disease, particularly, a pancreatic disease, may be determined to be high. More preferably, when the expression level measured from the biopsy is at least 1.5 times, at least 2 times, at least 3 times, at least 4 times, at least 5 times or at least 6 times higher than the expression level in the normal control group, the likelihood of the onset of a pancreatic disease may be determined to be high.

In addition, in the present invention, when the expression level of interleukin 22 receptor measured from the biopsy isolated from the subject of interest, for example, the number of cells expressing interleukin 22 receptor, is 2 to 10 times, 3 to 9 times, 4 to 8 times, 5 to 7 times, or 6 to 7 times larger than that in a normal control group, the likelihood of onset of a pancreatic disease, particularly pancreatic cancer, may be determined to be high. In addition, in the present invention, when the mRNA expression level of the gene encoding interleukin 22 receptor, measured from the biopsy isolated from the subject of interest, is 1.5 to 10 times, 1.5 to 9 times, 1.5 to 8 times, or 2 to 7 times higher than the expression level in the normal control group, the likelihood of the onset of a pancreatic disease, particularly pancreatic cancer, may be determined to be high.

In the present invention, when the expression level of interleukin 22 or the mRNA expression level of the gene encoding interleukin 22, measured from the biopsy isolated from the subject of interest, is higher than the expression level in the normal control group, the likelihood of the onset of a disease, particularly a pancreatic disease, may be determined to be high. More preferably, when the expression level measured from the biopsy is at least 2 times, at least 3 times, at least 4 times, at least 5 times or at least 6 times higher than the expression level in the normal control group, the likelihood of the onset of a pancreatic disease may be determined to be high.

In addition, in the present invention, when the expression level of interleukin 22 measured from the biopsy isolated from the subject of interest, for example, the number of cells expressing interleukin 22, is 3 to 16 times, 4 to 15 times, 4 to 13 times, 5 to 10 times, 5 to 8 times, 5 to 7 times, or 6 to 7 times greater than that in the normal control group, the likelihood of onset of a pancreatic disease, particularly pancreatic cancer, may be determined to be high.

In addition, in the present invention, when the mRNA expression level of the gene encoding interleukin 22, measured from the biopsy isolated from the subject of interest, is 2 to 15 times, 2.5 to 15 times, 3 to 13 times, 3.5 to 13 times, 4 to 13 times, or 4 to 10 times higher than that in the normal control group, the likelihood of onset of a pancreatic disease, particularly pancreatic cancer, may be determined to be high.

In the present invention, when the expression level of interleukin 29 or the mRNA expression level of the gene encoding interleukin 29, measured from the biopsy isolated from the subject of interest, is higher than the expression level in the normal control group, the likelihood of the onset of a disease, particularly a pancreatic disease, may be determined to be high. More preferably, when the expression level measured from the biopsy is at least 2 times, at least 3 times, at least 4 times, at least 5 times or at least 6 times higher than the expression level in the normal control group, the likelihood of the onset of a pancreatic disease may be determined to be high.

In addition, in the present invention, when the expression level of interleukin 29 measured from the biopsy isolated from the subject of interest, for example, the number of cells expressing interleukin 29, is 3 to 16 times, 4 to 15 times, 4 to 13 times, 5 to 10 times, 5 to 8 times, 5 to 7 times, or 6 to 7 times higher than that in the normal control group, the likelihood of onset of a pancreatic disease, particularly pancreatic cancer, may be determined to be high.

In addition, in the present invention, when the expression level of the mRNA expression level of the gene encoding interleukin 29, measured from the biopsy isolated from the subject of interest, is 2 to 15 times, 2.5 to 15 times, 3 to 13 times, 3.5 to 13 times, 4 to 13 times, or 4 to 10 times higher than that in the normal control group, the likelihood of onset of a pancreatic disease, particularly pancreatic cancer, may be determined to be high.

In the present invention, when the expression level of interferon lambda receptor 1 isoform 1 or the mRNA expression level of the gene encoding interferon lambda receptor 1 isoform 1, measured from the biopsy isolated from the subject of interest, is higher than the expression level in the normal control group, the likelihood of the onset of a disease, particularly, a pancreatic disease, may be determined to be high. More preferably, when the expression level measured from the biopsy is at least 1.5 times, at least 2 times, at least 3 times, at least 4 times, at least 5 times or at least 6 times higher than the expression level in the normal control group, the likelihood of the onset of a pancreatic disease may be determined to be high.

In addition, in the present invention, when the expression level of interferon lambda receptor 1 isoform 1 measured from the biopsy isolated from the subject of interest, for example, the number of cells expressing interferon lambda receptor 1 isoform 1, is 3 to 16 times, 4 to 15 times, 4 to 13 times, 5 to 10 times, 5 to 8 times, 5 to 7 times, or 6 to 7 times that higher than that in the normal control group, the likelihood of onset of a pancreatic disease, particularly pancreatic cancer, may be determined to be high.

In addition, in the present invention, when the expression level of the mRNA expression level of the gene encoding interferon lambda receptor 1 isoform 1, measured from the biopsy isolated from the subject of interest, is 1.5 to 15 times, 2 to 15 times, 2.5 to 15 times, 3 to 13 times, 3.5 to 13 times, 4 to 13 times, or 4 to 10 times higher than that in the normal control group, the likelihood of the onset of a pancreatic disease, particularly pancreatic cancer, may be determined to be high.

According to the present invention, the likelihood of onset of pancreatic cancer may be diagnosed with high accuracy by measuring the expression level of a marker of interleukin 10 receptor, preferably markers of interleukin 10 receptor and interleukin 22 receptor, more preferably markers of interleukin 10 receptor, interleukin 22 receptor and at least one selected from among interleukin 22, interleukin 28A, interleukin 28B isoform 1, interleukin 28B isoform 2, interleukin 29 and interferon lambda receptor 1 isoform 1, as described above.

Furthermore, the method of the present invention may further comprise subjecting the subject of interest to appropriate treatment such as administration of a drug for the disease (such as an anti-cancer drug for pancreatic cancer), gene therapy, radiotherapy or immunotherapy, when the likelihood of onset of a disease, particularly a pancreatic disease, preferably pancreatic cancer, is predicted or diagnosed to be high by measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of the gene encoding the protein, in the biopsy isolated from the subject of interest.

In yet another aspect, the present invention is directed to a method for providing information for predicting the likelihood of recurrence of disease comprising a step of measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, in a biopsy isolated from a patient who has received treatment.

Examples of the disease whose recurrence or likelihood of recurrence may be predicted in the method for providing information according to the present invention comprise, pancreatic disease, autoimmune disease, allergy, Grave's disease, Hashimoto's thyroiditis, autoimmune lymphoproliferative syndrome, myasthenia gravis, Kawasaki disease, and psoriasis.

According to the present invention, it is possible to relatively accurately diagnose and predict the likelihood of recurrence of the aforementioned diseases, particularly pancreatic diseases such as pancreatic cancer or pancreatitis, more preferably pancreatic cancer.

According to the method for providing information according to the present invention, it is possible to detect or diagnose pancreatic cancer patients by distinguishing between a patient group with a high likelihood of recurrence and a patient group with a low likelihood of recurrence after treatment.

In the present invention, the patient who has received treatment refers to a patient who has received one or more of surgery, chemotherapy, gene therapy, radiotherapy and immunotherapy due to the onset of the above-listed diseases or the high likelihood of onset thereof, for example, a patient who received treatment before at least 2 months, at least 3 month or at least 6 months.

Previously, early diagnosis of pancreatic cancer was difficult, and it was almost impossible to diagnose the likelihood of recurrence of pancreatic cancer after treatment. However, according to the present invention, it is possible to rapidly, simply and very accurately predict the likelihood of recurrence of pancreatic cancer by measuring the expression level of the disease biomarker according to the present invention in the mononuclear cells, granulocytes or exosomes contained in the blood, serum or plasma which is a biopsy isolated from a patient (subject of interest) who has received treatment, preferably a liquid biopsy.

The method for providing information according to the present invention may comprise a step of measuring the mRNA expression level of a gene encoding interleukin 10 receptor or interleukin 10 receptor as a biomarker from the biopsy isolated as described above. In addition, the method for providing information according to the present invention may further comprise a step of measuring the expression level of at least one protein selected from the group consisting of interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, from the biopsy, in order to further increase accuracy in predicting the likelihood of recurrence of the disease.

The method for providing information according to the present invention preferably comprises a step of measuring the expression level of each of the interleukin 10 receptor and interferon lambda receptor 1 isoform 1 proteins from the biopsy.

The method for providing information according to the present invention preferably comprises a step of measuring the mRNA expression level of a gene expressing each of the interleukin 10 receptor and interferon lambda receptor 1 isoform 1 proteins from the biopsy.

The method for providing information according to the present invention preferably comprises a step of measuring the expression level of each of the interleukin 10 receptor and interleukin 22 receptor proteins from the biopsy.

The method for providing information according to the present invention preferably comprises a step of measuring the mRNA expression level of a gene encoding each of the interleukin 10 receptor and interleukin 22 receptor proteins from the biopsy.

The method for providing information according to the present invention preferably comprises a step of measuring the expression level of each of the interleukin 10 receptor and interleukin 22 proteins from the biopsy.

The method for providing information according to the present invention preferably comprises a step of measuring the mRNA expression level of a gene encoding each of the interleukin 10 receptor and interleukin 22 proteins from the biopsy.

The method for providing information according to the present invention preferably comprises a step of measuring the expression of each of the interleukin 10 receptor, interleukin 22 receptor and interleukin 22 proteins from the biopsy.

The method for providing information according to the present invention preferably comprises a step of measuring the mRNA expression level of a gene encoding each of the interleukin 10 receptor, interleukin 22 receptor and interleukin 22 proteins from the biopsy.

The method for providing information according to the present invention preferably comprises a step of measuring the expression level of interleukin 28A, interleukin 28B isoform 1 or interleukin 28B isoform 2 protein from the biopsy.

The method for providing information according to the present invention preferably comprises a step of measuring the mRNA expression level of a gene encoding interleukin 28A, interleukin 28B isoform 1 or interleukin 28B isoform 2 protein from the biopsy.

In the present invention, the agent for measuring the expression level of interleukin 10 receptor, interleukin 22 receptor, interleukin 22, interleukin 28A, interleukin 28B isoform 1, interleukin 28B isoform 2, interleukin 29 or interferon lambda receptor 1 isoform 1 is not particularly limited, but preferably comprises at least one selected from the group consisting of antibodies, oligopeptides, ligands, PNAs (peptide nucleic acids) and aptamers, which bind specifically to each of the interleukin 10 receptor, interleukin 22 receptor, interleukin 22, interleukin 28A, interleukin 28B isoform 1, interleukin 28B isoform 2, interleukin 29, and interferon lambda receptor 1 isoform 1.

In the present invention, preferably, the expression level of the interleukin 10 receptor, interleukin 22 receptor, interleukin 22, interleukin 28A, interleukin 28B isoform 1, interleukin 28B isoform 2, interleukin 29 or interferon lambda receptor 1 isoform 1 may be measured and compared using an antibody that binds specifically to the interleukin 10 receptor, interleukin 22 receptor, interleukin 22, interleukin 28A, interleukin 28B isoform 1, interleukin 28B isoform 2, interleukin 29 or interferon lambda receptor 1 isoform 1. This may be carried out using a method of allowing the antibody and the corresponding protein in the biological sample to form an antigen-antibody complex and detecting the antigen-antibody complex.

Meanwhile, in the present invention, methods for measuring or comparatively analyzing the expression level of the protein comprise, but are not limited to, protein chip analysis, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, and enzyme-linked immunosorbent assay (ELISA).

In addition, in the present invention, the agent for measuring the mRNA expression level of the gene encoding interleukin 10 receptor, interleukin 22 receptor, interleukin 22, interleukin 28A, interleukin 28B isoform 1, interleukin 28B isoform 2, interleukin 29 or interferon lambda receptor 1 isoform 1 may comprise at least one selected from the group consisting of primers, probes and antisense nucleotides, which bind specifically to the mRNA of the gene encoding each of the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 proteins. Since information on the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 according to the present invention is known, any person skilled in the art can easily design primers, probes, or antisense nucleotides, which specifically bind to the mRNA of the gene encoding the protein, based on the information.

In the present invention, measurement or comparison of the mRNA expression level of the gene encoding the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 may be performed using, but not limited to, reverse transcription polymerase chain reaction (RT-PCR), competitive reverse transcription polymerase chain reaction (competitive RT-PCR), real-time RT-PCR, RNase protection assay (RPA), Northern blotting, or DNA chip assay. Through these measurement methods, the mRNA expression level in the normal control group and the mRNA expression level in the subject in need of diagnosis as to the onset of disease may be determined, and the likelihood of onset of a disease such as pancreatic cancer may be diagnosed or predicted through comparison between these expression levels.

In the present invention, when the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, measured from the biopsy isolated from the patient who has received treatment, is higher than the expression level in the normal control group, the likelihood of recurrence of a disease, particularly pancreatic cancer, may be determined to be high.

In the present invention, when the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, measured from the biopsy isolated from the patient who has received treatment, is higher than the expression level in the same patient before treatment, the likelihood of recurrence of a disease, particularly pancreatic cancer, may be determined to be high.

According to the present invention, the likelihood of recurrence of pancreatic cancer may be predicted with high accuracy by measuring the expression level of a marker of interleukin 10 receptor, preferably markers of interleukin 10 receptor and interleukin 22 receptor, more preferably markers of interleukin 10 receptor, interleukin 22 receptor and at least one selected from among interleukin 22, interleukin 28A, interleukin 28B isoform 1, interleukin 28B isoform 2, interleukin 29 and interferon lambda receptor 1 isoform 1, as described above.

Furthermore, the method of the present invention may further comprise subjecting the subject of interest to appropriate treatment such as administration of a drug for the disease (such as an anti-cancer drug for pancreatic cancer), gene therapy, radiotherapy or immunotherapy, when the likelihood of recurrence of a disease, particularly pancreatic cancer, is predicted or diagnosed to be high by measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of the gene encoding the protein, in the biopsy isolated from the subject of interest.

In still yet another aspect, the present invention is directed to a method for screening a drug for disease treatment comprising steps of:
(a) measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, in a biopsy isolated from a disease patient;
(b) administering a candidate drug to the patient; and
(c) measuring the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of the gene encoding the protein, in a biopsy isolated from the patient after administration of the candidate drug.

Examples of the disease for which the efficacy or sensitivity of the drug may be predicted in the drug screening method of the present invention comprise, but are not limited to, pancreatic disease, autoimmune disease, allergy, Grave's disease, Hashimoto's thyroiditis, autoimmune lymphoproliferative syndrome, myasthenia gravis, Kawasaki disease, and psoriasis.

According to the present invention, it is possible to relatively accurately diagnose and predict the efficacy or sensitivity of a therapeutic drug for the aforementioned diseases, particularly pancreatic diseases such as pancreatic cancer or pancreatitis, more preferably pancreatic cancer.

In the present invention, the specific types of the autoimmune disease and allergy overlap with those described in the diagnostic composition of the present invention, and thus description thereof will be omitted below.

In addition, the biopsy isolated from the disease patient is tissue for histopathological examination, which is collected by inserting a hollow needle or the like into an *in vivo* organ without cutting the skin of the patient having a high likelihood of onset of the disease. Examples of the biopsy comprise a patient's tissue, cells, blood, serum, plasma, saliva or sputum. The biopsy is preferably the patient's blood, serum or plasma, more preferably mononuclear cells, granulocytes or exosomes isolated from the blood, serum or plasma.

The drug screening method of the present invention preferably comprises a step of measuring the expression level of interleukin 10 receptor as a biomarker or the mRNA expression level of a gene encoding the interleukin 10 receptor, in the biopsy isolated from the patient in each of steps (a) and (c) . In addition, the drug screening method of the present invention may further comprise a step of measuring the expression level of at least one protein selected from the group consisting of interleukin 22 receptor, interleukin 22, interleukin 28A, interleukin 28B isoform 1, interleukin 28B isoform 2, interleukin 29 and interferon lambda receptor 1 isoform 1 or the mRNA expression level of a gene encoding the protein, in addition to measuring the expression level of interleukin 10 receptor or the mRNA expression level of a gene encoding the interleukin 10 receptor, in the biopsy isolated from the patient in each of steps (a) and (c), in order to further increase accuracy in predicting the sensitivity of the therapeutic drug to the disease.

The method for providing information according to the present invention preferably comprises a step of measuring the expression level of each of the interleukin 10 receptor and interferon lambda receptor 1 isoform 1 proteins from the biopsy.

The method for providing information according to the present invention preferably comprises a step of measuring the mRNA expression level of a gene encoding each of the interleukin 10 receptor and interferon lambda receptor 1 isoform 1 proteins from the biopsy.

The method for providing information according to the present invention preferably comprises a step of measuring the expression level of each of the interleukin 10 receptor and interleukin 22 receptor proteins from the biopsy.

The method for providing information according to the present invention preferably comprises a step of measuring the mRNA expression level of a gene encoding each of the interleukin 10 receptor and interleukin 22 receptor proteins from the biopsy.

The method for providing information according to the present invention preferably comprises a step of measuring the expression level of each of the interleukin 10 receptor and interleukin 22 proteins from the biopsy.

The method for providing information according to the present invention preferably comprises a step of measuring the mRNA expression level of a gene encoding each of the interleukin 10 receptor and interleukin 22 proteins from the biopsy.

The method for providing information according to the present invention preferably comprises a step of measuring the expression level of each of the interleukin 10 receptor, interleukin 22 receptor and interleukin 22 proteins from the biopsy.

The method for providing information according to the present invention preferably comprises a step of measuring the mRNA expression level of a gene encoding each of the interleukin 10 receptor, interleukin 22 receptor and interleukin 22 proteins from the biopsy.

The method for providing information according to the present invention preferably comprises a step of measuring the expression level of the interleukin 28A, interleukin 28B isoform 1 or interleukin 28B isoform 2 protein from the biopsy.

The method for providing information according to the present invention preferably comprises a step of measuring the mRNA expression level of a gene encoding the interleukin 28A, interleukin 28B isoform 1 or interleukin 28B isoform 2 protein from the biopsy.

In the present invention, the agent for measuring the expression level of interleukin 10 receptor, interleukin 22 receptor, interleukin 22, interleukin 28A, interleukin 28B isoform 1, interleukin 28B isoform 2, interleukin 29 or interferon lambda receptor 1 isoform 1 is not particularly limited, but preferably comprises at least one selected from the group consisting of antibodies, oligopeptides, ligands, PNAs (peptide nucleic acids) and aptamers, which bind specifically to each of the interleukin 10 receptor, interleukin 22 receptor, interleukin 22, interleukin 28A, interleukin 28B isoform 1, interleukin 28B isoform 2, interleukin 29, and interferon lambda receptor 1 isoform 1.

In the present invention, preferably, the expression level of the interleukin 10 receptor, interleukin 22 receptor, interleukin 22, interleukin 28A, interleukin 28B isoform 1, interleukin 28B isoform 2, interleukin 29 or interferon lambda receptor 1 isoform 1 may be measured and compared using an antibody that binds specifically to the interleukin 10 receptor, interleukin 22 receptor, interleukin 22, interleukin 28A, interleukin 28B isoform 1, interleukin 28B isoform 2, interleukin 29 or interferon lambda receptor 1 isoform 1. This may be carried out using a method of allowing the antibody and the corresponding protein in the biological sample to form an antigen-antibody complex and detecting the antigen-antibody complex.

Meanwhile, methods for measuring or comparatively analyzing the expression level of the protein comprise, but are not limited to, protein chip assay, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) assay, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, and enzyme-linked immunosorbent assay (ELISA).

In addition, in the present invention, the agent for measuring the mRNA expression level of the gene encoding interleukin 10 receptor, interleukin 22 receptor, interleukin 22, interleukin 28A, interleukin 28B isoform 1, interleukin 28B isoform 2, interleukin 29 or interferon lambda receptor 1 isoform 1 may comprise at least one selected from the group consisting of primers, probes and antisense nucleotides, which bind specifically to the mRNA of the gene encoding each of the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 proteins. Since information on the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 according to the present invention is known, any person skilled in the art can easily design primers, probes, or antisense nucleotides, which specifically bind to the mRNA of the gene encoding the protein, based on the information.

In the present invention, measurement or comparison of the mRNA expression level of the gene encoding the interleukin 10 receptor, interleukin 22 receptor, interleukin 22, interleukin 28A, interleukin 28B isoform 1, interleukin 28B isoform 2, interleukin 29 and interferon lambda receptor 1 isoform 1 may be performed using, but not limited to, reverse transcription polymerase chain reaction (RT-PCR), competitive reverse transcription polymerase chain reaction (competitive RT-PCR), real-time RT-PCR, RNase protection assay (RPA), Northern blotting, or DNA chip assay. Through these measurement methods, the mRNA expression level in the normal control group and the mRNA expression level in the subject in need of diagnosis as to the onset of disease may be determined, and the likelihood of onset of a disease such as pancreatic cancer may be diagnosed or predicted through comparison between these expression levels.

In the present invention, the method of the present invention may further comprise a step of selecting the candidate drug as a therapeutic drug suitable for treatment of the disease, when the expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or the mRNA expression level of a gene encoding the protein, measured in step (c), is lower than the expression level of the same biomarker, measured in step (a) .

In a further aspect, the present invention is directed to a composition for use in diagnosis of pancreatic disease comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein.

In further another aspect, the present disclosure is directed to a method for diagnosing pancreatic disease comprising a step of: measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, in a biopsy isolated from a subject of interest.

In still another further aspect, the present invention is directed to the use of a composition comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, for diagnosis of pancreatic disease.

In yet another further aspect, the present invention is directed to the use of a composition comprising an agent for measuring the expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or the mRNA expression level of a gene encoding the protein, in the manufacture of a medicament for diagnosis of pancreatic disease.

Hereinafter, the present invention will be described in more detail with reference to examples. It will be obvious to those skilled in the art that these examples serve merely to illustrate the present invention, and the scope of the present invention is not limited by these examples.

### [Preparation Example 1]

Histopaq 1077 was purchased from Sigma, and Brefeldin A was purchased from BioLegend. As antibodies, Alexa 647 anti-IL-22 and PE anti-mouse IL-10R were purchased from BioLegend, and PerCP mouse IL-22R was purchased from R & D. In addition, phosphate buffered saline (PBS) containing 0.5% bovine serum albumin (BSA) was used as a FACS buffer. A cell fixation buffer and a perm/wash buffer were also purchased from BioLegend.

### [Example 1]

### 1. Isolation of Peripheral Blood Mononuclear cells (PBMCs)

Blood was sampled from 3 pancreatic cancer patients, 3 pancreatitis patients, 3 normal control subjects, 3 lung cancer patients and 3 colorectal cancer patients and stored at room temperature. Since the isolation of mononuclear cells is difficult when blood viscosity is high, the blood samples were diluted at 1:1 in 1×PBS. Ficoll (Histopaque 1077, Sigma) was prepared in the same volume as blood in a 15-ml conical tube. Each of the blood samples was added onto Ficoll so as not to be mixed. The resulting blood samples were centrifuged at 400g for 30 minutes with minimum acceleration and deceleration. During this process, a white-cell (mononuclear cell) layer was separately collected, and then PBS was added thereto, followed by washing by centrifugation at 400g for 3 minutes. The washed cells were collected, resuspended in 50 µl FACS buffer, and then incubated on ice for 1 hour with a PE anti-mouse IL-10R antibody and a PerCP mouse IL-22R antibody. The cells were then washed twice with FACS buffer and resuspended in 500 µl FACS buffer.

### 2. Treatment with Brefeldin A

The collected cells were counted, seeded at 1×10⁶ cells/ml in RPMI 1640 medium containing 2% penicillin and streptomycin, treated with 1×Brefeldin A, and incubated for 6 hours.

### 3. Fixation, Permeabilization and Staining

The incubated cells were collected, washed twice with FACS buffer (0.5% BSA in PBS), and fixed with 500 µl of fixation buffer on ice for 30 minutes. Then, the cells were washed twice with FACS buffer, resuspended in 1 ml of perm buffer and then centrifuged twice for 20 minutes each time. Then, the cells were collected again, resuspended in 50 µl of perm buffer and then incubated on ice for 1 hour with Alexa 647 anti-IL-22 antibody. The cells were washed twice with FACS and resuspended in 500 µl of FACS buffer.

### 4. FACS Analysis

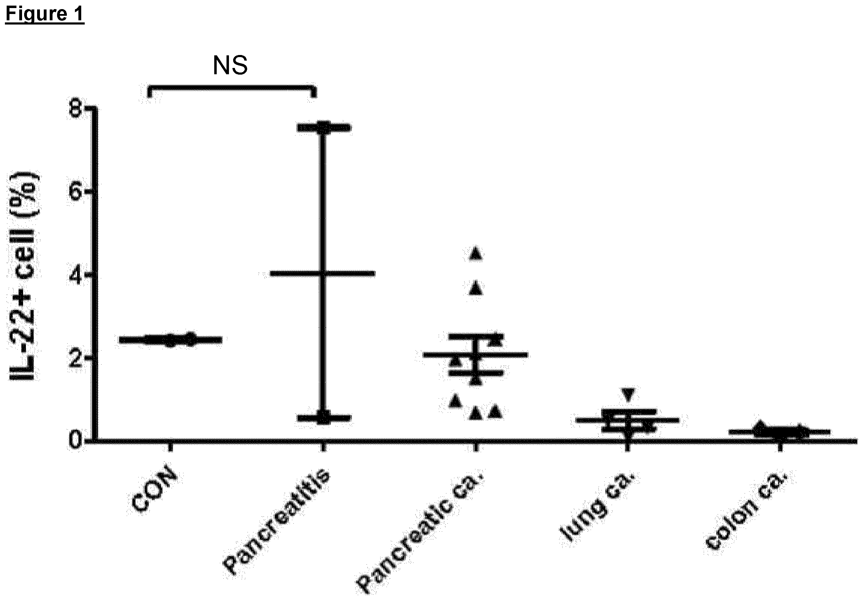

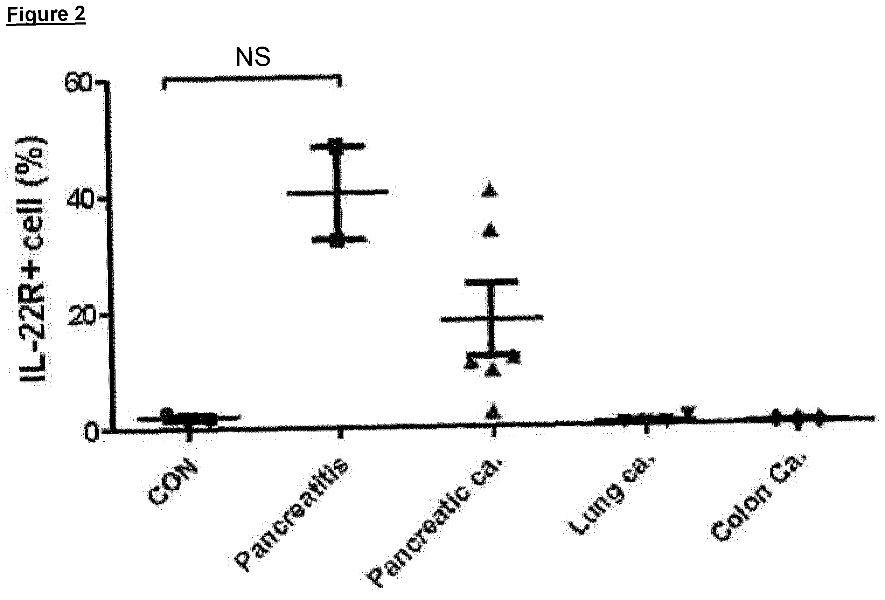

The number of cells expressing IL-10R, IL-22R or IL-22 was expressed as a percentage relative to the total number of mononuclear cells using FACSCalibur (BD Biosciences, San Jose, CA). The results are shown in Table 1 below and FIGS. 1 to 3. In Table 1 below, data are expressed as mean ± SD, and statistical analysis was performed by Kruskal-Wallis test using Dunn's post hoc analysis.

**[Table 1]**

| | Mean ± SD (%) | | | | |
|---|---|---|---|---|---|
| | Normal control | Pancreatitis | Pancreatic cancer | Lung cancer | Colorectal cancer |
| IL-22 | 2.44±0.02 | 4.06±3.49 | 2.25±1.31 | 0.51±0.43 | 0.21±0.01 |
| IL-22R | 2.16±0.49 | 40.30±8.00 | 14.20±14.88 | 0.60±0.55 | 0.37±0.13 |
| IL-10R | 2.59±0.75 | 38.40±7.80 | 23.20±8.65 | 7.13±4.93 | 2.01±0.89 |

As shown in Table 1 above and FIGS. 1 to 3, it could be confirmed that the expression levels of IL-22 in the pancreatitis patients and the pancreatic cancer patients were about 1.7 times and 0.9 times, respectively, the expression level of IL-22 in the normal control group, whereas the expression level of IL-10R, to which IL-22 binds, was about 14.8 times higher in the pancreatitis patients than in the normal control group, and was about 9.0 times higher in the pancreatic cancer patients than in the normal control group. However, it could be confirmed that the expression level of IL-10R was about 2.75 times higher in the lung cancer patients than in the normal control group, and the expression level of IL-10R in the colorectal cancer patients was about 0.78 times that in the normal control group.

In addition, it could be confirmed that the expression level of IL-22R was about 18.66 times higher in the pancreatitis patients than in the normal control group, and was about 6.57 times higher in the pancreatic cancer patients than in the normal control group. However, it could be confirmed that the expression level of IL-22R in the lung cancer patients decreased to about 0.28 times that in the normal control group, and the expression level of IL-22R in the colorectal cancer patients significantly decreased to 0.17 times.

Furthermore, regarding the expression level of IL-22R relative to IL-22, it could be confirmed that the expression level of IL-22R relative to the expression level of IL-22 in the pancreatitis patients was about 9.93 times, and the expression level of IL-22R relative to the expression level of IL-22 in the pancreatic cancer patients was about 6.31 times, suggesting that, even though IL-22 binds to IL-22R, the expression level of IL-22R significantly increases compared to that of IL-22. However, the expression level of IL-22R relative to the expression level of IL-22 in the lung cancer patients was about 1.18 times, and the expression level of IL-22R relative to the expression level of IL-22 in the colorectal cancer patients was about 1.76 times, suggesting that the expression level of IL-22 is equivalent to that of IL-22R.

These results demonstrate that the expression patterns of IL-10R in pancreatitis or pancreatic cancer patients are clearly different from those in the normal control group or patients with other types of disease, and that the expression patterns of IL-10R are also different between pancreatitis and pancreatic cancer.

Therefore, the use of the above-described method according to the present invention makes it possible to not only detect and diagnose pancreatitis and pancreatic cancer with high accuracy, but also to distinguish and diagnose pancreatitis and pancreatic cancer.

### [Example 2]

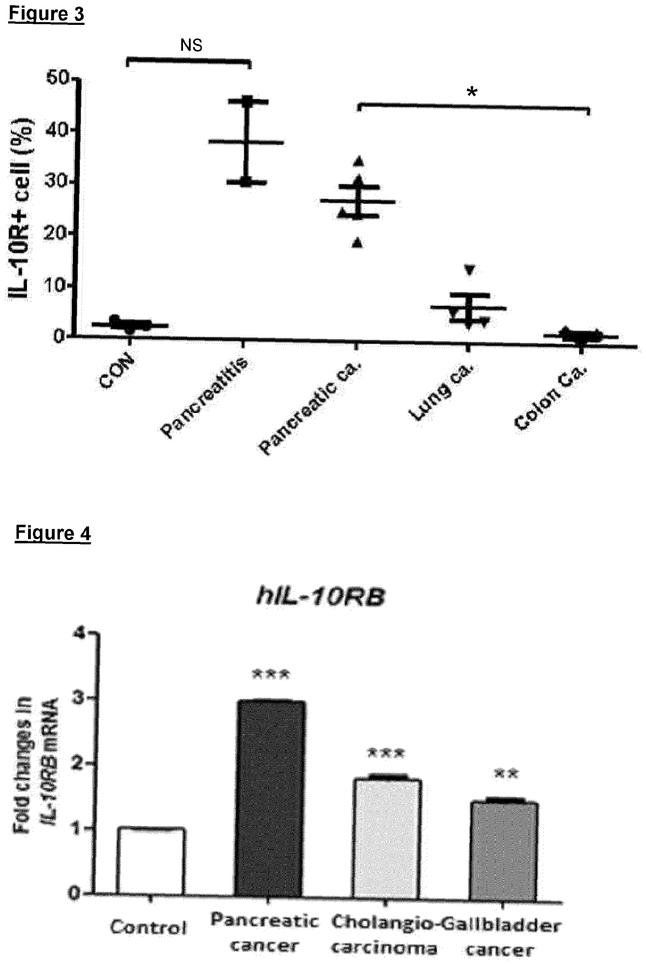

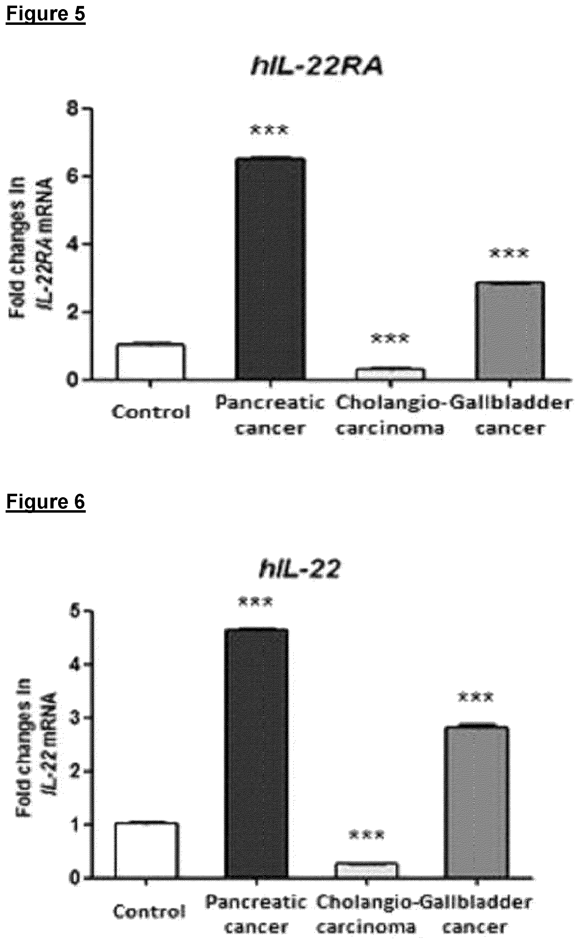

Blood was sampled from 4 normal control subjects, 4 pancreatic cancer patients, 2 cholangiocarcinoma patients and 2 gallbladder cancer patients, and then peripheral blood mononuclear cells (PBMCs) were isolated therefrom in the same manner as in Example 1. The ratios of the mRNA expression levels of IL-10R beta subunits (IL-10RB) and IL-22R alpha subunits (IL-22RA) in the mononuclear cells to those in the normal control group were measured, and the results are shown in FIGS. 4 and 5.

As shown in FIGS. 4 and 5, it could be confirmed that the IL-10RB mRNA expression level and the IL-22RA mRNA expression level increased about 3 times and about 6 times, respectively, in the mononuclear cells isolated from the pancreatic cancer patients, compared to the normal control group, whereas the expression level of IL-10RB mRNA in the cholangiocarcinoma and gallbladder cancer patients increased about 1 to 2 times, and the expression level of IL-22RA mRNA decreased in the cholangiocarcinoma patients.

These results indicate that the mRNA expression levels of IL-10RB and IL-22RA in the mononuclear cells significantly increase in pancreatic cancer compared to other types of cancer.

### [Example 3]

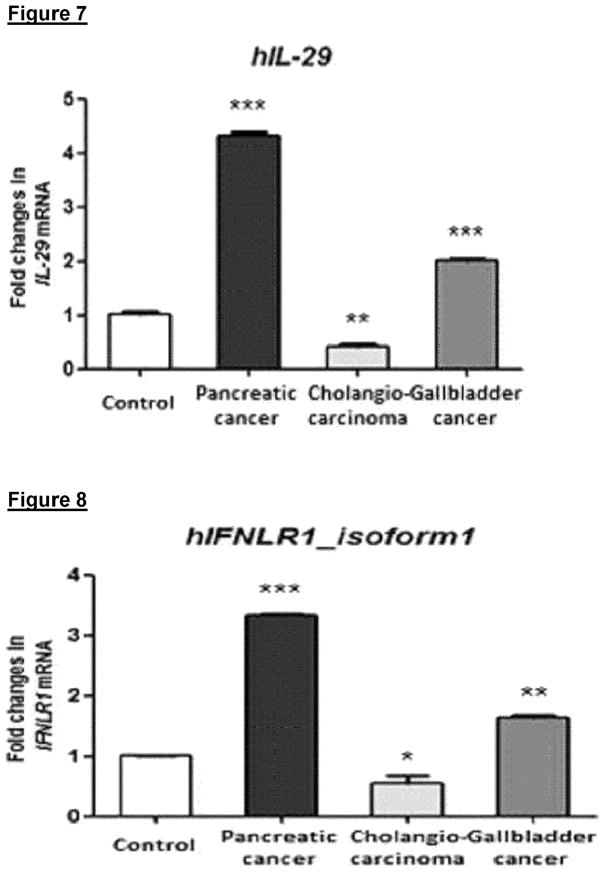

Blood was sampled from 4 normal control subjects, 4 pancreatic cancer patients, 2 cholangiocarcinoma patients and 2 gallbladder cancer patients, and peripheral blood mononuclear cells (PBMCs) were isolated therefrom in the same manner as in Example 1. The ratios of the mRNA expression levels of IL-22 and IL-29 in the PBMCs to those in the normal control group were measured, and the results are shown in FIGS. 6 and 7.

As shown in FIGS. 6 and 7, the expression levels of IL-22 mRNA and IL-29 mRNA were increased about 4 times or more in the mononuclear cells isolated from the pancreatic cancer patients, compared to those in the normal control group, but the expression levels of the two markers in the cholangiocarcinoma patients decreased, and the expression levels thereof in the gallbladder cancer patients increased only about 2 to 3 times.

These results indicate that the mRNA expression levels of IL-22 and IL-29 in the mononuclear cells significantly increase in pancreatic cancer compared to other types of cancer.

### [Example 4]

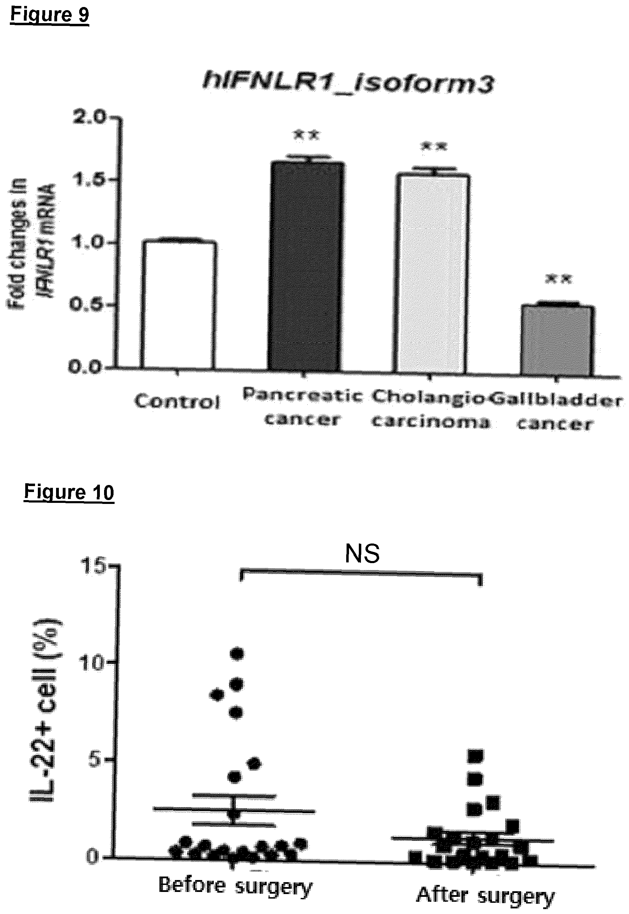

Blood was sampled from 4 normal control subjects, 4 pancreatic cancer patients, 2 cholangiocarcinoma patients and 2 gallbladder cancer patients, and peripheral blood mononuclear cells (PBMCs) were then isolated therefrom in the same manner as in Example 1. The ratios of the mRNA expression levels of interferon receptor 1 isoform 1 (IFNLR1 isoform 1) and interferon receptor 1 isoform 3 (IFNLR1 isoform 3), which bind to IL-29, in the mononuclear cells to those in the normal control group were measured, and the results are shown in FIGS. 8 and 9.

As shown in FIGS. 8 and 9, it could be confirmed that the mRNA expression level of interferon receptor 1 isoform 1, which binds to IL-29, increased about 3 times or more in the mononuclear cells isolated from the pancreatic cancer patients compared to the normal control group, but increased only about 1 to 2 times in the cholangiocarcinoma and gallbladder cancer patients.

These results indicate that the mRNA expression level of interferon receptor 1 isoform 1 in PBMCs was significantly increased in pancreatic cancer compared to other types of cancer.

### [Example 5]

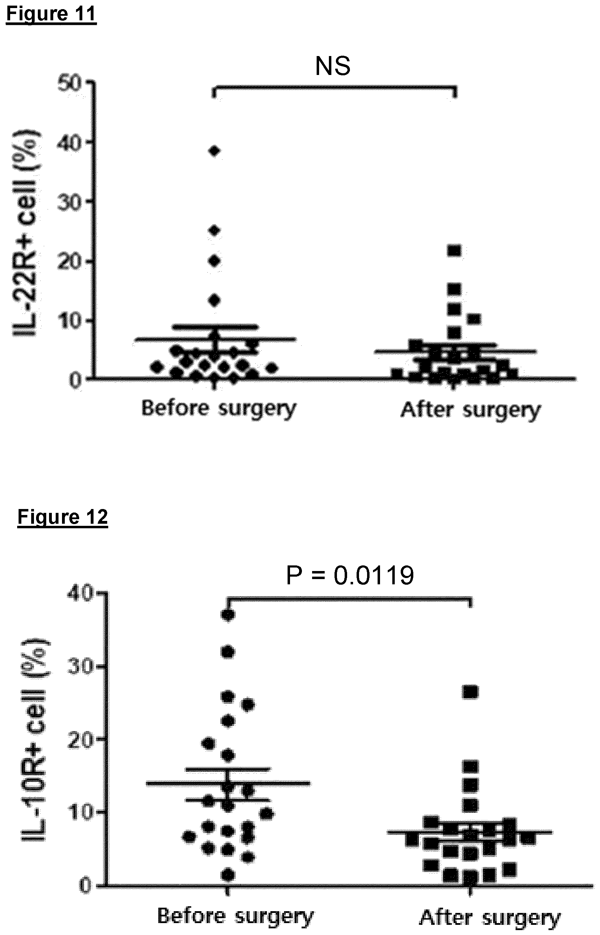

For pancreatic cancer patients who underwent pancreatic cancer resection (pancreatectomy), blood was sampled from the patients before and after the surgery, and peripheral blood mononuclear cells (PBMCs) were then isolated therefrom in the same manner as in Example 1. The number of cells expressing IL-10R, IL-22R or IL-22 was expressed as a percentage relative to the total number of the mononuclear cells using FACSCalibur (BD Biosciences, San Jose, CA). The results are shown in Table 2 below and FIGS. 10 to 12.

**[Table 2]**

| | Mean ± SD (%) | |
|---|---|---|
| | Before surgery | After surgery |
| IL-22 | 2.56±3.45 | 1.30±1.53 |
| IL-22R | 6.98±9.75 | 4.65±5.78 |
| IL-10R | 13.89±9.74 | 7.41±5.85 |

As shown in Table 2 above and FIGS. 10 to 12, it could be confirmed that, when the total mass of pancreatic cancer tissue in the pancreatic cancer patients was decreased due to pancreatectomy, the expression levels of IL-10R, IL-22 and IL-22R in the mononuclear cells significantly decreased, and in particular, the expression level of IL-10R significantly decreased.

### [Example 6]

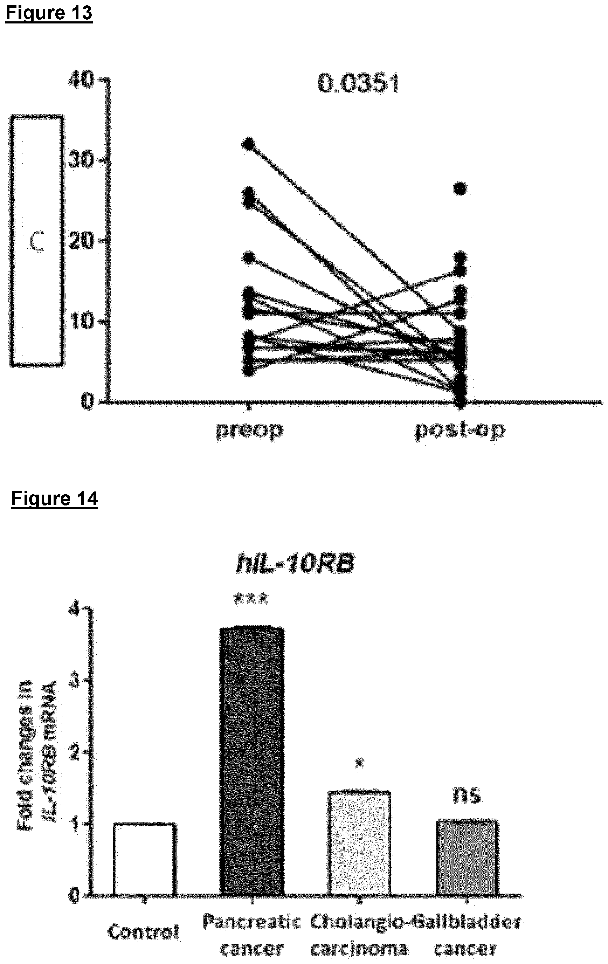

For patients who underwent pancreatic cancer resection (pancreatectomy), blood was sampled from the patients before and 3 months after the surgery, and peripheral blood mononuclear cells (PBMCs) were then isolated from therefrom in the same manner as in Example 1. The proportion of cells expressing IL-10R (marker C) was measured using FACSCalibur (BD Biosciences, San Jose, CA) and then analyzed by paired T test. The results are shown in FIG. 13.

As shown in FIG. 13, it could be confirmed that, when cancer tissue was removed from the pancreatic cancer patients by pancreatectomy, the proportion of monocular cells expressing IL-10R significantly decreased compared to that before surgery.

### [Example 7]

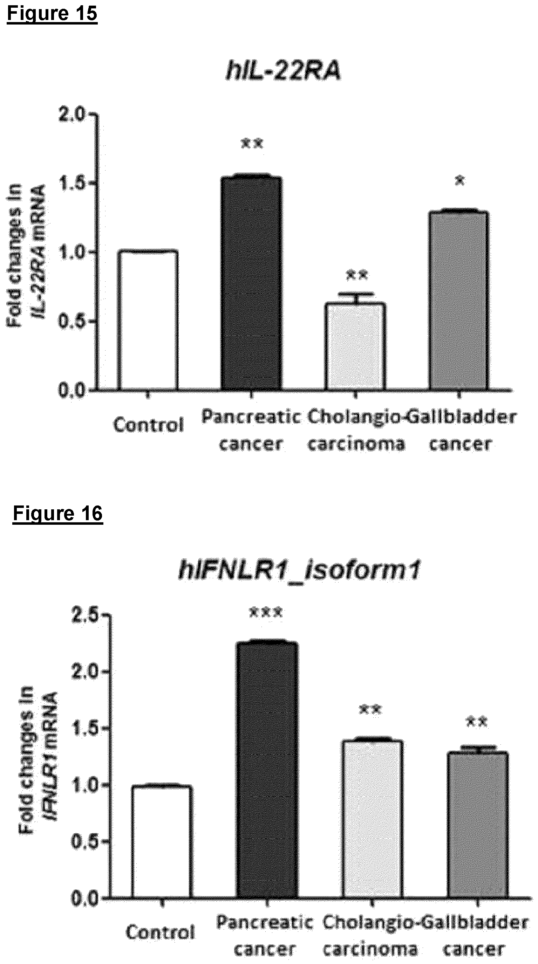

Blood was sampled from 4 normal control subjects, 4 pancreatic cancer patients, 2 cholangiocarcinoma patients and 2 gallbladder cancer patients, and the exosomes circulating in the plasma were isolated therefrom. The ratio of the expression level of IL-10RB, IL-22RA and IFNLR1 isoform 1 to that in the normal control group was measured, and the results are shown in FIGS. 14 to 16.

As shown in FIGS. 14 to 16, it could be confirmed that the mRNA expression levels of IL-10RB, IL-22RA and IFNLR1 isoform 1 in the exosomes isolated from the pancreatic cancer patients increased about 4 times, about 1.5 times and about 2.5 times, respectively, compared to those in the normal control group. However, it could be confirmed that the mRNA expression levels of IL-10RB and IFNLR1 isoform 1 in the exosomes isolated from the cholangiocarcinoma patients increased about 1.5 times and about 1.5 times, respectively, compared to those in the normal control group, and in particular, the mRNA expression level of IL-22RA rather decreased compared to that in the normal control group. In addition, it could be confirmed that the expression level of IL-10RB in the exosomes isolated from the cholangiocarcinoma patients was equivalent to that in the normal control group, and the mRNA expression levels of IL-22RA and IFNLR1 isoform 1 in the exosomes increased approximately less than 1.5 times compared to those in the normal control group.

These results suggest that the mRNA expression levels of 10RB, IL-22RA and IFNLR1 isoform 1 in the exosomes isolated from the plasma of pancreatic cancer patients significantly increase compared to those in the normal control group or other types of cancer.

### [Example 8]

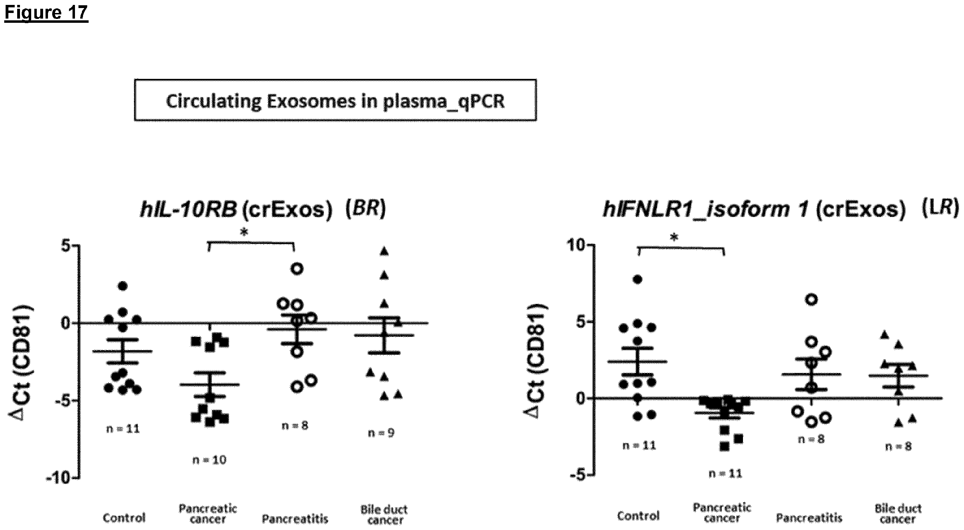

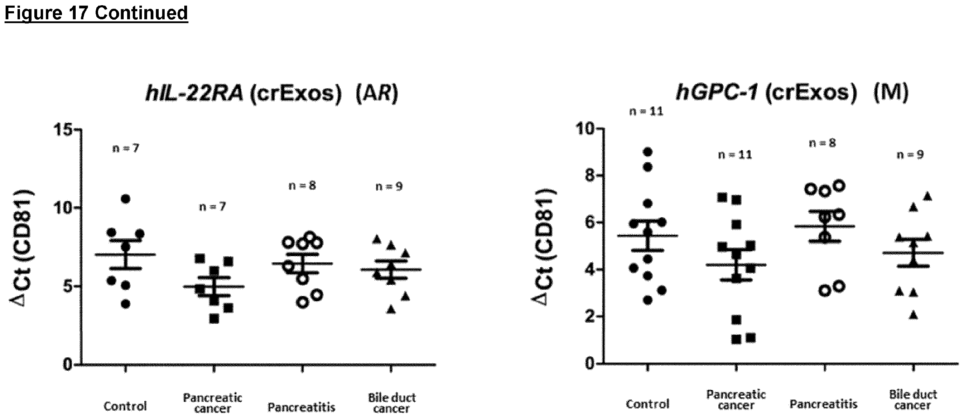

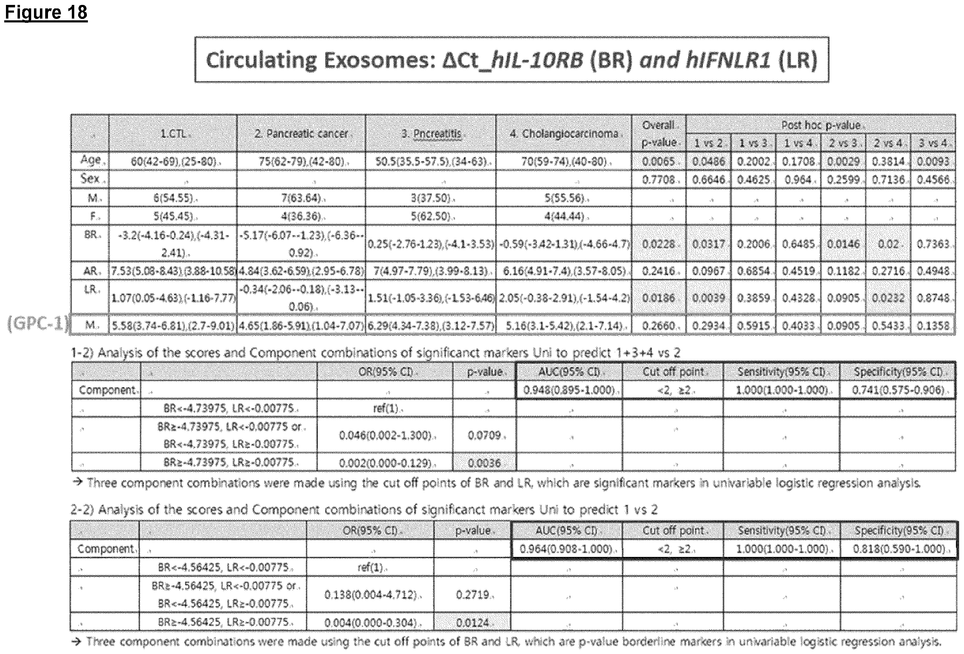

Blood was sampled from 11 normal control subjects, 10 pancreatic cancer patients, 8 pancreatitis patients and 9 cholangiocarcinoma patients, and exosomes circulating in the plasma were isolated therefrom. The mRNA expression level of each of IL-10RB (BR), IL-22RA (AR) and IFNLR1 isoform 1 (LR) in each of the groups was measured using qRT-PCR, and the results are shown in FIG. 17. In addition, as a positive control for comparison, the mRNA expression level of GPC-1 (M) known as a biomarker of pancreatic cancer was measured, and the results are also shown in FIG. 17. In addition, AUC, sensitivity and specificity were measured by score analysis for a combination of IL-10RB (BR) and IFNLR1 isoform 1 (LR) for prediction of pancreatic cancer patients, and the results are shown in FIG. 18.

As shown in FIG. 17, it could be confirmed that the mRNA expression levels of IL-10RB mRNA, IL-22RA mRNA and IFNLR1 isoform 1 in the exosomes isolated from the pancreatic cancer patients significantly increased compared to those in the normal control group. However, it could be confirmed that the mRNA expression levels of IL-10RB mRNA, IL-22RA mRNA and IFNLR1 isoform 1 in the exosomes isolated from the pancreatitis patients and the cholangiocarcinoma patients were equivalent to those in the normal control group.

As shown in FIG. 18, as a result of performing score analysis for a combination of IL-10RB (BR) and IFNLR1 isoform 1 (LR) in the pancreatic cancer patient group, the normal control group, the pancreatitis patient group and the cholangiocarcinoma patient for prediction of pancreatic cancer, it could be seen that the sensitivity was 1.00, the specificity was 0.741 or 0.818, and the AUC was 0.948 or 0.964, indicating that the accuracy of prediction using this combination was very high. Meanwhile, in the case of GPC-1 previously known as a pancreatic cancer biomarker, it can be seen that neither the overall p-value nor the post-hoc p-value are statistically significant.

### [Example 9]

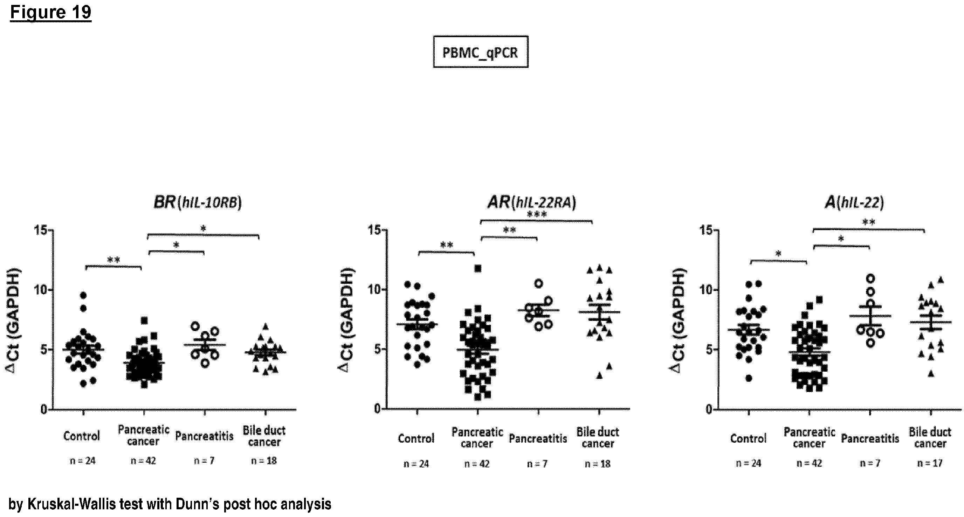

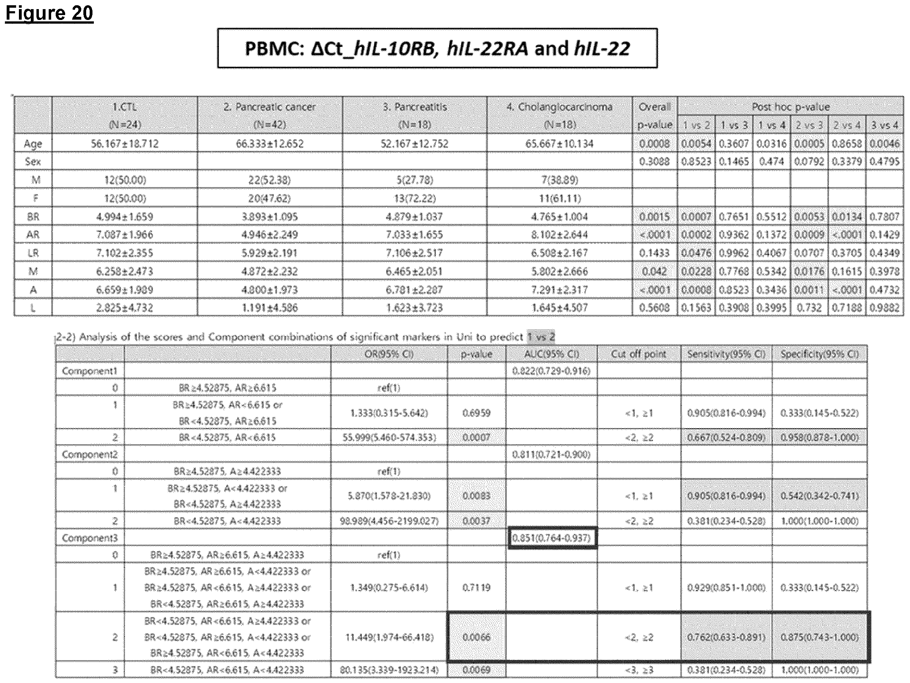

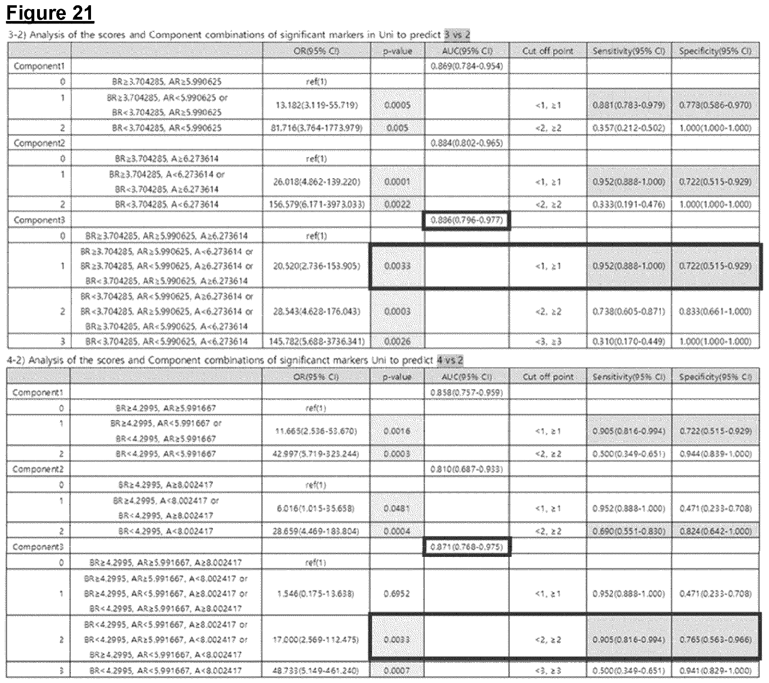

Blood was sampled from 24 normal control subjects, 42 pancreatic cancer patients, 7 pancreatitis patients and 18 cholangiocarcinoma patients, and peripheral blood mononuclear cells (PBMCs) were isolated therefrom. The mRNA expression level of each of IL-10RB (BR), IL-22RA (AR) and IL-22 (A) in each of the groups was measured using qRT-PCR, and the results are shown in FIG. 19. In addition, AUC, sensitivity and specificity were measured by score analysis for various combinations of IL-10RB (BR), IL-22RA (AR) and IL-22 (A) for prediction of pancreatic cancer patients, and the results are shown in FIGS. 20 and 21.

As shown in FIG. 19, it can be confirmed that the mRNA expression levels of IL-10RB mRNA, IL-22RA mRNA and IL-22 in the mononuclear cells isolated from the pancreatic cancer patients significantly increased compared to those in the normal control group. However, it could be confirmed that the mRNA expression levels of IL-10RB mRNA, IL-22RA mRNA and IL-22 in the mononuclear cells isolated from the pancreatitis patients and the cholangiocarcinoma patients were equal to or increased compared to those in the normal control group.

In addition, as shown in FIGS. 20 and 21, as a result of performing score analysis for a combination of IL-10RB (BR) and IL-22RA (AR), a combination of IL-10RB (BR) and IL-22 (A), and a combination of 22RA (AR) and IL-22 (A) in the pancreatic cancer patient group, the pancreatitis patient group and the cholangiocarcinoma patient group for prediction of pancreatic cancer, it could be seen that sensitivity, specificity and AUC in all the three combinations were very high in the pancreatic patients, indicating that the accuracy of prediction of pancreatic cancer using these combinations was very high. In particular, it could be confirmed that the accuracy of prediction in a combination of IL-10RB (BR), IL-22RA (AR) and IL-22 (A) was very high.

### [Example 10]

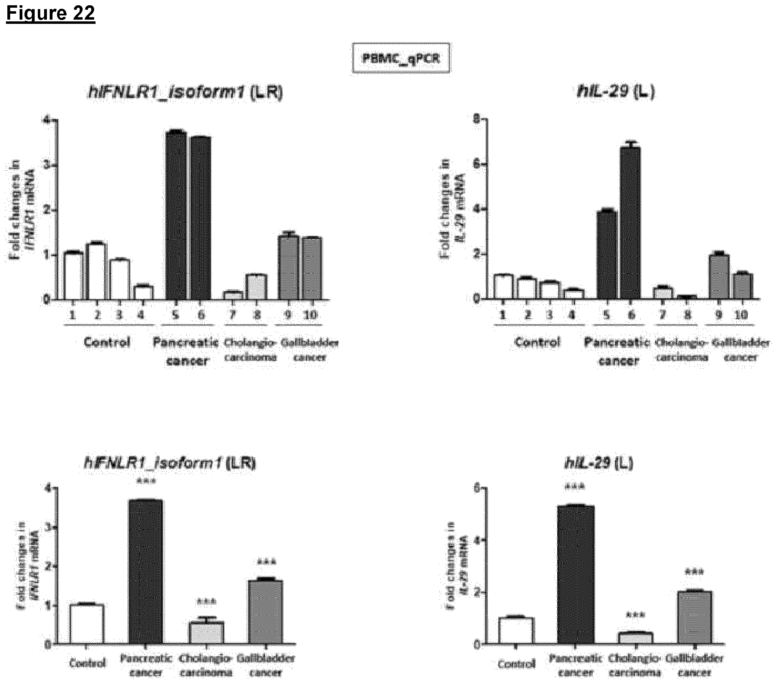

Blood was sampled from 4 normal control subjects, 2 pancreatic cancer patients, 2 gallbladder cancer patients and 2 cholangiocarcinoma patients, and peripheral blood mononuclear cells (PBMCs) were then isolated therefrom. The mRNA expression level of each of IFNLR1 isoform 1 (LR) and IL-29 (L) in each of the groups was measured using qRT-PCR, and the ratios of the mRNA expression levels of IFNLR1 isoform 1 (LR) and IL-29 (L) to those in the normal control group were measured. The results are shown in FIG. 22.

As shown in FIG. 22, it could be confirmed that the mRNA expression levels of IFNLR1 isoform 1 (LR) and IL-29 (L) in the mononuclear cells isolated from the pancreatic cancer patients significantly increased compared to those in the normal control group, whereas the mRNA expression levels of IFNLR1 isoform 1 (LR) and IL-29 (L) in the mononuclear cells isolated from the gallbladder cancer patients and the cholangiocarcinoma patients were only equivalent to those in the normal control group.

### [Example 11]

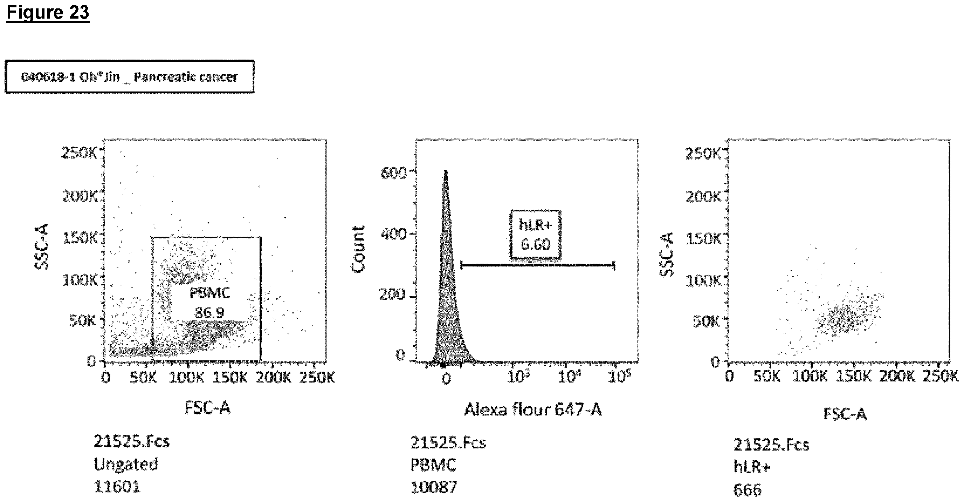

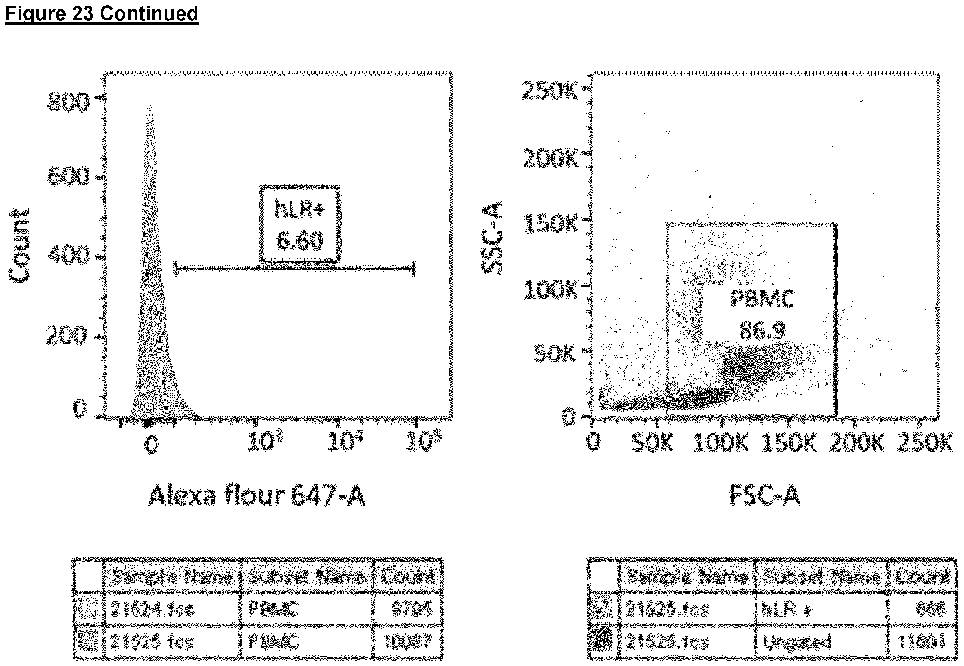

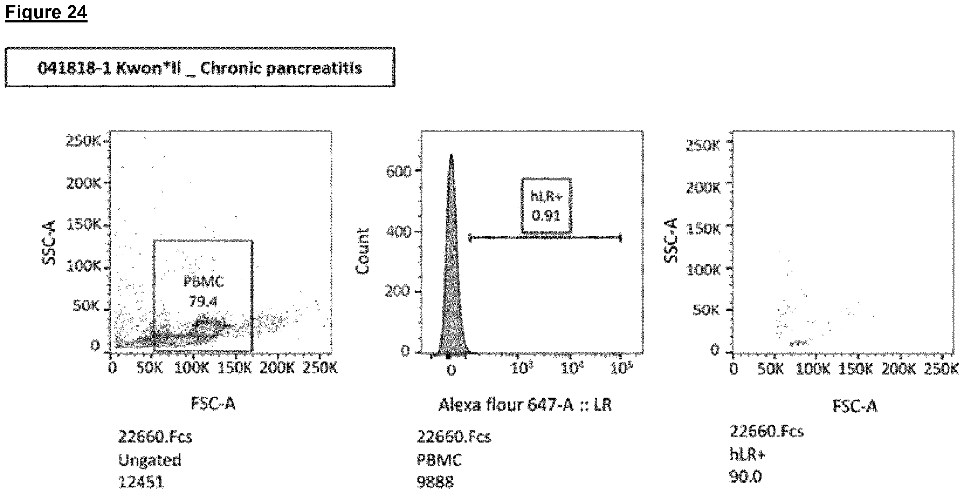

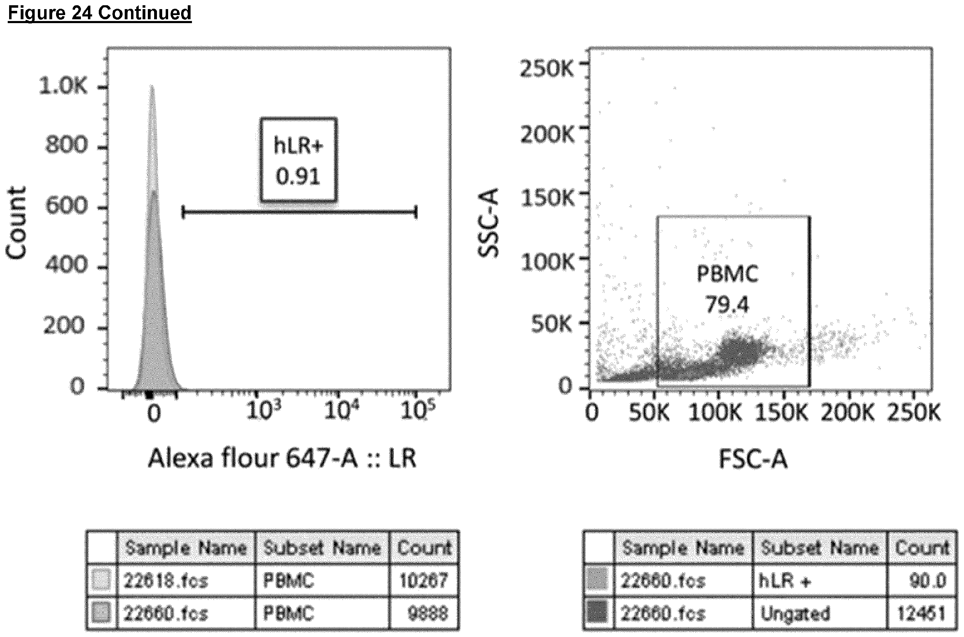

Blood was sampled from pancreatic cancer patients and chronic pancreatitis patients, and then peripheral blood mononuclear cells (PBMCs) were isolated therefrom and subjected to FACS analysis using an antibody specific for IFNLR1 isoform 1. The results are shown in FIGS. 23 and 24.

As shown in FIGS. 23 and 24, it could be confirmed that the proportion of cells expressing IFNLR1 isoform 1 in the mononuclear cells isolated from the pancreatic cancer patients increased compared to that from the chronic pancreatitis patients, indicating that the expression level of IFNLR1 isoform 1 protein in the pancreatic cancer patients increased.

### [Example 12]

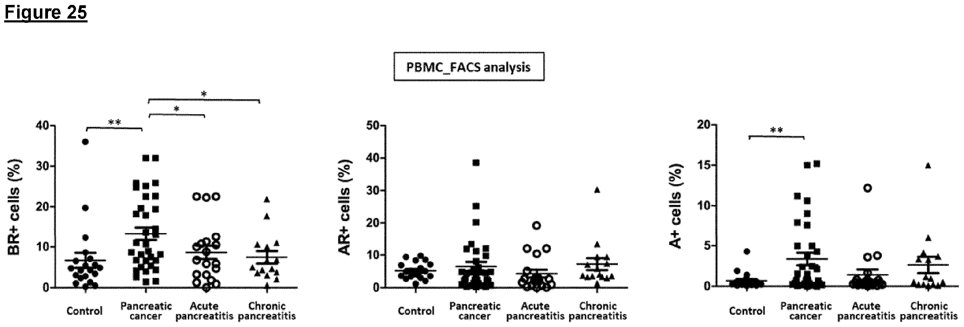

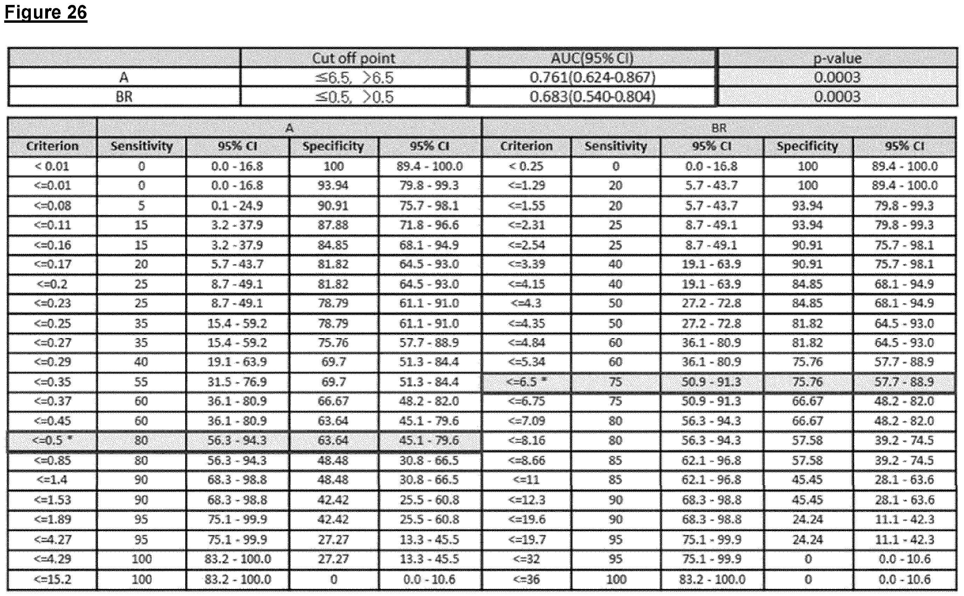

Blood was sampled from normal control subjects, pancreatic cancer patients, acute pancreatitis patients and chronic pancreatitis patients, and then peripheral blood mononuclear cells (PBMCs) were isolated therefrom and subjected to FACS analysis using an antibody specific for each of IL-10RB (BR), IL-22RA (AR) and IL-22 (A). Then, the proportion of cells expressing each of IL-10RB (BR), IL-22RA (AR) and IL-22 (A) in each of the groups was measured, and the results are shown in FIG. 25. In addition, the results of the FACS analysis were statistically analyzed, and the results are shown in FIG. 26.

As shown in FIG. 25, it could be confirmed that the proportion of cells expressing IL-10RB (BR), IL-22RA (AR) and IL-22 (A) in the mononuclear cells isolated from the pancreatic cancer patients increased compared to that in the acute or chronic pancreatitis patients, indicating that the expression levels of these biomarkers in the pancreatic cancer patients increased.

In addition, as shown in FIG. 26, it could be seen that the sensitivity, specificity and AUC for IL-10RB (BR) and IL-22 (A) in the pancreatic cancer patients were measured to be high, indicating that the accuracy of prediction of pancreatic cancer using these biomarkers is very high.

### [Example 13]

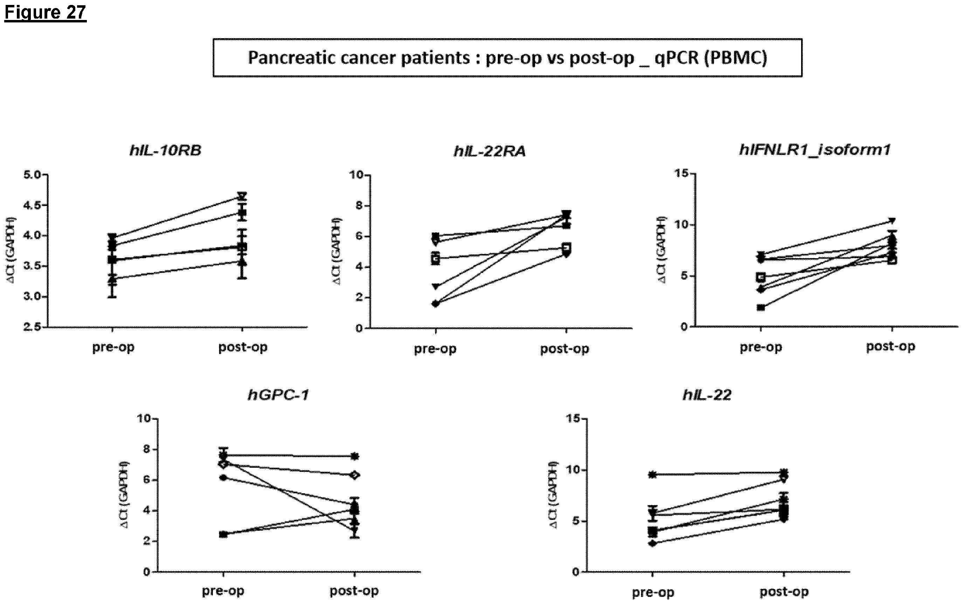

For 11 pancreatic cancer patients who underwent surgical resection, blood was sampled from the patients before and after surgical resection, and peripheral blood mononuclear cells were isolated therefrom. Then, the mRNA expression level of each of IL-10RB, IL-22RA, IFNLR1 isoform 1 and IL-22 in the cells before and after the surgery was measured using qRT-PCR, and the results are shown in FIG. 27. In addition, as a positive control for comparison, the expression level of GPC-1 mRNA known as a biomarker of pancreatic cancer was also measured, and the results are shown in FIG. 27.

As shown in FIG. 27, it could be confirmed that, after the pancreatic cancer was removed by surgical resection, the mRNA expression levels of IL-10RB, IL-22RA, IFNLR1 isoform 1 and IL-22 all decreased compared to those before surgical resection. However, it could be confirmed that the mRNA expression level of GPC-1 remained constant or tended to increase.

### [Example 14]

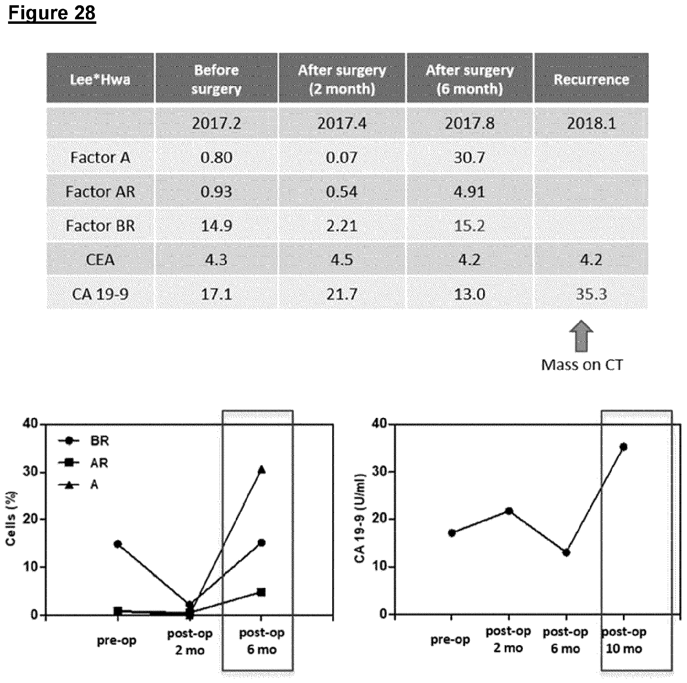

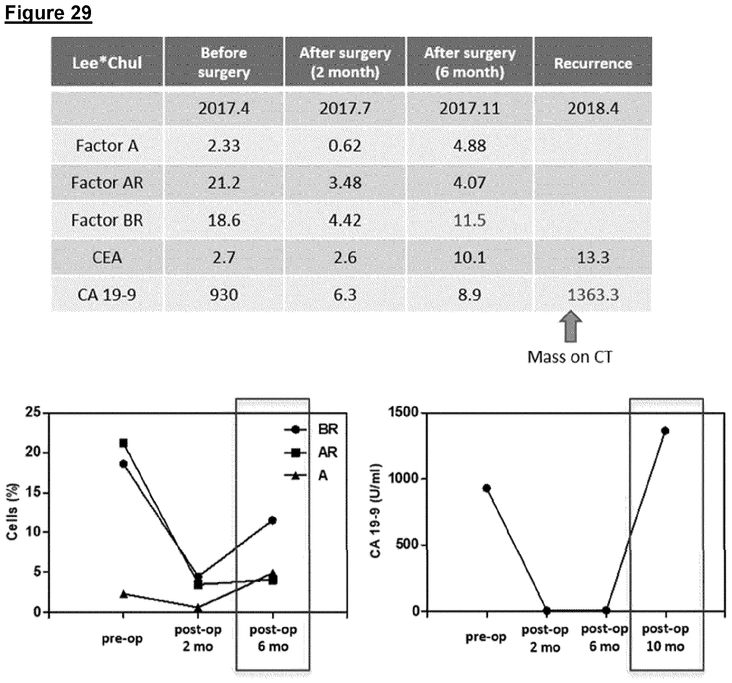

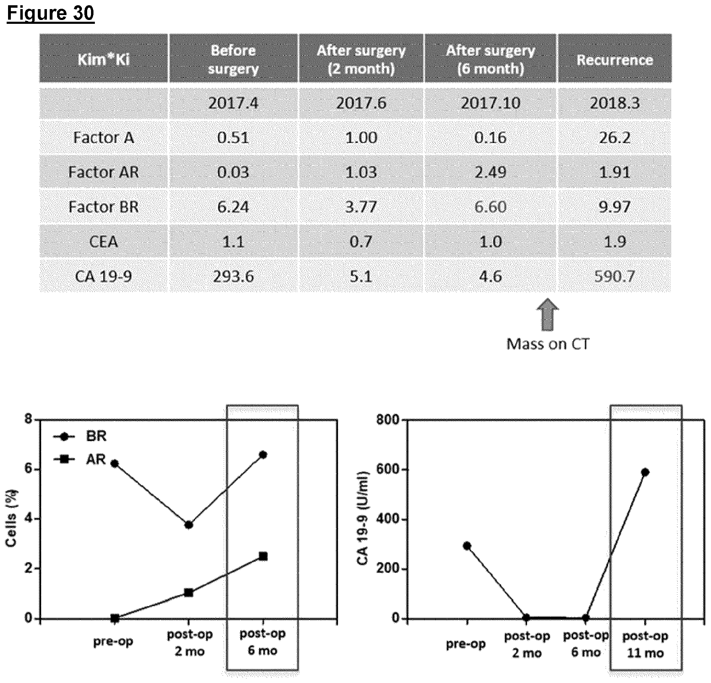

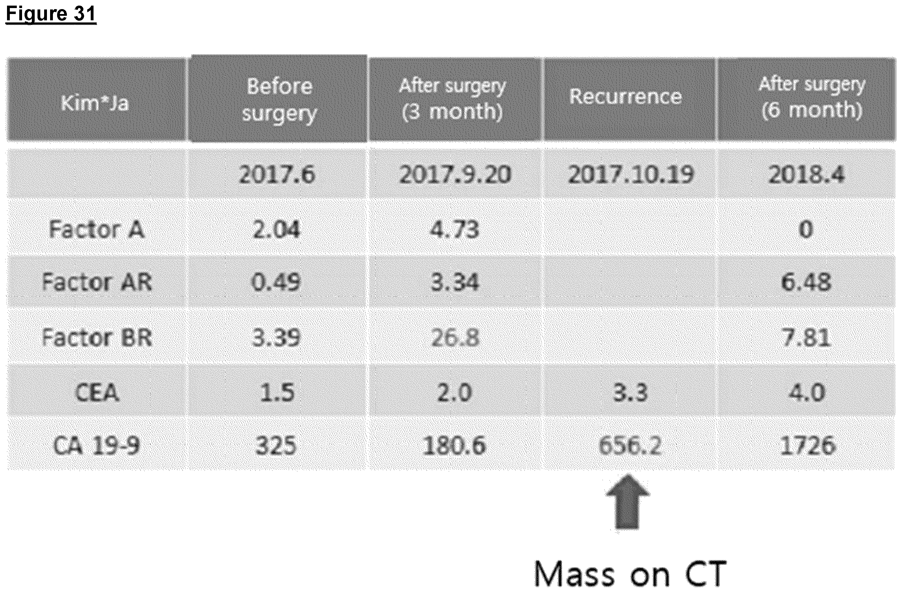

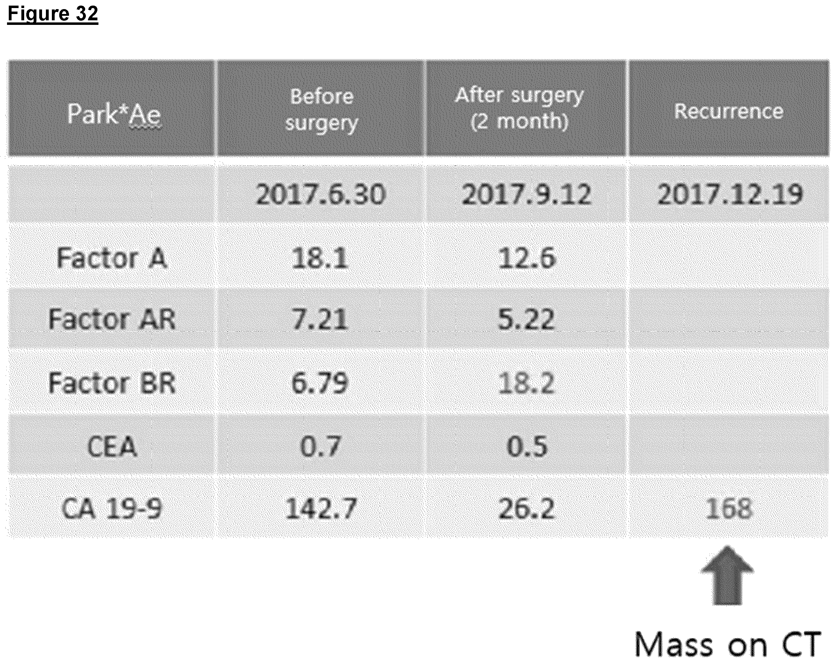

For 5 pancreatic cancer patients who recurred after undergoing surgical resection, blood was sampled before surgical resection, 2 and 6 months after surgical resection, and upon recurrence. Blood peripheral mononuclear cells were isolated from the blood samples. Then, the mRNA expression level of each of IL-10RB (BR), IL-22RA (AR) and IL-22 (A) in the cells before and after the surgery was measured using qRT-PCR, and the proportion of cells expressing IL-10RB (BR), IL-22RA (AR) and IL-22 (A) was measured by FACS analysis. As positive controls for comparison, the mRNA expression levels of CEA and CA known as biomarkers of pancreatic cancer were measured, and in particular, in the case of CA 19-9, the change in the expression level thereof at each time point was measured. The experimental results for each patient are shown in FIGS. 28 to 32.

As shown in FIGS. 28 to 32, it could be confirmed that, when the expression level of any one or more of IL-10RB (BR), IL-22RA (AR) and IL-22 (A) significantly increased before that before surgical resection even after pancreatic cancer was removed by surgical resection, pancreatic cancer recurred. However, it could be confirmed that the expression levels of CEA and CA 19-9 remained decreased even after surgical resection, and the expression level of CA 19-9 increased only 10 months after surgical resection.

These results suggest that the use of the IL-10RB (BR), IL-22RA (AR) and IL-22 (A) according to the present invention makes it possible to predict the recurrence of pancreatic cancer with high accuracy early.

### [Example 15]

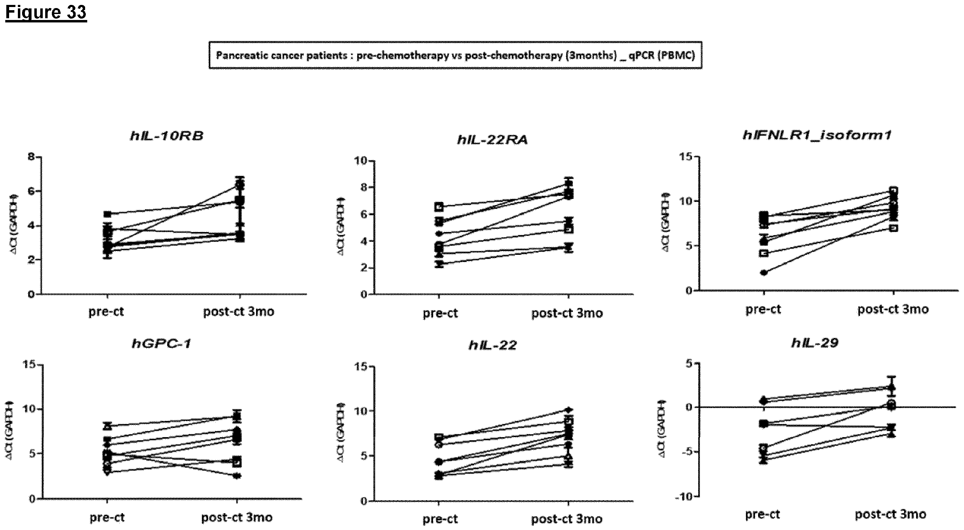

For 12 pancreatic cancer patients who received anticancer chemotherapy, blood was sampled from the patients before and after chemotherapy, and peripheral mononuclear blood cells were isolated therefrom. Then, the mRNA expression level of each of IL-10RB, IL-22RA, IFNLR1 isoform 1, IL-22 and IL-29 before and after the chemotherapy was measured using qRT-PCR, and the results are shown in FIG. 33. As a positive control for comparison, the mRNA expression level of GPC-1 known as a biomarker of pancreatic cancer was also measured, and the results are shown in FIG. 33.

As shown in FIG. 33, it could be seen that, after the pancreatic cancer patients were subjected to anticancer chemotherapy, the mRNA expression levels of IL-10RB, IL-22RA, IFNLR1 isoform 1, IL-22 and IL-29 all decreased compared to those before the chemotherapy. However, it could be confirmed that the mRNA expression level of GPC-1 remained constant or tended to increase.

### [Example 16]

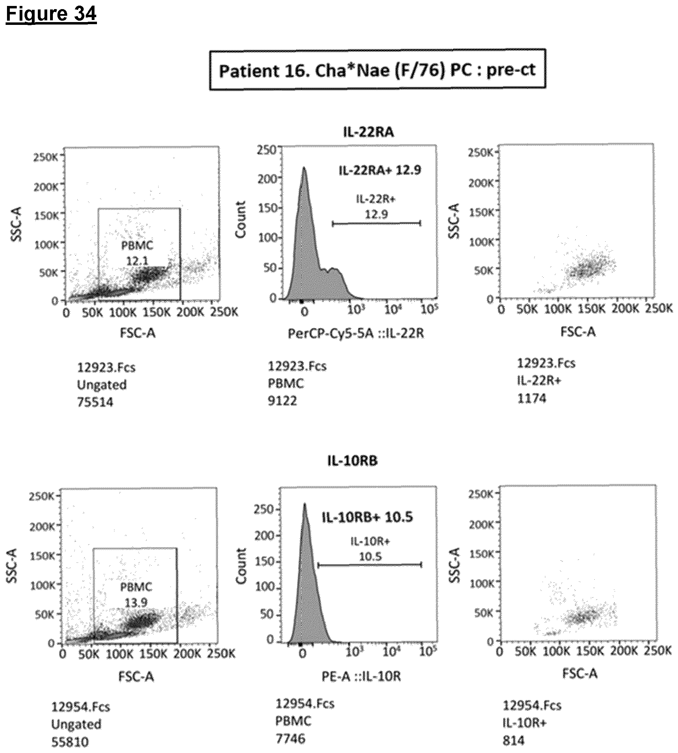

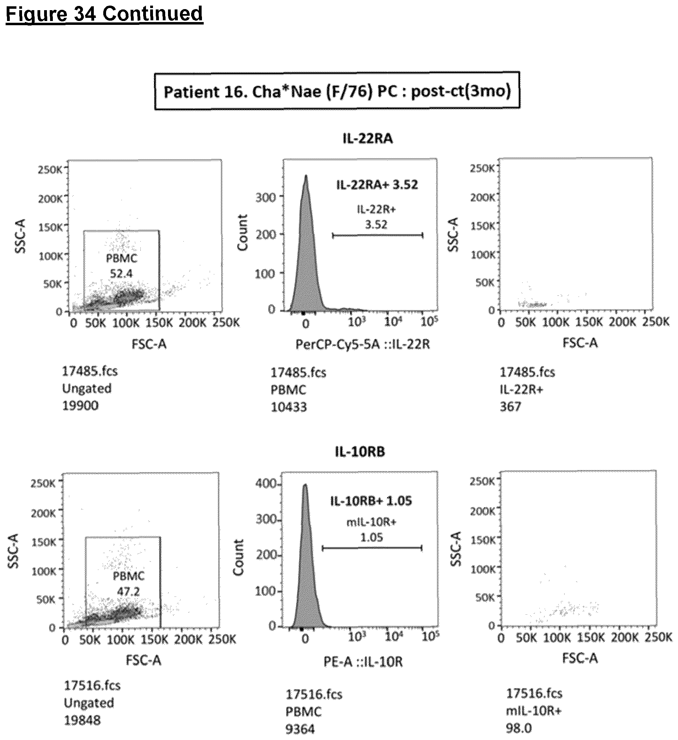

For pancreatic cancer patients who received anticancer chemotherapy, blood was sampled before chemotherapy and 3 months after chemotherapy, and peripheral blood mononuclear cells were isolated therefrom. Then, the proportion of cells expressing IL-10RB (BR) and IL-22RA (AR) in the mononuclear cells before and after chemotherapy was measured by FACS analysis, and the results are shown in FIG. 34.

As shown in FIG. 34, it could be confirmed that the proportion of cells expressing IL-10RB (BR) was 12.9% before chemotherapy, but decreased to 3.52% after chemotherapy, and the proportion of cells expressing IL-22RA (AR) was 10.5% before chemotherapy, but decreased to 1.05% after chemotherapy.

### [Example 17]

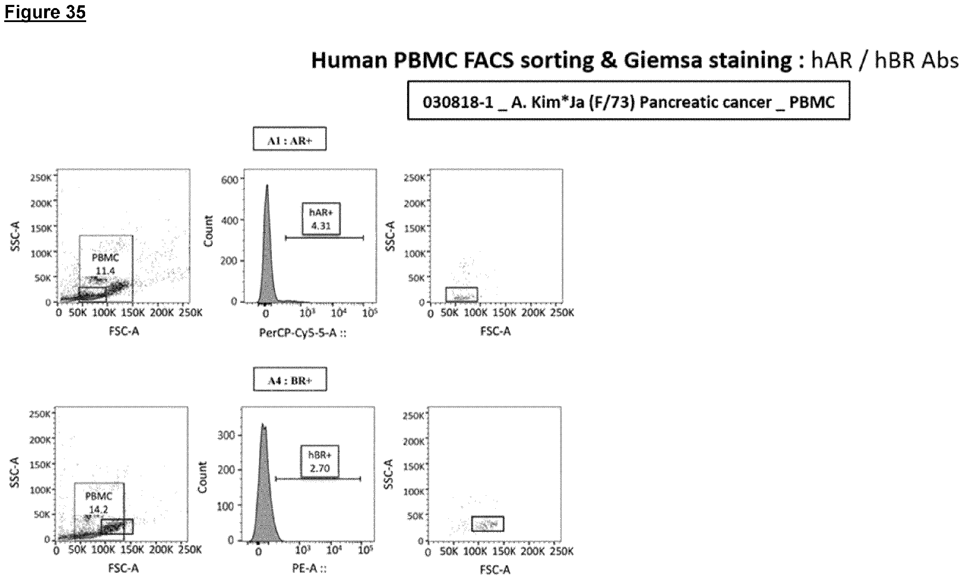

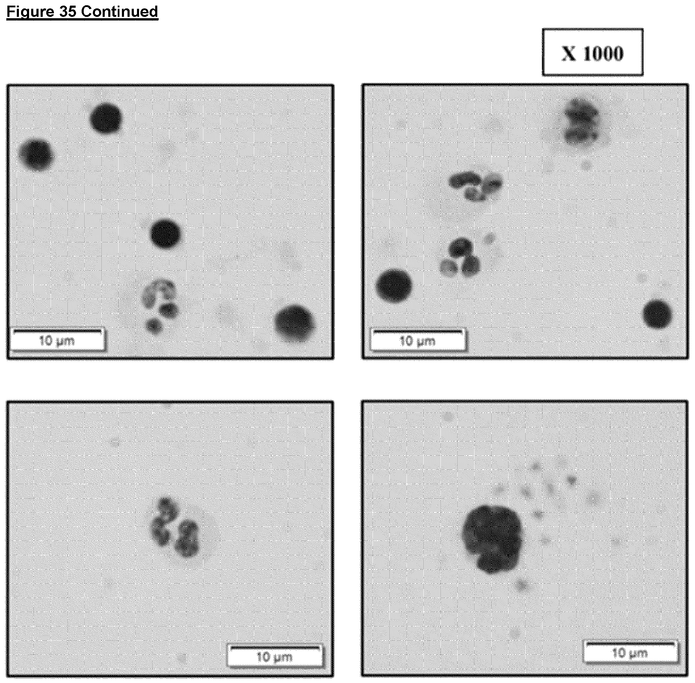

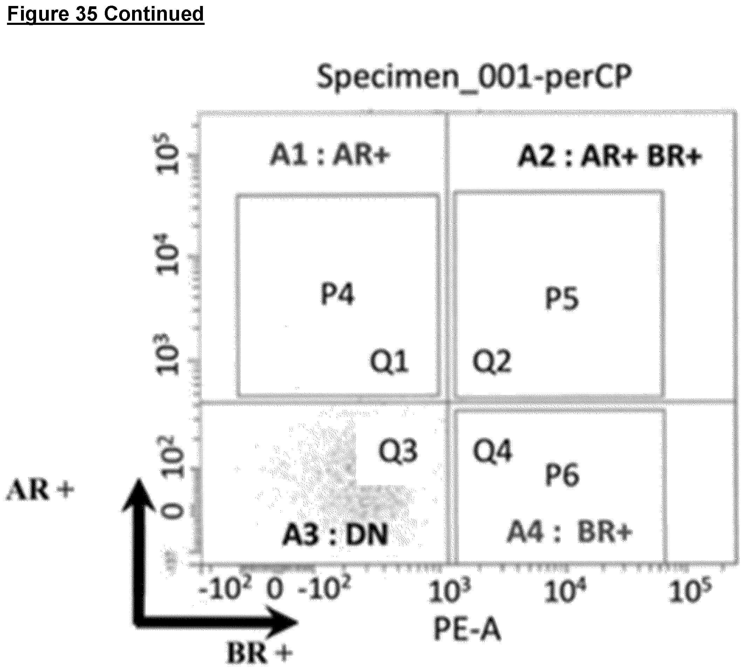

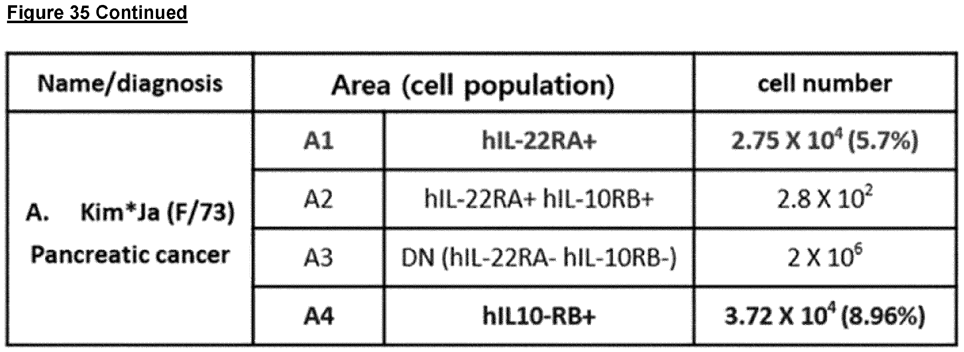

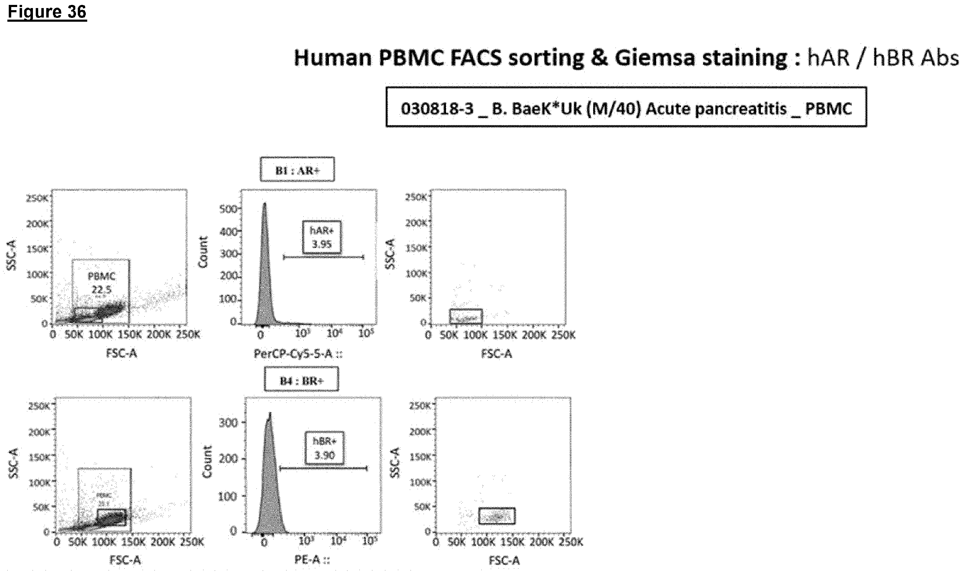

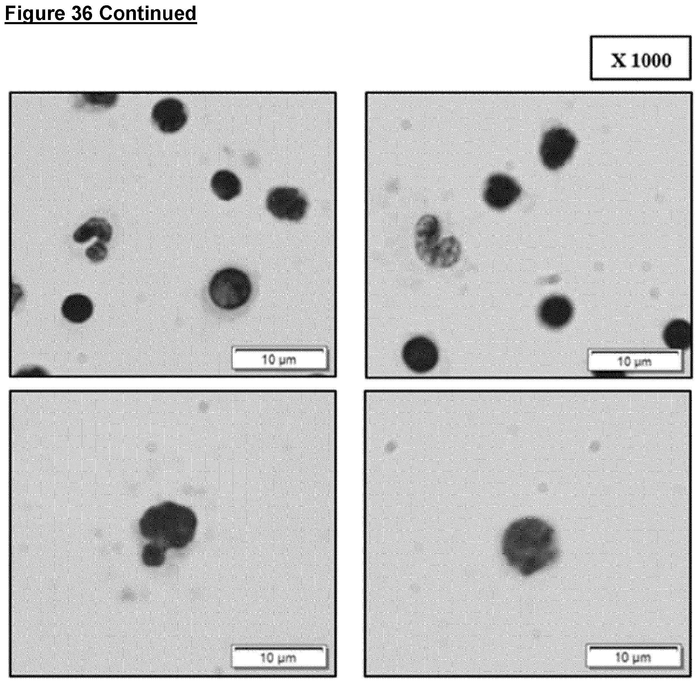

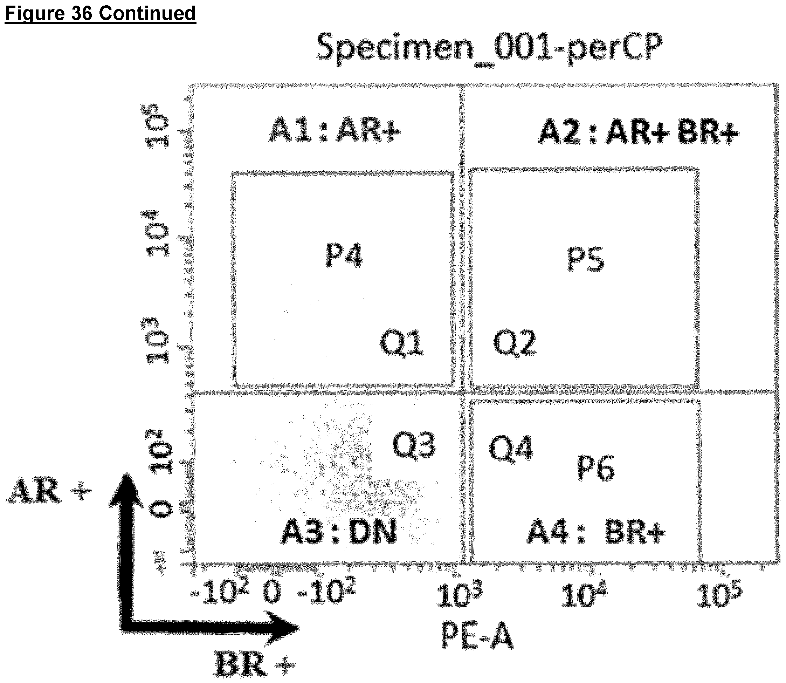

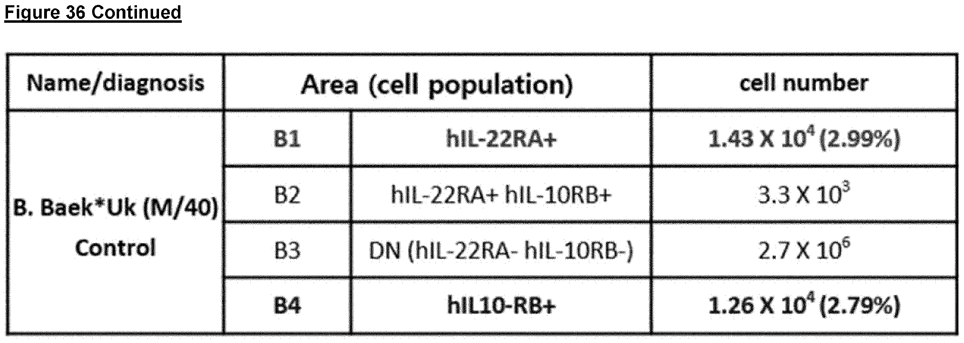

Blood was sampled from pancreatic cancer patients and acute pancreatitis patients, and then peripheral blood mononuclear cells (PBMCs) were isolated therefrom and subjected to FACS staining using antibodies specific to IL-10RB (BR) and IL-22RA (AR). Then, IL-10RB-expressing cells (BR+) and IL-22RA-expressing cells (AR+) were isolated and subjected to Giemsa staining. The results for the pancreatic cancer patients and the acute pancreatitis patients are shown in FIGS. 35 and 36, respectively.

As shown in FIGS. 35 and 36, as a result of analyzing the morphologies of IL-10RB-expressing cells (BR+) and IL-22RA-expressing cells for the pancreatic cancer patients, it could be confirmed that the IL-10RB-expressing cells (BR+) were of bone marrow origin and the IL-22RA-expressing cells (AR+) were of lymph origin, indicating that these cells were of different origins. In addition, the IL-22RA-expressing cells (AR+) were more heterogeneous than the IL-10RB-expressing cells (BR+). At this time, it could be confirmed that the proportions of the IL-22RA-expressing cells (AR+) and the IL-10RB-expressing cells (BR+) in the pancreatic cancer patients were very high compared to those in the acute pancreatitis patients.

### [Example 18]

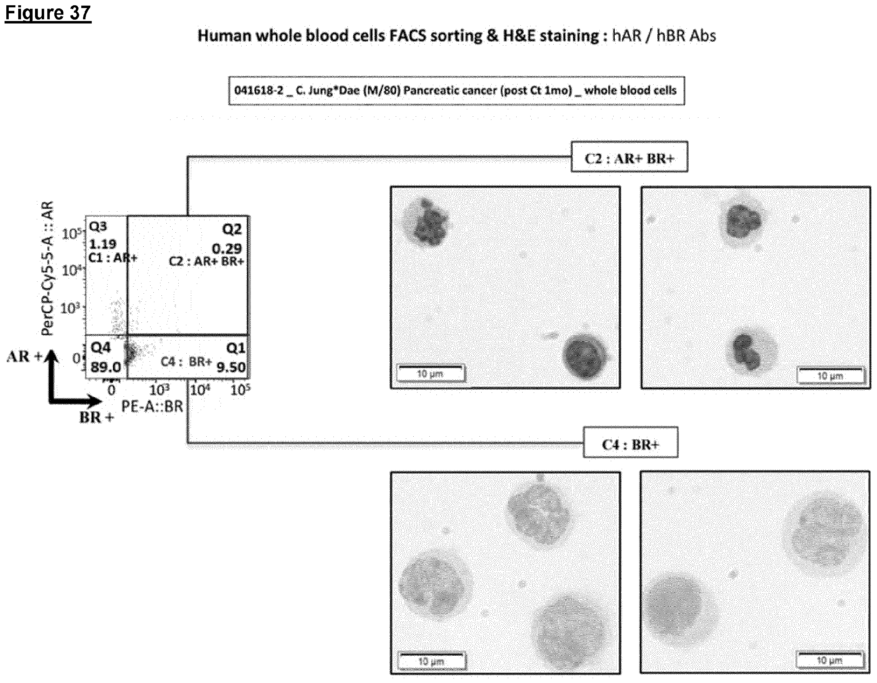

Blood was sampled from pancreatic cancer patients, stored in EDTA tubes, and then centrifuged at 400g for 5 minutes. The plasma was stored separately, and the plasma pellet was incubated in RBC lysis buffer at room temperature for 3 minutes and then washed with phosphate buffered saline (PBS). The whole-blood cells isolated as described above were subjected to FACS staining using antibodies specific to IL-10RB (BR) and IL-22RA (AR), and then IL-22RA- and IL-10RB-expressing cells (AR+BR+) and IL-10RB-expressing cells (BR+) were isolated and subjected to H&E staining. The results are shown in FIG. 37.

As shown in FIG. 37, as a result of comparing the morphologies of the IL-22RA- and IL-10RB-expressing cells (AR+BR+) and the IL-10RB-expressing cells (BR+), it could be seen that these cells were of different origins.

### [Example 19]

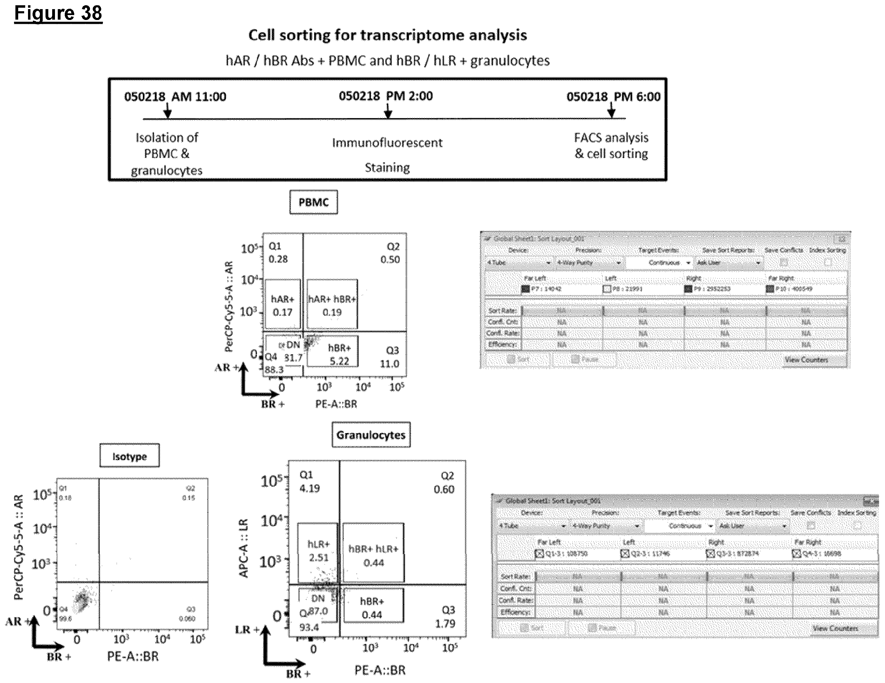

Blood was sampled from pancreatic cancer patients, and whole-blood cells were isolated therefrom, and then subjected to FACS staining using antibodies specific to IL-10RB (BR), IL-22RA (AR) and IFNLR1 isoform 1 (LR). Then, IL-22RA-expressing cells (AR+), IL-10RB-expressing cells (BR+) and IFNLR1 isoform 1-expressing cells (LR+) were isolated and then subjected to transcriptome analysis. The results are shown in FIG. 38.

As shown in FIG. 38, the cells expressing IL-22RA, IL-10RB and IFNLR1 isoform 1 could be observed in the whole-blood cells obtained from the pancreatic cancer patients.

### [Example 20]

### 1. Preparation of Orthotopic Mouse Model of Pancreatic Cancer

The mouse abdomen was incised, and pancreatic cancer cells, Panc-1 cells and Aspc cells, were injected into the pancreas tail in an amount of 2×10⁶ cells/50 µl. After 2 weeks, the abdomen was incised and whether pancreatic cancer had grown was checked.

### 2. Isolation of Pancreatic Cells

Pancreatic tissue was isolated from the mice, and then treated with 0.5 mg/ml collagenase XI, 0.25 mg/ml dispase 1 and 0.25 mg/ml dispase 2 at 37°C for 1 hour. When the pancreatic tissue became mushy, it was ground in a strainer, and then only cells were collected, washed, and then centrifuged. An adipose layer was separated from the precipitate layer and only cells were collected and separated into mononuclear cells, granulocytes and RBCs using Histopaque 1083 and 1119 (800 g/25 min). Among the separated layers, plasma was stored separately, and the remaining two cell layers except for RBC were collected and subjected to FACS.

### 3. Isolation of Spleen Cells

Spleen tissue was isolated from the mice and ground in a strainer, and then only cells were collected, and separation was performed using Histopaque in the same manner as for the isolation of pancreatic cells.

### 4. Isolation of Whole-Blood Cells

Mouse blood was collected in PBS syringes containing 2mM EDTA, and then centrifuged at 400g for 5 minutes. Then, the plasma was stored separately, and the lower pellet layer was resuspended, and separation was performed using Histopaque in the same manner as for the isolation of pancreatic cells.

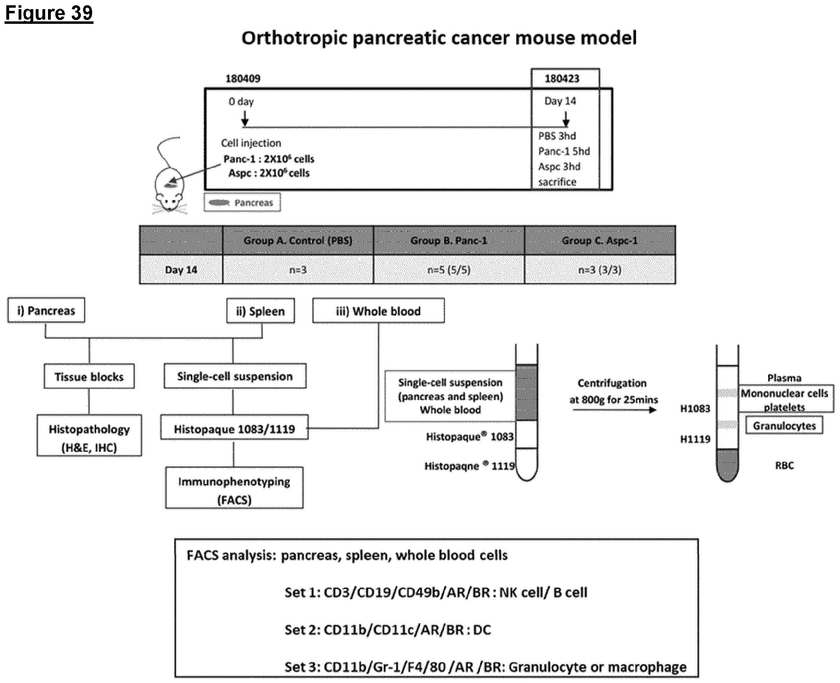

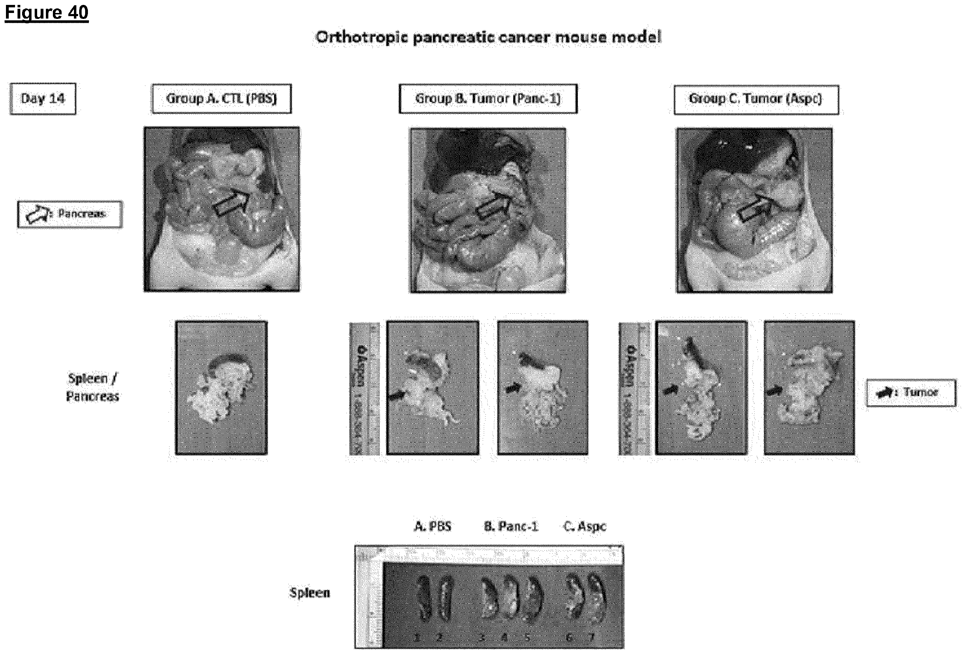

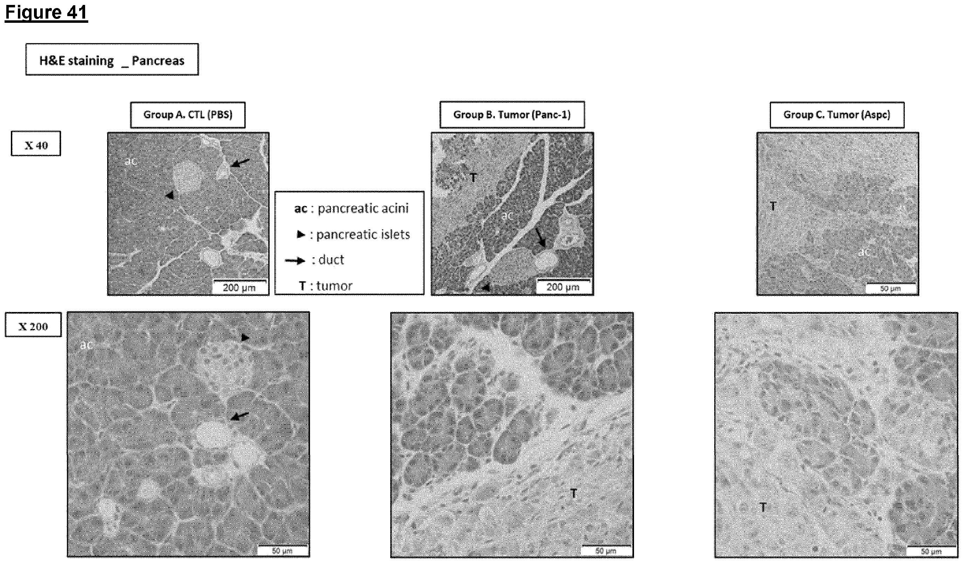

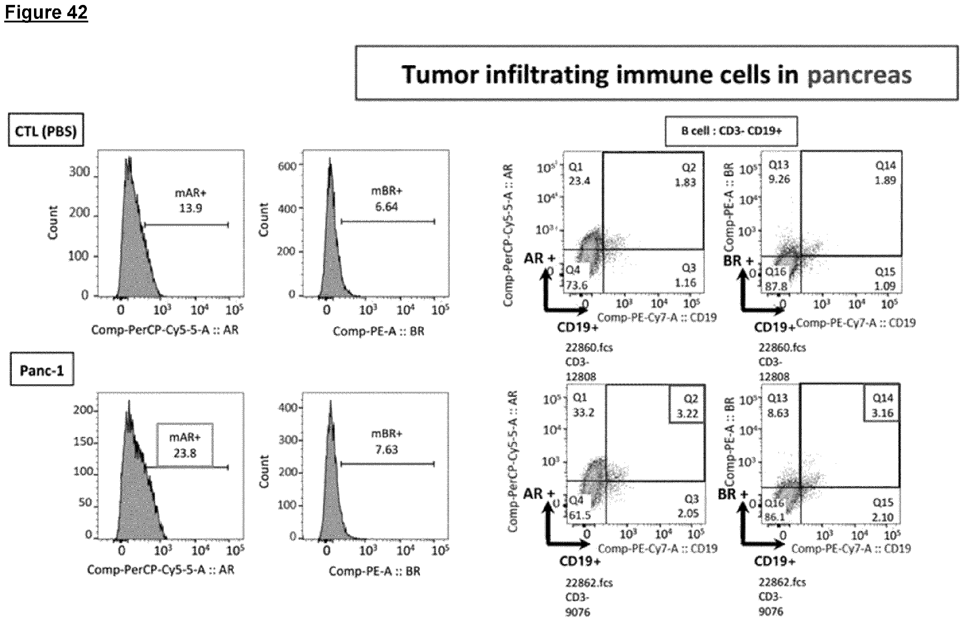

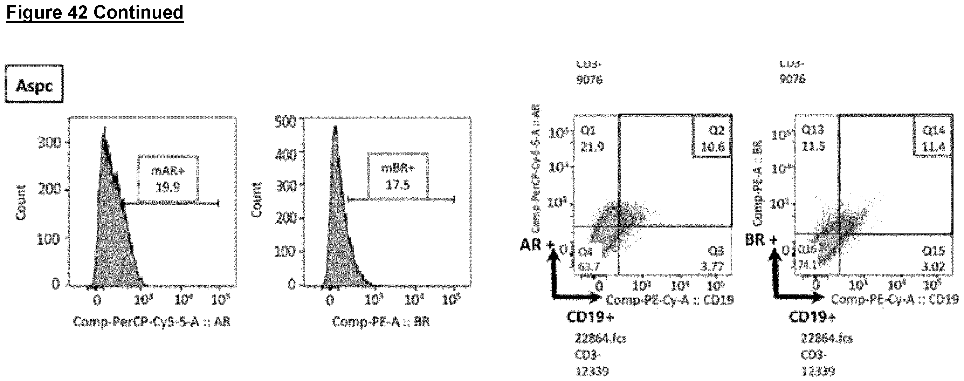

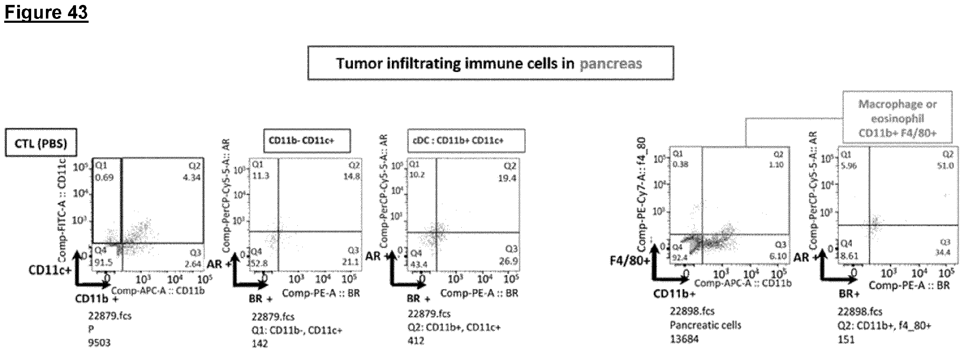

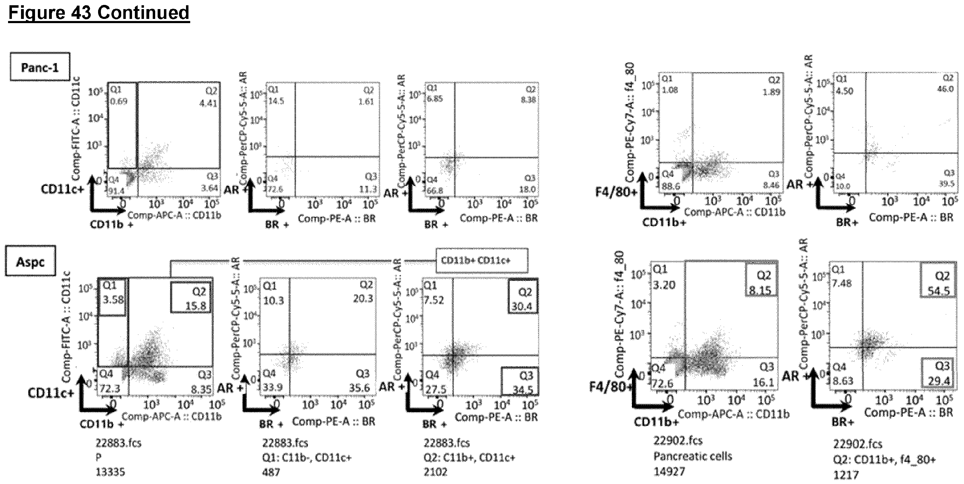

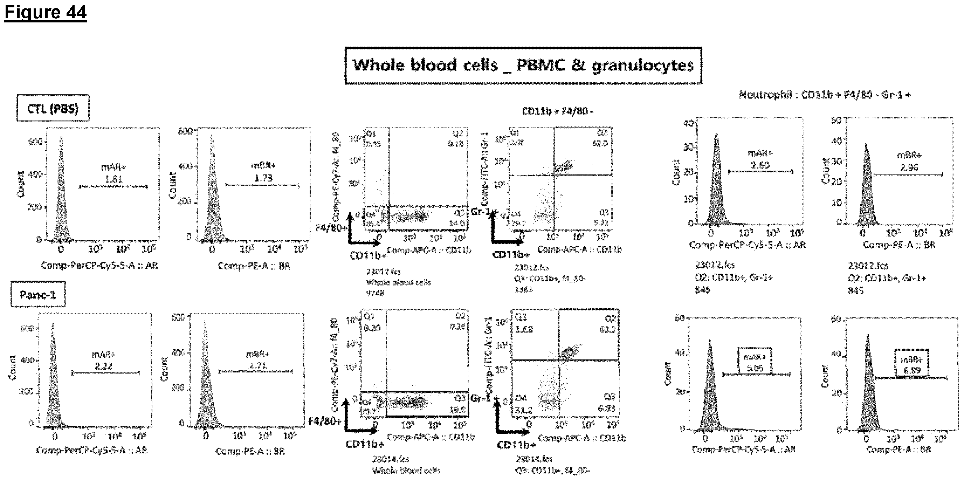

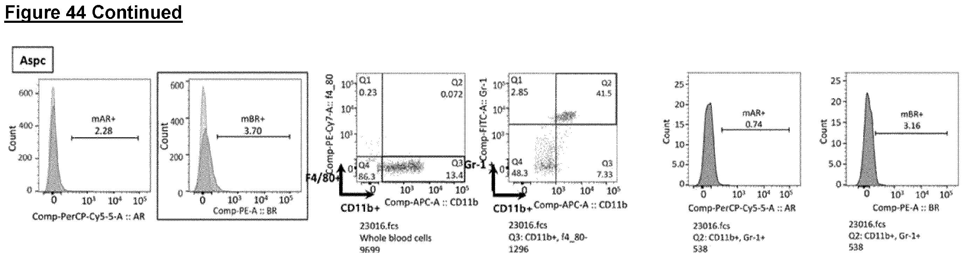

FIG. 39 shows the design of the experiment, FIG. 40 shows images of pancreases and spleen after transplantation of pancreatic cancer cells, and FIG. 41 shows H&E staining images of pancreatic tissue. In addition, FIGS. 42 and 43 show the results of performing FACS analysis of immune cells infiltrating the pancreases using antibodies specific to IL-10RB (BR) and IL-22RA (AR), and FIG. 44 shows the results of performing FACS analysis of the mononuclear cells and granulocytes of whole-blood cells using antibodies specific to IL-10RB (BR) and IL-22RA (AR).

As shown in FIG. 40, it could be confirmed that, in the mice into which Panc-1 cells and Aspc cells were directly injected, pancreatic cancer grew and the size of the spleen also increased. As shown in FIG. 41, it could be confirmed that, in the normal control group, normal pancreatic acinus, pancreatic ducts and pancreatic islets were observed, whereas, in the group in which pancreatic cancer grew, immune cells infiltrated cancer tissue, and connective tissue was formed (desmoplasia).

As shown in FIG. 42, as a result of performing FACS analysis of cells infiltrating pancreatic cancer, it could be confirmed that the proportions of IL-22RA-expressing B cells (AR+) and IL-10RB-expressing B cells (BR+) in the group into which pancreatic cancer grew by direct injection of Panc-1 cells and Aspc cells increased compared to those in the normal control group. In addition, as shown in FIG. 43, it could be confirmed that the proportions of dendritic cells (CD11b+ CD11c+ cells) expressing IL-22RA and IL-10RB or expressing IL-10RB and macrophages (CD11b+ F4/80+ cells) expressing IL-22RA and IL-10RB or expressing IL-10RB, in the group in which pancreatic cancer grew by direct injection of Panc-1 cells and Aspc cells, increased compared to those in the normal control group. In addition, as shown in FIG. 44, it could be confirmed that the proportion of neutrophil cells (CD11b+ F4/80- Gr-1+ cells) expressing IL-22RA and IL-10RB, in the group in which pancreatic cancer grew by direct injection of Panc-1 cells and Aspc cells, increased compared to that in the normal control group.

### [Example 21]

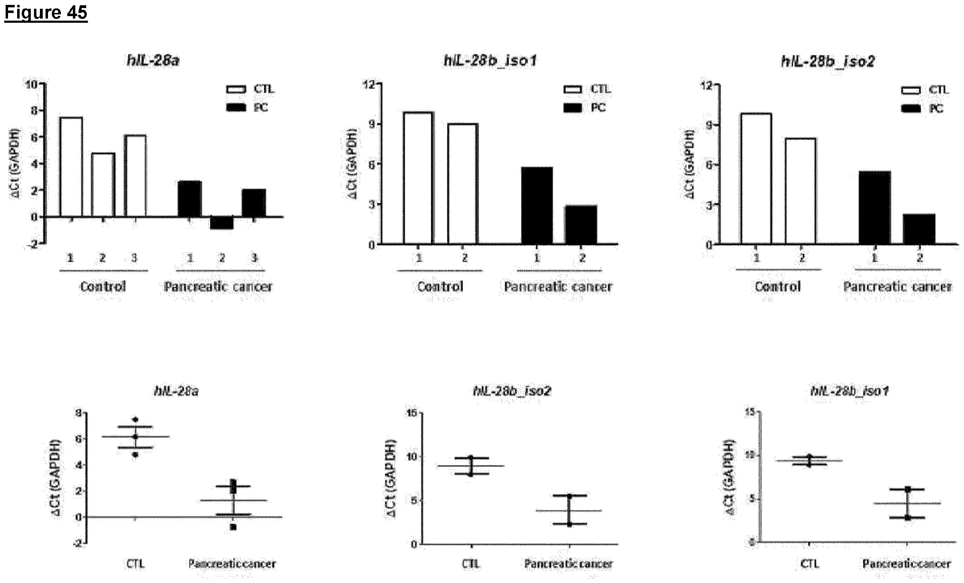

Blood was sampled from 3 normal control subjects and 3 pancreatic cancer patients, and peripheral blood mononuclear cells were isolated therefrom. Then, the mRNA expression level of each of IL-28A, IL-28B isoform 1 and IL-28B isoform 2 in the mononuclear cells was measured using qRT-PCR, and the results are shown in FIG. 45.

As shown in FIG. 45, it could be confirmed that the mRNA expression levels of IL-28A, IL-28B isoform 1 and IL-28B isoform 2 in the mononuclear cells isolated from the pancreatic cancer patients all increased compared to those in the normal control group.

Therefore, it can be seen that expression of IL-28A, IL-28B isoform 1 and IL-28B isoform 2 in the mononuclear cells corresponds to an effective biomarker for diagnosis of pancreatic cancer.

### Industrial Applicability

According to the present invention, it is possible to accurately predict or diagnose the onset or likelihood of onset of various diseases, comprising pancreatic disease, autoimmune disease, allergy, Grave's disease, Hashimoto's thyroiditis, psoriasis, Kawasaki disease and autoimmune lymphoproliferative syndrome, particularly pancreatic cancer, by measuring the expression level of interleukin 10 receptor, interleukin 22 receptor, interleukin 22, interleukin 28A, interleukin 28B isoform 1, interleukin 28B isoform 2, interleukin 29 and/or interferon lambda receptor 1 isoform 1 as biomarker(s).

In addition, according to the present invention, since it is possible to predict or diagnose the onset of diseases by measuring the expression level of the biomarker from a liquid biopsy isolated from a subject, it is possible to rapidly, simply and very accurately diagnose diseases such as pancreatic cancer by a non-invasive method, compared to a conventional art.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Sequence Listing Free Test

Electronic file is attached.

## Claims

1. A composition for diagnosing pancreatic disease comprising an agent for measuring an expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or an mRNA expression level of a gene encoding the protein.

2. The composition according to claim 1, wherein the composition is for a liquid biopsy isolated from a subject of interest.

3. The composition according to claim 1, wherein the composition is for mononuclear cells, granulocytes or exosomes isolated from a subject of interest.

4. The composition according to claim 1, wherein the composition comprises an agent for measuring the expression level of interleukin 10 receptor or the mRNA expression level of a gene encoding the interleukin 10 receptor.

5. The composition according to claim 4, wherein the composition further comprises an agent for measuring the expression level of interleukin 22 receptor or the mRNA expression level of a gene encoding the interleukin 22 receptor.

6. The composition according to claim 4, wherein the composition further comprises an agent for measuring the expression level of at least one protein selected from the group consisting of interleukin 22, interleukin 28A, interleukin 28B isoform 1, interleukin 28B isoform 2, interleukin 29 and interferon lambda receptor 1 isoform 1, or the mRNA expression level of a gene encoding the protein.

7. The composition according to claim 1, wherein the agent for measuring the expression level of the protein comprises at least one selected from the group consisting of antibodies, oligopeptides, ligands, PNAs (peptide nucleic acids) and aptamers, which bind specifically to the protein.

8. The composition according to claim 1, wherein the agent for measuring the mRNA expression level comprises at least one selected from the group consisting of primers, probes and antisense nucleotides, which bind specifically bind to the mRNA.

9. The composition according to claim 1, wherein the pancreatic disease is pancreatic cancer.

10. A kit for diagnosing pancreatic disease comprising the composition of any one of claims 1 to 9.

11. The kit according to claim 10, wherein the kit is an RT-PCR kit, a DNA chip kit, an ELISA kit, a protein chip kit, a rapid kit, or a multiple-reaction-monitoring (MRM) kit.

12. A method of providing information for diagnosis of pancreatic disease comprising a step of:
measuring an expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or an mRNA expression level of a gene encoding the protein, in a biopsy isolated from a subject of interest.

13. The method according to claim 12, wherein the biopsy is a liquid biopsy.

14. The method according to claim 12, wherein the biopsy contains at least one of mononuclear cells, granulocytes and exosomes isolated from the subject.

15. The method according to claim 12, comprising a step of measuring the expression level of interleukin 10 receptor or the mRNA expression level of a gene encoding the interleukin 10 receptor, in the biopsy.

16. The method according to claim 15, further comprising a step of measuring the expression level of interleukin 22 receptor or the mRNA expression level of a gene encoding the interleukin 22 receptor, in the biopsy.

17. The method according to claim 16, further comprising a step of measuring the expression level of at least one protein selected from the group consisting of interleukin 22, interleukin 28A, interleukin 28B isoform 1, interleukin 28B isoform 2, interleukin 29 and interferon lambda receptor 1 isoform 1, or the mRNA expression level of a gene encoding the protein, in the biopsy.

18. The method according to claim 12, wherein, when the expression level of the protein or the mRNA expression level of the gene, measured from the biopsy isolated from the subject of interest, is higher than the expression level in a normal control group, likelihood of onset of the pancreatic disease is determined to be high.

19. The method according to claim 16, wherein, when the expression level of interleukin 22 receptor or the mRNA expression level of a gene encoding the interleukin 22 receptor, measured from the biopsy isolated from the subject of interest, is higher than the expression level in a normal control group, likelihood of onset of the pancreatic disease is determined to be high.

20. The method according to claim 12, wherein the pancreatic disease is pancreatic cancer.

21. A method of providing information for predicting likelihood of recurrence of disease comprising a step of:
measuring an expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or an mRNA expression level of a gene encoding the protein, in a biopsy isolated from a pancreatic disease patient who received treatment.

22. The method according to claim 21, wherein the biopsy is a liquid biopsy.

23. The method according to claim 21, wherein, when the expression level of the protein or the mRNA expression level of the gene, measured from the biopsy isolated from the patient, is higher than expression level in a normal control group, the likelihood of recurrence of the pancreatic disease is determined to be high.

24. The method according to claim 21, wherein the pancreatic disease is pancreatic cancer.

25. A method for screening a drug for disease treatment comprising steps of:
(a) measuring an expression level of at least one protein selected from the group consisting of interleukin 10 receptor (IL-10R), interleukin 22 receptor (IL-22R), interleukin 22 (IL-22), interleukin 28A (IL-28A), interleukin 28B isoform 1 (IL-28B isoform 1), interleukin 28B isoform 2 (IL-28B isoform 2), interleukin 29 (IL-29) and interferon lambda receptor 1 isoform 1 (IFNLR1 isoform 1), or an mRNA expression level of a gene encoding the protein, in a biopsy isolated from a pancreatic disease patient;
(b) administering a candidate drug to the patient; and
(c) measuring an expression level of at least one protein selected from the group consisting of interleukin 10 receptor, interleukin 22 receptor, interleukin 22, interleukin 28A, interleukin 28B isoform 1, interleukin 28B isoform 2, interleukin 29 and interferon lambda receptor 1 isoform 1, or an mRNA expression level of the gene encoding the protein, in a biopsy isolated from the patient after administration of the candidate drug.

26. The method according to claim 25, wherein the biopsy is a liquid biopsy.

27. The method according to claim 25, further comprising a step of selecting the candidate drug as a drug for disease treatment, when the expression level of the protein or the mRNA expression level of the gene encoding the protein, measured in step (c), decreases compared to the expression level of the protein or the mRNA expression level of the gene encoding the protein, measured in step (a).

28. The method according to claim 25, wherein the pancreatic disease is pancreatic cancer.


<110>    HuVet bio

<120>    Composition for diagnosing disease

<130>    PP-B2223

<150>    KR 10-2018-0058/419
<151>    2018-05-23

<160>    8

<170>    KoPatentIn 3.0

<210>    1
<211>    325
<212>    PRT
<213>    Homo sapiens

<400>    1
Met Ala Trp Ser Leu Gly Ser Trp Leu Gly Gly Cys Leu Leu Val Ser
  1               5                  10                  15

Ala Leu Gly Met Val Pro Pro Pro Glu Asn Val Arg Met Asn Ser Val
             20                  25                  30

Asn Phe Lys Asn Ile Leu Gln Trp Glu Ser Pro Ala Phe Ala Lys Gly
         35                  40                  45

Asn Leu Thr Phe Thr Ala Gln Tyr Leu Ser Tyr Arg Ile Phe Gln Asp
     50                  55                  60

Lys Cys Met Asn Thr Thr Leu Thr Glu Cys Asp Phe Ser Ser Leu Ser
 65                  70                  75                  80

Lys Tyr Gly Asp His Thr Leu Arg Val Arg Ala Glu Phe Ala Asp Glu
                 85                  90                  95

His Ser Asp Trp Val Asn Ile Thr Phe Cys Pro Val Asp Asp Thr Ile
            100                 105                 110

Ile Gly Pro Pro Gly Met Gln Val Glu Val Leu Ala Asp Ser Leu His
        115                 120                 125

Met Arg Phe Leu Ala Pro Lys Ile Glu Asn Glu Tyr Glu Thr Trp Thr
    130                 135                 140

Met Lys Asn Val Tyr Asn Ser Trp Thr Tyr Asn Val Gln Tyr Trp Lys
145                 150                 155                 160

Asn Gly Thr Asp Glu Lys Phe Gln Ile Thr Pro Gln Tyr Asp Phe Glu
                165                 170                 175

Val Leu Arg Asn Leu Glu Pro Trp Thr Thr Tyr Cys Val Gln Val Arg
            180                 185                 190

Gly Phe Leu Pro Asp Arg Asn Lys Ala Gly Glu Trp Ser Glu Pro Val
        195                 200                 205

Cys Glu Gln Thr Thr His Asp Glu Thr Val Pro Ser Trp Met Val Ala
    210                 215                 220

Val Ile Leu Met Ala Ser Val Phe Met Val Cys Leu Ala Leu Leu Gly
225                 230                 235                 240

Cys Phe Ala Leu Leu Trp Cys Val Tyr Lys Lys Thr Lys Tyr Ala Phe
                245                 250                 255

Ser Pro Arg Asn Ser Leu Pro Gln His Leu Lys Glu Phe Leu Gly His
            260                 265                 270

Pro His His Asn Thr Leu Leu Phe Phe Ser Phe Pro Leu Ser Asp Glu
        275                 280                 285

Asn Asp Val Phe Asp Lys Leu Ser Val Ile Ala Glu Asp Ser Glu Ser
    290                 295                 300

Gly Lys Gln Asn Pro Gly Asp Ser Cys Ser Leu Gly Thr Pro Pro Gly
305                 310                 315                 320

Gln Gly Pro Gln Ser
                325


<210>    2
<211>    574
<212>    PRT
<213>    Homo sapiens

<400>    2
Met Arg Thr Leu Leu Thr Ile Leu Thr Val Gly Ser Leu Ala Ala His
  1               5                  10                  15

Ala Pro Glu Asp Pro Ser Asp Leu Leu Gln His Val Lys Phe Gln Ser
            20                  25                  30

Ser Asn Phe Glu Asn Ile Leu Thr Trp Asp Ser Gly Pro Glu Gly Thr
        35                  40                  45

Pro Asp Thr Val Tyr Ser Ile Glu Tyr Lys Thr Tyr Gly Glu Arg Asp
    50                  55                  60

Trp Val Ala Lys Lys Gly Cys Gln Arg Ile Thr Arg Lys Ser Cys Asn
 65                  70                  75                  80

Leu Thr Val Glu Thr Gly Asn Leu Thr Glu Leu Tyr Tyr Ala Arg Val
                85                  90                  95

Thr Ala Val Ser Ala Gly Gly Arg Ser Ala Thr Lys Met Thr Asp Arg
            100                 105                 110

Phe Ser Ser Leu Gln His Thr Thr Leu Lys Pro Pro Asp Val Thr Cys
        115                 120                 125

Ile Ser Lys Val Arg Ser Ile Gln Met Ile Val His Pro Thr Pro Thr
    130                 135                 140

Pro Ile Arg Ala Gly Asp Gly His Arg Leu Thr Leu Glu Asp Ile Phe
145                 150                 155                 160

His Asp Leu Phe Tyr His Leu Glu Leu Gln Val Asn Arg Thr Tyr Gln
                165                 170                 175

Met His Leu Gly Gly Lys Gln Arg Glu Tyr Glu Phe Phe Gly Leu Thr
            180                 185                 190

Pro Asp Thr Glu Phe Leu Gly Thr Ile Met Ile Cys Val Pro Thr Trp

195                 200                 205

Ala Lys Glu Ser Ala Pro Tyr Met Cys Arg Val Lys Thr Leu Pro Asp
    210                 215                 220

Arg Thr Trp Thr Tyr Ser Phe Ser Gly Ala Phe Leu Phe Ser Met Gly
225                 230                 235                 240

Phe Leu Val Ala Val Leu Cys Tyr Leu Ser Tyr Arg Tyr Val Thr Lys
                245                 250                 255

Pro Pro Ala Pro Pro Asn Ser Leu Asn Val Gln Arg Val Leu Thr Phe
            260                 265                 270

Gln Pro Leu Arg Phe Ile Gln Glu His Val Leu Ile Pro Val Phe Asp
        275                 280                 285

Leu Ser Gly Pro Ser Ser Leu Ala Gln Pro Val Gln Tyr Ser Gln Ile
    290                 295                 300

Arg Val Ser Gly Pro Arg Glu Pro Ala Gly Ala Pro Gln Arg His Ser
305                 310                 315                 320

Leu Ser Glu Ile Thr Tyr Leu Gly Gln Pro Asp Ile Ser Ile Leu Gln
                325                 330                 335

Pro Ser Asn Val Pro Pro Pro Gln Ile Leu Ser Pro Leu Ser Tyr Ala
            340                 345                 350

Pro Asn Ala Ala Pro Glu Val Gly Pro Pro Ser Tyr Ala Pro Gln Val
        355                 360                 365

Thr Pro Glu Ala Gln Phe Pro Phe Tyr Ala Pro Gln Ala Ile Ser Lys
    370                 375                 380

Val Gln Pro Ser Ser Tyr Ala Pro Gln Ala Thr Pro Asp Ser Trp Pro
385                 390                 395                 400

Pro Ser Tyr Gly Val Cys Met Glu Gly Ser Gly Lys Asp Ser Pro Thr
                405                 410                 415

Gly Thr Leu Ser Ser Pro Lys His Leu Arg Pro Lys Gly Gln Leu Gln
            420                 425                 430

Lys Glu Pro Pro Ala Gly Ser Cys Met Leu Gly Gly Leu Ser Leu Gln
        435                 440                 445

Glu Val Thr Ser Leu Ala Met Glu Glu Ser Gln Glu Ala Lys Ser Leu
    450                 455                 460

His Gln Pro Leu Gly Ile Cys Thr Asp Arg Thr Ser Asp Pro Asn Val
465                 470                 475                 480

Leu His Ser Gly Glu Glu Gly Thr Pro Gln Tyr Leu Lys Gly Gln Leu
                485                 490                 495

Pro Leu Leu Ser Ser Val Gln Ile Glu Gly His Pro Met Ser Leu Pro
            500                 505                 510

Leu Gln Pro Pro Ser Arg Pro Cys Ser Pro Ser Asp Gln Gly Pro Ser
        515                 520                 525

Pro Trp Gly Leu Leu Glu Ser Leu Val Cys Pro Lys Asp Glu Ala Lys

530                 535                 540

Ser Pro Ala Pro Glu Thr Ser Asp Leu Glu Gln Pro Thr Glu Leu Asp
545                 550                 555                 560

Ser Leu Phe Arg Gly Leu Ala Leu Thr Val Gln Trp Glu Ser
                565                 570

<210>    3
<211>    179
<212>    PRT
<213>    Homo sapiens

<400>    3
Met Ala Ala Leu Gln Lys Ser Val Ser Ser Phe Leu Met Gly Thr Leu
1               5                   10                  15

Ala Thr Ser Cys Leu Leu Leu Leu Ala Leu Leu Val Gln Gly Gly Ala
            20                  25                  30

Ala Ala Pro Ile Ser Ser His Cys Arg Leu Asp Lys Ser Asn Phe Gln
        35                  40                  45

Gln Pro Tyr Ile Thr Asn Arg Thr Phe Met Leu Ala Lys Glu Ala Ser
    50                  55                  60

Leu Ala Asp Asn Asn Thr Asp Val Arg Leu Ile Gly Glu Lys Leu Phe
65                  70                  75                  80

His Gly Val Ser Met Ser Glu Arg Cys Tyr Leu Met Lys Gln Val Leu
                85                  90                  95

Asn Phe Thr Leu Glu Glu Val Leu Phe Pro Gln Ser Asp Arg Phe Gln
            100                 105                 110

Pro Tyr Met Gln Glu Val Val Pro Phe Leu Ala Arg Leu Ser Asn Arg
        115                 120                 125

Leu Ser Thr Cys His Ile Glu Gly Asp Asp Leu His Ile Gln Arg Asn
    130                 135                 140

Val Gln Lys Leu Lys Asp Thr Val Lys Lys Leu Gly Glu Ser Gly Glu
145                 150                 155                 160

Ile Lys Ala Ile Gly Glu Leu Asp Leu Leu Phe Met Ser Leu Arg Asn
                165                 170                 175

Ala Cys Ile

<210>    4
<211>    200
<212>    PRT
<213>    Homo sapiens

<400>    4
Met Lys Leu Asp Met Thr Gly Asp Cys Thr Pro Val Leu Val Leu Met
1               5                   10                  15

Ala Ala Val Leu Thr Val Thr Gly Ala Val Pro Val Ala Arg Leu His
            20                  25                  30

Gly Ala Leu Pro Asp Ala Arg Gly Cys His Ile Ala Gln Phe Lys Ser
            35                  40                  45

Leu Ser Pro Gln Glu Leu Gln Ala Phe Lys Arg Ala Lys Asp Ala Leu
        50                  55                  60

Glu Glu Ser Leu Leu Leu Lys Asp Cys Arg Cys His Ser Arg Leu Phe
65                  70                  75                  80

Pro Arg Thr Trp Asp Leu Arg Gln Leu Gln Val Arg Glu Arg Pro Met
                85                  90                  95

Ala Leu Glu Ala Glu Leu Ala Leu Thr Leu Lys Val Leu Glu Ala Thr
            100                 105                 110

Ala Asp Thr Asp Pro Ala Leu Val Asp Val Leu Asp Gln Pro Leu His
        115                 120                 125

Thr Leu His His Ile Leu Ser Gln Phe Arg Ala Cys Ile Gln Pro Gln
    130                 135                 140

Pro Thr Ala Gly Pro Arg Thr Arg Gly Arg Leu His His Trp Leu Tyr
145                 150                 155                 160

Arg Leu Gln Glu Ala Pro Lys Lys Glu Ser Pro Gly Cys Leu Glu Ala
                165                 170                 175

Ser Val Thr Phe Asn Leu Phe Arg Leu Leu Thr Arg Asp Leu Asn Cys
            180                 185                 190

Val Ala Ser Gly Asp Leu Cys Val
        195                 200

<210>   5
<211>   200
<212>   PRT
<213>   Homo sapiens

<400>   5
Met Lys Leu Asp Met Thr Gly Asp Cys Met Pro Val Leu Val Leu Met
1               5                   10                  15

Ala Ala Val Leu Thr Val Thr Gly Ala Val Pro Val Ala Arg Leu Arg
            20                  25                  30

Gly Ala Leu Pro Asp Ala Arg Gly Cys His Ile Ala Gln Phe Lys Ser
        35                  40                  45

Leu Ser Pro Gln Glu Leu Gln Ala Phe Lys Arg Ala Lys Asp Ala Leu
    50                  55                  60

Glu Glu Ser Leu Leu Leu Lys Asp Cys Lys Cys Arg Ser Arg Leu Phe
65                  70                  75                  80

Pro Arg Thr Trp Asp Leu Arg Gln Leu Gln Val Arg Glu Arg Pro Val
                85                  90                  95

Ala Leu Glu Ala Glu Leu Ala Leu Thr Leu Lys Val Leu Glu Ala Thr
            100                 105                 110

Ala Asp Thr Asp Pro Ala Leu Gly Asp Val Leu Asp Gln Pro Leu His

115                 120                 125

Thr Leu His His Ile Leu Ser Gln Leu Arg Ala Cys Ile Gln Pro Gln
    130                 135                 140

Pro Thr Ala Gly Pro Arg Thr Arg Gly Arg Leu His His Trp Leu His
145                 150                 155                 160

Arg Leu Gln Glu Ala Pro Lys Lys Glu Ser Pro Gly Cys Leu Glu Ala
                165                 170                 175

Ser Val Thr Phe Asn Leu Phe Arg Leu Leu Thr Arg Asp Leu Asn Cys
            180                 185                 190

Val Ala Ser Gly Asp Leu Cys Val
        195                 200

<210>    6
<211>    196
<212>    PRT
<213>    Homo sapiens

<400>    6
Met Thr Gly Asp Cys Met Pro Val Leu Val Leu Met Ala Ala Val Leu
 1               5                  10                  15

Thr Val Thr Gly Ala Val Pro Val Ala Arg Leu Arg Gly Ala Leu Pro
            20                  25                  30

Asp Ala Arg Gly Cys His Ile Ala Gln Phe Lys Ser Leu Ser Pro Gln
        35                  40                  45

Glu Leu Gln Ala Phe Lys Arg Ala Lys Asp Ala Leu Glu Glu Ser Leu
    50                  55                  60

Leu Leu Lys Asp Cys Lys Cys Arg Ser Arg Leu Phe Pro Arg Thr Trp
65                  70                  75                  80

Asp Leu Arg Gln Leu Gln Val Arg Glu Arg Pro Val Ala Leu Glu Ala
                85                  90                  95

Glu Leu Ala Leu Thr Leu Lys Val Leu Glu Ala Thr Ala Asp Thr Asp
            100                 105                 110

Pro Ala Leu Gly Asp Val Leu Asp Gln Pro Leu His Thr Leu His His
        115                 120                 125

Ile Leu Ser Gln Leu Arg Ala Cys Ile Gln Pro Gln Pro Thr Ala Gly
    130                 135                 140

Pro Arg Thr Arg Gly Arg Leu His His Trp Leu His Arg Leu Gln Glu
145                 150                 155                 160

Ala Pro Lys Lys Glu Ser Pro Gly Cys Leu Glu Ala Ser Val Thr Phe
                165                 170                 175

Asn Leu Phe Arg Leu Leu Thr Arg Asp Leu Asn Cys Val Ala Ser Gly
            180                 185                 190

Asp Leu Cys Val
        195

<210>    7
<211>    200
<212>    PRT
<213>    Homo sapiens

<400>    7
Met Ala Ala Ala Trp Thr Val Val Leu Val Thr Leu Val Leu Gly Leu
1               5                   10                  15

Ala Val Ala Gly Pro Val Pro Thr Ser Lys Pro Thr Thr Thr Gly Lys
            20                  25                  30

Gly Cys His Ile Gly Arg Phe Lys Ser Leu Ser Pro Gln Glu Leu Ala
        35                  40                  45

Ser Phe Lys Lys Ala Arg Asp Ala Leu Glu Glu Ser Leu Lys Leu Lys
    50                  55                  60

Asn Trp Ser Cys Ser Ser Pro Val Phe Pro Gly Asn Trp Asp Leu Arg
65                  70                  75                  80

Leu Leu Gln Val Arg Glu Arg Pro Val Ala Leu Glu Ala Glu Leu Ala
                85                  90                  95

Leu Thr Leu Lys Val Leu Glu Ala Ala Ala Gly Pro Ala Leu Glu Asp
            100                 105                 110

Val Leu Asp Gln Pro Leu His Thr Leu His His Ile Leu Ser Gln Leu
        115                 120                 125

Gln Ala Cys Ile Gln Pro Gln Pro Thr Ala Gly Pro Arg Pro Arg Gly
    130                 135                 140

Arg Leu His His Trp Leu His Arg Leu Gln Glu Ala Pro Lys Lys Glu
145                 150                 155                 160

Ser Ala Gly Cys Leu Glu Ala Ser Val Thr Phe Asn Leu Phe Arg Leu
                165                 170                 175

Leu Thr Arg Asp Leu Lys Tyr Val Ala Asp Gly Asn Leu Cys Leu Arg
            180                 185                 190

Thr Ser Thr His Pro Glu Ser Thr
        195                 200


<210>    8
<211>    520
<212>    PRT
<213>    Homo sapiens

<400>    8
Met Ala Gly Pro Glu Arg Trp Gly Pro Leu Leu Leu Cys Leu Leu Gln
1               5                   10                  15

Ala Ala Pro Gly Arg Pro Arg Leu Ala Pro Pro Gln Asn Val Thr Leu
            20                  25                  30

Leu Ser Gln Asn Phe Ser Val Tyr Leu Thr Trp Leu Pro Gly Leu Gly
        35                  40                  45

Asn Pro Gln Asp Val Thr Tyr Phe Val Ala Tyr Gln Ser Ser Pro Thr

50                  55                  60

Arg Arg Arg Trp Arg Glu Val Glu Glu Cys Ala Gly Thr Lys Glu Leu
65                  70                  75                  80

Leu Cys Ser Met Met Cys Leu Lys Lys Gln Asp Leu Tyr Asn Lys Phe
                85                  90                  95

Lys Gly Arg Val Arg Thr Val Ser Pro Ser Ser Lys Ser Pro Trp Val
            100                 105                 110

Glu Ser Glu Tyr Leu Asp Tyr Leu Phe Glu Val Glu Pro Ala Pro Pro
        115                 120                 125

Val Leu Val Leu Thr Gln Thr Glu Glu Ile Leu Ser Ala Asn Ala Thr
    130                 135                 140

Tyr Gln Leu Pro Pro Cys Met Pro Pro Leu Asp Leu Lys Tyr Glu Val
145                 150                 155                 160

Ala Phe Trp Lys Glu Gly Ala Gly Asn Lys Thr Leu Phe Pro Val Thr
                165                 170                 175

Pro His Gly Gln Pro Val Gln Ile Thr Leu Gln Pro Ala Ala Ser Glu
            180                 185                 190

His His Cys Leu Ser Ala Arg Thr Ile Tyr Thr Phe Ser Val Pro Lys
        195                 200                 205

Tyr Ser Lys Phe Ser Lys Pro Thr Cys Phe Leu Leu Glu Val Pro Glu
    210                 215                 220

Ala Asn Trp Ala Phe Leu Val Leu Pro Ser Leu Leu Ile Leu Leu Leu
225                 230                 235                 240

Val Ile Ala Ala Gly Gly Val Ile Trp Lys Thr Leu Met Gly Asn Pro
                245                 250                 255

Trp Phe Gln Arg Ala Lys Met Pro Arg Ala Leu Asp Phe Ser Gly His
            260                 265                 270

Thr His Pro Val Ala Thr Phe Gln Pro Ser Arg Pro Glu Ser Val Asn
        275                 280                 285

Asp Leu Phe Leu Cys Pro Gln Lys Glu Leu Thr Arg Gly Val Arg Pro
    290                 295                 300

Thr Pro Arg Val Arg Ala Pro Ala Thr Gln Gln Thr Arg Trp Lys Lys
305                 310                 315                 320

Asp Leu Ala Glu Asp Glu Glu Glu Glu Asp Glu Glu Asp Thr Glu Asp
                325                 330                 335

Gly Val Ser Phe Gln Pro Tyr Ile Glu Pro Pro Ser Phe Leu Gly Gln
            340                 345                 350

Glu His Gln Ala Pro Gly His Ser Glu Ala Gly Gly Val Asp Ser Gly
        355                 360                 365

Arg Pro Arg Ala Pro Leu Val Pro Ser Glu Gly Ser Ser Ala Trp Asp
    370                 375                 380

Ser Ser Asp Arg Ser Trp Ala Ser Thr Val Asp Ser Ser Trp Asp Arg

385                 390                 395                 400

Ala Gly Ser Ser Gly Tyr Leu Ala Glu Lys Gly Pro Gly Gln Gly Pro
                405                 410                 415

Gly Gly Asp Gly His Gln Glu Ser Leu Pro Pro Pro Glu Phe Ser Lys
            420                 425                 430

Asp Ser Gly Phe Leu Glu Glu Leu Pro Glu Asp Asn Leu Ser Ser Trp
        435                 440                 445

Ala Thr Trp Gly Thr Leu Pro Pro Glu Pro Asn Leu Val Pro Gly Gly
    450                 455                 460

Pro Pro Val Ser Leu Gln Thr Leu Thr Phe Cys Trp Glu Ser Ser Pro
465                 470                 475                 480

Glu Glu Glu Glu Glu Ala Arg Glu Ser Glu Ile Glu Asp Ser Asp Ala
                485                 490                 495

Gly Ser Trp Gly Ala Glu Ser Thr Gln Arg Thr Glu Asp Arg Gly Arg
            500                 505                 510

Thr Leu Gly His Tyr Met Ala Arg
        515                 520